# EUROPÄISCHE PATENTSCHRIFT

(11) **EP 2 694 536 B1**
(45) Veröffentlichungstag und Bekanntmachung des Hinweises auf die Patenterteilung: **25.07.2018**
(21) Anmeldenummer: 12710298.6
(22) Anmeldetag: 27.03.2012
(51) Int. Cl.: C07K 14/34, C12P 13/08, C12N 1/21

(54) **MIKROORGANISMUS UND VERFAHREN ZUR FERMENTATIVEN HERSTELLUNG EINER ORGANISCH-CHEMISCHEN VERBINDUNG**
MICROORGANISM AND METHOD FOR THE FERMENTATIVE PRODUCTION OF AN ORGANO CHEMICAL COMPOUND
MICROORGANISME ET PROCEDE DE PRODUCTION D'UN COMPOSE ORGANO CHIMIQUE PAR FERMENTATION

(30) Priorität: 04.04.2011 DE 102011006716
(43) Veröffentlichungstag der Anmeldung: 12.02.2014
(73) Patentinhaber: Evonik Degussa GmbH, 45128 Essen (DE)
(72) Erfinder: HANS, Stephan, 49078 Osnabrück (DE); BATHE, Brigitte, 33154 Salzkotten (DE); RETH, Alexander, 33615 Bielefeld (DE); CLAES, Wilfried, 33619 Bielefeld (DE); KRÄMER, Reinhard, 52428 Jülich (DE); SEIBOLD, Gerd, 50321 Brühl (DE); HENRICH, Alexander, 50674 Köln (DE)
(86) Internationale Anmeldenummer: PCT/EP2012/055359
(87) Internationale Veröffentlichungsnummer: WO 2012/136506

(56) Entgegenhaltungen:
- EP-A2- 1 087 015
- EP-A2- 2 107 128
- WO-A1-02/22669
- WO-A1-02/26989
- WO-A2-03/040681
- DATABASE UniProt [Online] 1. Oktober 2002 (2002-10-01), Nakagawa S.: "SubName: Full=ABC transporter, membrane spanning protein", XP002683459, Database accession no. Q8NSF1
- DATABASE UniProt [Online] 31. Oktober 2006 (2006-10-31), Nishio Y. et al.: "Putative ABC transporter permease protein", XP002683460, Database accession no. Q8FRL2
- DATABASE UniProt [Online] 1. Oktober 2002 (2002-10-01), Nakagawa S. et al.: "SubName: Full=ABC-type sugar transport systems, ATPase component", XP002677414, Database accession no. Q8NSE8
- DATABASE UniProt [Online] 1. März 2003 (2003-03-01), Nishio Y. et al.: "SubName: Full=Putative ABC transporter ATP-binding protein", XP002677415, Database accession no. Q8FRK9

### SEQUENCE LISTING

<110> Evonik Degussa GmbH
<120> Trehalose-Importer
<130> 201000450
<160> 31
<170> PatentIn version 3.3
<210> 1
   <211> 1299
   <212> DNA
   <213> Corynebacterium glutamicum
<220>
   <221> CDS
   <222> (151)..(1149)
   <223> ATP-bindendes und -hydrolysierendes (ATPase) Protein des ABC-Transporters mit der Aktivität eines Trehalose-Importers
<400> 1
   ctttgagctt gatgccgccc caaaagagtt gttgccaccg atcgcgaact ttggcagtag      60
   ccatgcgttc tgctcctgac cttgaacagc ggtcccaatt tagacccgct aaacccacaa     120
   tgtgtactgg tgctggtaat ttagtagaac atg gca acg gtc aca ttc gac aag     174
                                    Met Ala Thr Val Thr Phe Asp Lys
                                    1               5
   gtc aca atc cgg tac ccc ggc gcg gag cgc gca aca gtt cat gag ctt      222
   Val Thr Ile Arg Tyr Pro Gly Ala Glu Arg Ala Thr Val His Glu Leu
       10                  15                  20
   gat tta gat atc gct gat ggc gag ttt ttg gtg ctc gtc ggc cct tcg      270
   Asp Leu Asp Ile Ala Asp Gly Glu Phe Leu Val Leu Val Gly Pro Ser
   25                  30                  35                  40
   ggt tgt ggt aaa tcc act acg ctg cgt gct ttg gcg ggg ctt gag ggc      318
   Gly Cys Gly Lys Ser Thr Thr Leu Arg Ala Leu Ala Gly Leu Glu Gly
                   45                  50                  55
   gtg gag tcg ggt gtg atc aaa att gat ggc aag gat gtc act ggt cag      366
   Val Glu Ser Gly Val Ile Lys Ile Asp Gly Lys Asp Val Thr Gly Gln
               60                  65                  70
   gag ccg gcg gat cgc gat atc gcg atg gtg ttc cag aat tat gct ctg      414
   Glu Pro Ala Asp Arg Asp Ile Ala Met Val Phe Gln Asn Tyr Ala Leu
           75                  80                  85
   tac cct cac atg acg gtg gcg aag aat atg ggt ttt gcg ctg aag ttg      462
   Tyr Pro His Met Thr Val Ala Lys Asn Met Gly Phe Ala Leu Lys Leu
       90                  95                  100
   gct aag ctg ccg cag gcg cag atc gat gcg aag gtc aat gag gct gcg      510
   Ala Lys Leu Pro Gln Ala Gln Ile Asp Ala Lys Val Asn Glu Ala Ala
   105                 110                 115                 120
   gaa att ctt ggg ttg acg gag ttt ttg gat cgc aag cct aag gat tta      558
   Glu Ile Leu Gly Leu Thr Glu Phe Leu Asp Arg Lys Pro Lys Asp Leu
                   125                 130                 135
   tcg ggt ggt cag cgt cag cgt gtg gcg atg ggt cgc gcg ttg gtg cgt      606
   Ser Gly Gly Gln Arg Gln Arg Val Ala Met Gly Arg Ala Leu Val Arg
               140                 145                 150
   gat ccg aag gtg ttc ctc atg gat gag ccg ctg tcc aac ctg gat gcg      654
   Asp Pro Lys Val Phe Leu Met Asp Glu Pro Leu Ser Asn Leu Asp Ala
           155                 160                 165
   aaa ttg cgc gtg caa acc cgc gcg gag gtc gct gct ttg cag cgt cgc      702
   Lys Leu Arg Val Gln Thr Arg Ala Glu Val Ala Ala Leu Gln Arg Arg
       170                 175                 180
   ctg ggc acc acc acg gtg tat gtc acc cac gat cag gtt gag gca atg      750
   Leu Gly Thr Thr Thr Val Tyr Val Thr His Asp Gln Val Glu Ala Met
   185                 190                 195                 200
   acg atg ggc gat cgg gtt gcg gtg ctc aag gac ggg ttg ctg cag cag      798
   Thr Met Gly Asp Arg Val Ala Val Leu Lys Asp Gly Leu Leu Gln Gln
                   205                 210                 215
   gtc gca ccg ccc agg gag ctt tac gac gcc ccg gtc aac gaa ttc gtt      846
   Val Ala Pro Pro Arg Glu Leu Tyr Asp Ala Pro Val Asn Glu Phe Val
               220                 225                 230
   gcg ggc ttc atc ggc tcg ccg tcc atg aac ctc ttc cct gcc aac ggg      894
   Ala Gly Phe Ile Gly Ser Pro Ser Met Asn Leu Phe Pro Ala Asn Gly
           235                 240                 245
   cac aag atg ggt gtg cgc ccg gag aag atg ctg gtc aat gag acc cct      942
   His Lys Met Gly Val Arg Pro Glu Lys Met Leu Val Asn Glu Thr Pro
       250                 255                 260
   gag ggt ttc aca agc att gat gct gtg gtg gat atc gtc gag gag ctt      990
   Glu Gly Phe Thr Ser Ile Asp Ala Val Val Asp Ile Val Glu Glu Leu
   265                 270                 275                 280
   ggc tcc gaa tcg tat gtt tat gcc act tgg gag ggc cac cgc ctg gtg     1038
   Gly Ser Glu Ser Tyr Val Tyr Ala Thr Trp Glu Gly His Arg Leu Val
                   285                 290                 295
   gcc cgt tgg gtg gaa ggc ccc gtg cca gcc cct ggc acg cct gtg act     1086
   Ala Arg Trp Val Glu Gly Pro Val Pro Ala Pro Gly Thr Pro Val Thr
               300                 305                 310
   ttt tcc tat gat gcg gcg cag gcg cat cat ttc gat ctg gag tcg ggc     1134
   Phe Ser Tyr Asp Ala Ala Gln Ala His His Phe Asp Leu Glu Ser Gly
           315                 320                 325
   gag cgt atc gct tag tttcggacgt ggggaggcgt cgaaaagcat ctttattttt     1189
   Glu Arg Ile Ala
       330
   gaccctccgg gggtgattta acctaaaatt ccacacaaac gtgttcgagg tcattagatt   1249
   gataagcatc tgttgttaag aaaggtgact tcctatgtcc tcgatttccc              1299
<210> 2
   <211> 332
   <212> PRT
   <213> Corynebacterium glutamicum
<400> 2
   Met Ala Thr Val Thr Phe Asp Lys Val Thr Ile Arg Tyr Pro Gly Ala
   1               5                   10                  15
   Glu Arg Ala Thr Val His Glu Leu Asp Leu Asp Ile Ala Asp Gly Glu
               20                  25                  30
   Phe Leu Val Leu Val Gly Pro Ser Gly Cys Gly Lys Ser Thr Thr Leu
           35                  40                  45
   Arg Ala Leu Ala Gly Leu Glu Gly Val Glu Ser Gly Val Ile Lys Ile
       50                  55                  60
   Asp Gly Lys Asp Val Thr Gly Gln Glu Pro Ala Asp Arg Asp Ile Ala
   65                  70                  75                  80
   Met Val Phe Gln Asn Tyr Ala Leu Tyr Pro His Met Thr Val Ala Lys
                   85                  90                  95
   Asn Met Gly Phe Ala Leu Lys Leu Ala Lys Leu Pro Gln Ala Gln Ile
               100                 105                 110
   Asp Ala Lys Val Asn Glu Ala Ala Glu Ile Leu Gly Leu Thr Glu Phe
           115                 120                 125
   Leu Asp Arg Lys Pro Lys Asp Leu Ser Gly Gly Gln Arg Gln Arg Val
       130                 135                 140
   Ala Met Gly Arg Ala Leu Val Arg Asp Pro Lys Val Phe Leu Met Asp
   145                 150                 155                 160
   Glu Pro Leu Ser Asn Leu Asp Ala Lys Leu Arg Val Gln Thr Arg Ala
                   165                 170                 175
   Glu Val Ala Ala Leu Gln Arg Arg Leu Gly Thr Thr Thr Val Tyr Val
               180                 185                 190
   Thr His Asp Gln Val Glu Ala Met Thr Met Gly Asp Arg Val Ala Val
           195                 200                 205
   Leu Lys Asp Gly Leu Leu Gln Gln Val Ala Pro Pro Arg Glu Leu Tyr
       210                 215                 220
   Asp Ala Pro Val Asn Glu Phe Val Ala Gly Phe Ile Gly Ser Pro Ser
   225                 230                 235                 240
   Met Asn Leu Phe Pro Ala Asn Gly His Lys Met Gly Val Arg Pro Glu
                   245                 250                 255
   Lys Met Leu Val Asn Glu Thr Pro Glu Gly Phe Thr Ser Ile Asp Ala
               260                 265                 270
   Val Val Asp Ile Val Glu Glu Leu Gly Ser Glu Ser Tyr Val Tyr Ala
           275                 280                 285
   Thr Trp Glu Gly His Arg Leu Val Ala Arg Trp Val Glu Gly Pro Val
       290                 295                 300
   Pro Ala Pro Gly Thr Pro Val Thr Phe Ser Tyr Asp Ala Ala Gln Ala
   305                 310                 315                 320
   His His Phe Asp Leu Glu Ser Gly Glu Arg Ile Ala
                   325                 330
<210> 3
   <211> 1575
   <212> DNA
   <213> Corynebacterium glutamicum
<220>
   <221> CDS
   <222> (151)..(1425)
   <223> periplasmatisches (oder Lipoprotein) Substrat-bindendes Protein des ABC-Transporters mit der Aktivität eines Trehalose-Importers
<400> 3
   cgagcgtatc gcttagtttc ggacgtgggg aggcgtcgaa aagcatcttt atttttgacc      60
   ctccgggggt gatttaacct aaaattccac acaaacgtgt tcgaggtcat tagattgata     120
   agcatctgtt gttaagaaag gtgacttcct atg tcc tcg att tcc cgc aag acc      174
                                    Met Ser Ser Ile Ser Arg Lys Thr
                                    1               5
   ggc gcg tca ctt gca gcc acc aca ctg ttg gca gcg atc gca ctg gcc       222
   Gly Ala Ser Leu Ala Ala Thr Thr Leu Leu Ala Ala Ile Ala Leu Ala
       10                  15                  20
   ggt tgt agt tca gac tca agc tcc gac tcc aca gat tcc acc gct agc       270
   Gly Cys Ser Ser Asp Ser Ser Ser Asp Ser Thr Asp Ser Thr Ala Ser
   25                  30                  35                  40
   gaa ggc gca gac agc cgc ggc ccc atc acc ttt gcg atg ggc aaa aac       318
   Glu Gly Ala Asp Ser Arg Gly Pro Ile Thr Phe Ala Met Gly Lys Asn
                   45                  50                  55
   gac acc gac aaa gtc att ccg atc atc gac cgc tgg aac gaa gcc cac       366
   Asp Thr Asp Lys Val Ile Pro Ile Ile Asp Arg Trp Asn Glu Ala His
               60                  65                  70
   ccc gat gag cag gta acg ctc aac gaa ctc gcc ggt gaa gcc gac gcg       414
   Pro Asp Glu Gln Val Thr Leu Asn Glu Leu Ala Gly Glu Ala Asp Ala
           75                  80                  85
   cag cgc gaa acc ctc gtg caa tcc ctg cag gcc ggc aac tct gac tac      462
   Gln Arg Glu Thr Leu Val Gln Ser Leu Gln Ala Gly Asn Ser Asp Tyr
       90                  95                  100
   gac gtc atg gcg ctc gac gtc atc tgg acc gca gac ttc gcg gca aac      510
   Asp Val Met Ala Leu Asp Val Ile Trp Thr Ala Asp Phe Ala Ala Asn
   105                 110                 115                 120
   caa tgg ctc gca cca ctt gaa ggc gac ctc gag gta gac acc tcc gga      558
   Gln Trp Leu Ala Pro Leu Glu Gly Asp Leu Glu Val Asp Thr Ser Gly
                   125                 130                 135
   ctg ctg caa tcc acc gtg gat tcc gca acc tac aac ggc acc ctc tac      606
   Leu Leu Gln Ser Thr Val Asp Ser Ala Thr Tyr Asn Gly Thr Leu Tyr
               140                 145                 150
   gca ctg cca cag aac acc aac ggc cag cta ctg ttc cgc aac acc gaa      654
   Ala Leu Pro Gln Asn Thr Asn Gly Gln Leu Leu Phe Arg Asn Thr Glu
           155                 160                 165
   atc atc cca gaa gca cca gca aac tgg gct gac ctc gtg gaa tcc tgc      702
   Ile Ile Pro Glu Ala Pro Ala Asn Trp Ala Asp Leu Val Glu Ser Cys
       170                 175                 180
   acg ctt gct gaa gaa gca ggc gtt gat tgc ctg acc act cag ctc aag      750
   Thr Leu Ala Glu Glu Ala Gly Val Asp Cys Leu Thr Thr Gln Leu Lys
   185                 190                 195                 200
   cag tac gaa ggc ctt tca gtg aac acc atc ggc ttc atc gaa ggt tgg      798
   Gln Tyr Glu Gly Leu Ser Val Asn Thr Ile Gly Phe Ile Glu Gly Trp
                   205                 210                 215
   gga ggc agc gtc cta gac gat gac ggc aac gtc acc gta gac agc gac      846
   Gly Gly Ser Val Leu Asp Asp Asp Gly Asn Val Thr Val Asp Ser Asp
               220                 225                 230
   gac gcc aag gca ggc ctt caa gcg ctt gtc gac ggc ttc gac gac ggc      894
   Asp Ala Lys Ala Gly Leu Gln Ala Leu Val Asp Gly Phe Asp Asp Gly
           235                 240                 245
   acc atc tcc aag gca tcc ctt gca gcg acc gaa gaa gaa acc aac ctc      942
   Thr Ile Ser Lys Ala Ser Leu Ala Ala Thr Glu Glu Glu Thr Asn Leu
       250                 255                 260
   gca ttc acc gaa ggc caa acc gcc tac gcc att aac tgg cca tac atg      990
   Ala Phe Thr Glu Gly Gln Thr Ala Tyr Ala Ile Asn Trp Pro Tyr Met
   265                 270                 275                 280
   tac acc aac tcc gaa gaa gcc gaa gca acc gca ggc aaa ttc gaa gta     1038
   Tyr Thr Asn Ser Glu Glu Ala Glu Ala Thr Ala Gly Lys Phe Glu Val
                   285                 290                 295
   cag ccc ctc gta ggt aaa gac ggc gtc ggc gta tcc acc ctt ggt ggc     1086
   Gln Pro Leu Val Gly Lys Asp Gly Val Gly Val Ser Thr Leu Gly Gly
               300                 305                 310
   tac aac aac ggc atc aac gtc aac tcc gaa aac aag gca acc gcc cgc     1134
   Tyr Asn Asn Gly Ile Asn Val Asn Ser Glu Asn Lys Ala Thr Ala Arg
           315                 320                 325
   gac ttc atc gaa ttc atc atc aac gaa gag aac caa acc tgg ttc gcg     1182
   Asp Phe Ile Glu Phe Ile Ile Asn Glu Glu Asn Gln Thr Trp Phe Ala
       330                 335                 340
   gac aac tcc ttc cca cca gtt ctg gca tcc atc tac gat gat gag tcc      1230
   Asp Asn Ser Phe Pro Pro Val Leu Ala Ser Ile Tyr Asp Asp Glu Ser
   345                 350                 355                 360
   ctt gtt gag cag tac cca tac ctg cca gca ctg aag gaa tcc ctg gaa      1278
   Leu Val Glu Gln Tyr Pro Tyr Leu Pro Ala Leu Lys Glu Ser Leu Glu
                   365                 370                 375
   aac gca gca cca cgc cca gtg tct cct ttc tac cca gcc atc tcc aag      1326
   Asn Ala Ala Pro Arg Pro Val Ser Pro Phe Tyr Pro Ala Ile Ser Lys
               380                 385                 390
   gca atc cag gac aac gcc tac gca gcg ctt aac ggc aac gtc gac gtt      1374
   Ala Ile Gln Asp Asn Ala Tyr Ala Ala Leu Asn Gly Asn Val Asp Val
           395                 400                 405
   gac cag gca acc acc gat atg aag gca gcg atc gaa aac gct tcc agc      1422
   Asp Gln Ala Thr Thr Asp Met Lys Ala Ala Ile Glu Asn Ala Ser Ser
       410                 415                 420
   tag ttcggtaatt tagttcattc tccggccacc ttccctgaaa tccttagcgg           1475
   atttccacaa aggtggccgg agttttgtcc tattgttggg tgtaattgaa cttgtgtgaa   1535
   aggagtccgg atggcttccg gcaaagatct tcaagtttcc                         1575
<210> 4
   <211> 424
   <212> PRT
   <213> Corynebacterium glutamicum
<400> 4
   Met Ser Ser Ile Ser Arg Lys Thr Gly Ala Ser Leu Ala Ala Thr Thr
   1               5                   10                  15
   Leu Leu Ala Ala Ile Ala Leu Ala Gly Cys Ser Ser Asp Ser Ser Ser
               20                  25                  30
   Asp Ser Thr Asp Ser Thr Ala Ser Glu Gly Ala Asp Ser Arg Gly Pro
           35                  40                  45
   Ile Thr Phe Ala Met Gly Lys Asn Asp Thr Asp Lys Val Ile Pro Ile
       50                  55                  60
   Ile Asp Arg Trp Asn Glu Ala His Pro Asp Glu Gln Val Thr Leu Asn
   65                  70                  75                  80
   Glu Leu Ala Gly Glu Ala Asp Ala Gln Arg Glu Thr Leu Val Gln Ser
                   85                  90                  95
   Leu Gln Ala Gly Asn Ser Asp Tyr Asp Val Met Ala Leu Asp Val Ile
               100                 105                 110
   Trp Thr Ala Asp Phe Ala Ala Asn Gln Trp Leu Ala Pro Leu Glu Gly
           115                 120                 125
   Asp Leu Glu Val Asp Thr Ser Gly Leu Leu Gln Ser Thr Val Asp Ser
       130                 135                 140
   Ala Thr Tyr Asn Gly Thr Leu Tyr Ala Leu Pro Gln Asn Thr Asn Gly
   145                 150                 155                 160
   Gln Leu Leu Phe Arg Asn Thr Glu Ile Ile Pro Glu Ala Pro Ala Asn
                   165                 170                 175
   Trp Ala Asp Leu Val Glu Ser Cys Thr Leu Ala Glu Glu Ala Gly Val
               180                 185                 190
   Asp Cys Leu Thr Thr Gln Leu Lys Gln Tyr Glu Gly Leu Ser Val Asn
           195                 200                 205
   Thr Ile Gly Phe Ile Glu Gly Trp Gly Gly Ser Val Leu Asp Asp Asp
       210                 215                 220
   Gly Asn Val Thr Val Asp Ser Asp Asp Ala Lys Ala Gly Leu Gln Ala
   225                 230                 235                 240
   Leu Val Asp Gly Phe Asp Asp Gly Thr Ile Ser Lys Ala Ser Leu Ala
                   245                 250                 255
   Ala Thr Glu Glu Glu Thr Asn Leu Ala Phe Thr Glu Gly Gln Thr Ala
               260                 265                 270
   Tyr Ala Ile Asn Trp Pro Tyr Met Tyr Thr Asn Ser Glu Glu Ala Glu
           275                 280                 285
   Ala Thr Ala Gly Lys Phe Glu Val Gln Pro Leu Val Gly Lys Asp Gly
       290                 295                 300
   Val Gly Val Ser Thr Leu Gly Gly Tyr Asn Asn Gly Ile Asn Val Asn
   305                 310                 315                 320
   Ser Glu Asn Lys Ala Thr Ala Arg Asp Phe Ile Glu Phe Ile Ile Asn
                   325                 330                 335
   Glu Glu Asn Gln Thr Trp Phe Ala Asp Asn Ser Phe Pro Pro Val Leu
               340                 345                 350
   Ala Ser Ile Tyr Asp Asp Glu Ser Leu Val Glu Gln Tyr Pro Tyr Leu
   355 360 365
   Pro Ala Leu Lys Glu Ser Leu Glu Asn Ala Ala Pro Arg Pro Val Ser
   370 375 380
   Pro Phe Tyr Pro Ala Ile Ser Lys Ala Ile Gln Asp Asn Ala Tyr Ala
   385 390 395 400
   Ala Leu Asn Gly Asn Val Asp Val Asp Gln Ala Thr Thr Asp Met Lys
   405 410 415
   Ala Ala Ile Glu Asn Ala Ser Ser
   420
<210> 5
   <211> 756
   <212> DNA
   <213> Corynebacterium glutamicum
<220>
   <221> CDS
   <222> (151)..(606)
   <223> Funktion unbekannt
<400> 5
   aaggcagcga tcgaaaacgc ttccagctag ttcggtaatt tagttcattc tccggccacc     60
   ttccctgaaa tccttagcgg atttccacaa aggtggccgg agttttgtcc tattgttggg    120
   tgtaattgaa cttgtgtgaa aggagtccgg atg gct tcc ggc aaa gat ctt caa     174
                                    Met Ala Ser Gly Lys Asp Leu Gln
                                    1               5
   gtt tcc aca ttt ggc tac atc tcc cgc tgc ccc gtg cag gtc tac gaa      222
   Val Ser Thr Phe Gly Tyr Ile Ser Arg Cys Pro Val Gln Val Tyr Glu
       10                  15                  20
   gca atc gca gat ccc aga caa cta gaa cgc tac ttc gcc acc ggc gga      270
   Ala Ile Ala Asp Pro Arg Gln Leu Glu Arg Tyr Phe Ala Thr Gly Gly
   25                  30                  35                  40
   gta tct ggc cgc ctc gaa acc gga tcg act gtc tat tgg gac ttc gtt      318
   Val Ser Gly Arg Leu Glu Thr Gly Ser Thr Val Tyr Trp Asp Phe Val
                   45                  50                  55
   gat ttt ccc ggt gcg ttt ccg gtc caa gtt gtc tca gct aca cag gct      366
   Asp Phe Pro Gly Ala Phe Pro Val Gln Val Val Ser Ala Thr Gln Ala
               60                  65                  70
   gaa cac att gaa ctc cgc tgg gga caa gca aat gag ctg cgt tcc gtc      414
   Glu His Ile Glu Leu Arg Trp Gly Gln Ala Asn Glu Leu Arg Ser Val
           75                  80                  85
   aac ttc gag ttc gaa cct ttt aga aat ttc acc cgc acg aaa ctc acc      462
   Asn Phe Glu Phe Glu Pro Phe Arg Asn Phe Thr Arg Thr Lys Leu Thr
       90                  95                  100
   atc acc gaa ggc agt tgg ccg ctc act ccc gca gga gcc caa gag gct      510
   Ile Thr Glu Gly Ser Trp Pro Leu Thr Pro Ala Gly Ala Gln Glu Ala
   105                 110                 115                 120
   ctg ggc agc cag atg gga tgg act ggc atg ctg tcc gca cta aaa gcg      558
   Leu Gly Ser Gln Met Gly Trp Thr Gly Met Leu Ser Ala Leu Lys Ala
                   125                 130                 135
   tgg ctg gaa tac gga gtg aac ctc cgc gac ggg ttt tat aag caa tag      606
   Trp Leu Glu Tyr Gly Val Asn Leu Arg Asp Gly Phe Tyr Lys Gln
               140                 145                 150
   gcaatgtgtc catcacgatg tgtggcggat tatgatccat gtaacaagaa tgtgcagttt    666
   cacagaactg acaatcaact tattttgacc tgacaaaagg agcgacgaca catggccaca    726
   ttcaaacagg ccagaagcgc tgcctggctg                                     756
<210> 6
   <211> 151
   <212> PRT
   <213> Corynebacterium glutamicum
<400> 6
   Met Ala Ser Gly Lys Asp Leu Gln Val Ser Thr Phe Gly Tyr Ile Ser
   1               5                   10                  15
   Arg Cys Pro Val Gln Val Tyr Glu Ala Ile Ala Asp Pro Arg Gln Leu
               20                  25                  30
   Glu Arg Tyr Phe Ala Thr Gly Gly Val Ser Gly Arg Leu Glu Thr Gly
           35                  40                  45
   Ser Thr Val Tyr Trp Asp Phe Val Asp Phe Pro Gly Ala Phe Pro Val
       50                  55                  60
   Gln Val Val Ser Ala Thr Gln Ala Glu His Ile Glu Leu Arg Trp Gly
   65                  70                  75                  80
   Gln Ala Asn Glu Leu Arg Ser Val Asn Phe Glu Phe Glu Pro Phe Arg
                   85                  90                  95
   Asn Phe Thr Arg Thr Lys Leu Thr Ile Thr Glu Gly Ser Trp Pro Leu
               100                 105                 110
   Thr Pro Ala Gly Ala Gln Glu Ala Leu Gly Ser Gln Met Gly Trp Thr
           115                 120                 125
   Gly Met Leu Ser Ala Leu Lys Ala Trp Leu Glu Tyr Gly Val Asn Leu
       130                 135                 140
   Arg Asp Gly Phe Tyr Lys Gln
                       145                 150
<210> 7
   <211> 1335
   <212> DNA
   <213> Corynebacterium glutamicum
<220>
   <221> CDS
   <222> (151)..(1185)
   <223> integrales Membranprotein (Permease) des ABC-Transporters mit der Aktivität eines Trehalose-Importers
<400> 7
   tacggagtga acctccgcga cgggttttat aagcaatagg caatgtgtcc atcacgatgt      60
   gtggcggatt atgatccatg taacaagaat gtgcagtttc acagaactga caatcaactt     120
   attttgacct gacaaaagga gcgacgacac atg gcc aca ttc aaa cag gcc aga      174
                                    Met Ala Thr Phe Lys Gln Ala Arg
                                    1               5
   agc gct gcc tgg ctg atc gcc ccc gcc ctc gtg gtc ctt gca gtg gtg       222
   Ser Ala Ala Trp Leu Ile Ala Pro Ala Leu Val Val Leu Ala Val Val
       10                  15                  20
   atc gga tat ccc atc gtc cga gca att tgg cta tcc ttc cag gcc gac       270
   Ile Gly Tyr Pro Ile Val Arg Ala Ile Trp Leu Ser Phe Gln Ala Asp
   25                  30                  35                  40
   aaa ggc ctc gac ccc acc acc gga ctc ttc acc gac ggt ggc ttc gca       318
   Lys Gly Leu Asp Pro Thr Thr Gly Leu Phe Thr Asp Gly Gly Phe Ala
                   45                  50                  55
   gga cta gac aat tac ctc tac tgg ctc acc caa cga tgc atg ggt tca       366
   Gly Leu Asp Asn Tyr Leu Tyr Trp Leu Thr Gln Arg Cys Met Gly Ser
               60                  65                  70
   gac ggc acc atc cgt acc tgc cca ccc ggc aca cta gcc acc gac ttc       414
   Asp Gly Thr Ile Arg Thr Cys Pro Pro Gly Thr Leu Ala Thr Asp Phe
           75                  80                  85
   tgg cca gca cta cgc atc acg ttg ttc ttc acc gtg gtt acc gtc ggc       462
   Trp Pro Ala Leu Arg Ile Thr Leu Phe Phe Thr Val Val Thr Val Gly
       90                  95                  100
   ttg gaa act atc ctc ggc acc gcc atg gca ctg atc atg aac aaa gaa       510
   Leu Glu Thr Ile Leu Gly Thr Ala Met Ala Leu Ile Met Asn Lys Glu
   105                 110                 115                 120
   ttc cgt ggc cgc gca ctt gtt cgc gca gcg att ctt atc cct tgg gca       558
   Phe Arg Gly Arg Ala Leu Val Arg Ala Ala Ile Leu Ile Pro Trp Ala
                   125                 130                 135
   atc ccc acc gcc gtc acc gca aaa ctg tgg cag ttc atc ttc gca cca       606
   Ile Pro Thr Ala Val Thr Ala Lys Leu Trp Gln Phe Ile Phe Ala Pro
               140                 145                 150
   caa ggc atc atc aac tcc atg ttt gga ctt agt gtc agt tgg acc acc       654
   Gln Gly Ile Ile Asn Ser Met Phe Gly Leu Ser Val Ser Trp Thr Thr
           155                 160                 165
   gat ccg tgg gca gct aga gcc gcc gtc att ctt gcc gac gtc tgg aaa      702
   Asp Pro Trp Ala Ala Arg Ala Ala Val Ile Leu Ala Asp Val Trp Lys
       170                 175                 180
   acc aca cca ttc atg gca ctg ctg atc ctc gcc ggt ctg caa atg atc      750
   Thr Thr Pro Phe Met Ala Leu Leu Ile Leu Ala Gly Leu Gln Met Ile
   185                 190                 195                 200
   ccg aag gaa acc tac gaa gca gcc cgc gtc gat ggc gca acc gcg tgg      798
   Pro Lys Glu Thr Tyr Glu Ala Ala Arg Val Asp Gly Ala Thr Ala Trp
                   205                 210                 215
   cag caa ttc acc aag atc acc ctc ccg ctg gtg cgc cca gct ttg atg      846
   Gln Gln Phe Thr Lys Ile Thr Leu Pro Leu Val Arg Pro Ala Leu Met
               220                 225                 230
   gtg gca gta ctc ttc cgc acc ctc gat gcg cta cgc atg tat gac ctc      894
   Val Ala Val Leu Phe Arg Thr Leu Asp Ala Leu Arg Met Tyr Asp Leu
           235                 240                 245
   ccc gtc atc atg atc tcc agc tcc tcc aac tcc ccc acc gct gtt atc      942
   Pro Val Ile Met Ile Ser Ser Ser Ser Asn Ser Pro Thr Ala Val Ile
       250                 255                 260
   tcc cag ctg gtt gtg gaa gac atg cgc caa aac aac ttc aac tcc gct      990
   Ser Gln Leu Val Val Glu Asp Met Arg Gln Asn Asn Phe Asn Ser Ala
   265                 270                 275                 280
   tcc gcc ctt tcc aca ctg atc ttc ctg ctg atc ttc ttc gtg gcg ttc     1038
   Ser Ala Leu Ser Thr Leu Ile Phe Leu Leu Ile Phe Phe Val Ala Phe
                   285                 290                 295
   atc atg atc cga ttc ctc ggc gca gat gtt tcg ggc caa cgc gga ata     1086
   Ile Met Ile Arg Phe Leu Gly Ala Asp Val Ser Gly Gln Arg Gly Ile
               300                 305                 310
   aag aaa aag aaa ctg ggc gga acc aag gat gag aaa ccc acc gct aag     1134
   Lys Lys Lys Lys Leu Gly Gly Thr Lys Asp Glu Lys Pro Thr Ala Lys
           315                 320                 325
   gat gct gtt gta aag gcc gat tct gct gtg aag gaa gcc gct aag cca     1182
   Asp Ala Val Val Lys Ala Asp Ser Ala Val Lys Glu Ala Ala Lys Pro
       330                 335                 340
   tga ctaaacgaac aaaaggactc atcctcaact acgccggagt ggtgttcatc          1235
   ctcttctggg gactagctcc cttctactgg atggttatca ccgcactgcg cgattccaag   1295
   cacacctttg acaccacccc atggccaacg cacgtcacct                         1335
<210> 8
   <211> 344
   <212> PRT
   <213> Corynebacterium glutamicum
<400> 8
   Met Ala Thr Phe Lys Gln Ala Arg Ser Ala Ala Trp Leu Ile Ala Pro
   1               5                   10                  15
   Ala Leu Val Val Leu Ala Val Val Ile Gly Tyr Pro Ile Val Arg Ala
               20                  25                  30
   Ile Trp Leu Ser Phe Gln Ala Asp Lys Gly Leu Asp Pro Thr Thr Gly
           35                  40                  45
   Leu Phe Thr Asp Gly Gly Phe Ala Gly Leu Asp Asn Tyr Leu Tyr Trp
       50                  55                  60
   Leu Thr Gln Arg Cys Met Gly Ser Asp Gly Thr Ile Arg Thr Cys Pro
   65                  70                  75                  80
   Pro Gly Thr Leu Ala Thr Asp Phe Trp Pro Ala Leu Arg Ile Thr Leu
                   85                  90                  95
   Phe Phe Thr Val Val Thr Val Gly Leu Glu Thr Ile Leu Gly Thr Ala
               100                 105                 110
   Met Ala Leu Ile Met Asn Lys Glu Phe Arg Gly Arg Ala Leu Val Arg
           115                 120                 125
   Ala Ala Ile Leu Ile Pro Trp Ala Ile Pro Thr Ala Val Thr Ala Lys
       130                 135                 140
   Leu Trp Gln Phe Ile Phe Ala Pro Gln Gly Ile Ile Asn Ser Met Phe
   145                 150                 155                 160
   Gly Leu Ser Val Ser Trp Thr Thr Asp Pro Trp Ala Ala Arg Ala Ala
                   165                 170                 175
   Val Ile Leu Ala Asp Val Trp Lys Thr Thr Pro Phe Met Ala Leu Leu
               180                 185                 190
   Ile Leu Ala Gly Leu Gln Met Ile Pro Lys Glu Thr Tyr Glu Ala Ala
           195                 200                 205
   Arg Val Asp Gly Ala Thr Ala Trp Gln Gln Phe Thr Lys Ile Thr Leu
       210                 215                 220
   Pro Leu Val Arg Pro Ala Leu Met Val Ala Val Leu Phe Arg Thr Leu
   225                 230                 235                 240
   Asp Ala Leu Arg Met Tyr Asp Leu Pro Val Ile Met Ile Ser Ser Ser
                   245                 250                 255
   Ser Asn Ser Pro Thr Ala Val Ile Ser Gln Leu Val Val Glu Asp Met
               260                 265                 270
   Arg Gln Asn Asn Phe Asn Ser Ala Ser Ala Leu Ser Thr Leu Ile Phe
           275                 280                 285
   Leu Leu Ile Phe Phe Val Ala Phe Ile Met Ile Arg Phe Leu Gly Ala
       290                 295                 300
   Asp Val Ser Gly Gln Arg Gly Ile Lys Lys Lys Lys Leu Gly Gly Thr
   305                 310                 315                 320
   Lys Asp Glu Lys Pro Thr Ala Lys Asp Ala Val Val Lys Ala Asp Ser
                   325                 330                 335
   Ala Val Lys Glu Ala Ala Lys Pro
               340
<210> 9
   <211> 1137
   <212> DNA
   <213> Corynebacterium glutamicum
<220>
   <221> CDS
   <222> (151)..(987)
   <223> integrales Membranprotein (Permease) des ABC-Transporters mit der Aktivität eines Trehalose-Importers
<400> 9
   ggcgttcatc atgatccgat tcctcggcgc agatgtttcg ggccaacgcg gaataaagaa      60
   aaagaaactg ggcggaacca aggatgagaa acccaccgct aaggatgctg ttgtaaaggc     120
   cgattctgct gtgaaggaag ccgctaagcc atg act aaa cga aca aaa gga ctc      174
                                    Met Thr Lys Arg Thr Lys Gly Leu
                                    1               5
   atc ctc aac tac gcc gga gtg gtg ttc atc ctc ttc tgg gga cta gct       222
   Ile Leu Asn Tyr Ala Gly Val Val Phe Ile Leu Phe Trp Gly Leu Ala
       10                  15                  20
   ccc ttc tac tgg atg gtt atc acc gca ctg cgc gat tcc aag cac acc       270
   Pro Phe Tyr Trp Met Val Ile Thr Ala Leu Arg Asp Ser Lys His Thr
   25                  30                  35                  40
   ttt gac acc acc cca tgg cca acg cac gtc acc ttg gat aac ttc cgg       318
   Phe Asp Thr Thr Pro Trp Pro Thr His Val Thr Leu Asp Asn Phe Arg
                   45                  50                  55
   gac gca ctg gcc acc gac aaa ggc aac aac ttc ctc gca gcc att ggc       366
   Asp Ala Leu Ala Thr Asp Lys Gly Asn Asn Phe Leu Ala Ala Ile Gly
               60                  65                  70
   aac tca ctg gtc atc agc gtc acc aca aca gcg atc gct gtt ctc gtg       414
   Asn Ser Leu Val Ile Ser Val Thr Thr Thr Ala Ile Ala Val Leu Val
           75                  80                  85
   gga gtg ttc acc gcc tac gct cta gcc cga ctg gaa ttc ccg ggc aaa       462
   Gly Val Phe Thr Ala Tyr Ala Leu Ala Arg Leu Glu Phe Pro Gly Lys
       90                  95                  100
   ggc att gtc acc ggc atc atc ttg gca gcc tcc atg ttc ccc ggc atc        510
   Gly Ile Val Thr Gly Ile Ile Leu Ala Ala Ser Met Phe Pro Gly Ile
   105                 110                 115                 120
   gcc ctg gtc act ccg ctg ttc cag ctc ttc ggt gac ctc aac tgg atc        558
   Ala Leu Val Thr Pro Leu Phe Gln Leu Phe Gly Asp Leu Asn Trp Ile
                   125                 130                 135
   ggc acc tac caa gcg ctg att atc ccg aac att tcc ttc gcg cta cct        606
   Gly Thr Tyr Gln Ala Leu Ile Ile Pro Asn Ile Ser Phe Ala Leu Pro
               140                 145                 150
   ctg acg atc tac acg ctc gta tcc ttc ttc agg caa ctg ccc tgg gaa        654
   Leu Thr Ile Tyr Thr Leu Val Ser Phe Phe Arg Gln Leu Pro Trp Glu
           155                 160                 165
   ctc gaa gaa tca gca cgt gtc gac ggc gcc aca cgt ggc caa gcc ttc        702
   Leu Glu Glu Ser Ala Arg Val Asp Gly Ala Thr Arg Gly Gln Ala Phe
       170                 175                 180
   cgc atg atc ctg ctt cct cta gca gcg ccc gca cta ttt acc acc gcg        750
   Arg Met Ile Leu Leu Pro Leu Ala Ala Pro Ala Leu Phe Thr Thr Ala
   185                 190                 195                 200
   atc ctc gca ttc att gca acg tgg aac gaa ttc atg ctg gcc cgc caa        798
   Ile Leu Ala Phe Ile Ala Thr Trp Asn Glu Phe Met Leu Ala Arg Gln
                   205                 210                 215
   cta tcc aac acc tcc aca gag cca gtg acc gtt gcg atc gca agg ttc        846
   Leu Ser Asn Thr Ser Thr Glu Pro Val Thr Val Ala Ile Ala Arg Phe
               220                 225                 230
   acc gga cca agc tcc ttc gaa tac ccc tac gcc tct gtc atg gca gcg        894
   Thr Gly Pro Ser Ser Phe Glu Tyr Pro Tyr Ala Ser Val Met Ala Ala
           235                 240                 245
   gga gct ttg gtg acc atc cca ctg atc atc atg gtt ctc atc ttc caa        942
   Gly Ala Leu Val Thr Ile Pro Leu Ile Ile Met Val Leu Ile Phe Gln
       250                 255                 260
   cgc cgc atc gtc tcc gga ctc acc gca ggt ggc gtg aaa gcc tag            987
   Arg Arg Ile Val Ser Gly Leu Thr Ala Gly Gly Val Lys Ala
   265                 270                 275
   actagatact catgagtgct gataaatccc aggaccaatc cgaatcgcaa cgcaaagggc     1047
   ttcaacccga agcgctgctt ggattcctgg gatttttctc attcctcgcc gtcatccagg     1107
   cagtcatcaa cgtgttacgc cccgaacctg                                      1137
<210> 10
   <211> 278
   <212> PRT
   <213> Corynebacterium glutamicum
<400> 10
   Met Thr Lys Arg Thr Lys Gly Leu Ile Leu Asn Tyr Ala Gly Val Val
   1               5                   10                  15
   Phe Ile Leu Phe Trp Gly Leu Ala Pro Phe Tyr Trp Met Val Ile Thr
   20 25 30
   Ala Leu Arg Asp Ser Lys His Thr Phe Asp Thr Thr Pro Trp Pro Thr
   35 40 45
   His Val Thr Leu Asp Asn Phe Arg Asp Ala Leu Ala Thr Asp Lys Gly
   50 55 60
   Asn Asn Phe Leu Ala Ala Ile Gly Asn Ser Leu Val Ile Ser Val Thr
   65 70 75 80
   Thr Thr Ala Ile Ala Val Leu Val Gly Val Phe Thr Ala Tyr Ala Leu
   85 90 95
   Ala Arg Leu Glu Phe Pro Gly Lys Gly Ile Val Thr Gly Ile Ile Leu
   100 105 110
   Ala Ala Ser Met Phe Pro Gly Ile Ala Leu Val Thr Pro Leu Phe Gln
   115 120 125
   Leu Phe Gly Asp Leu Asn Trp Ile Gly Thr Tyr Gln Ala Leu Ile Ile
   130 135 140
   Pro Asn Ile Ser Phe Ala Leu Pro Leu Thr Ile Tyr Thr Leu Val Ser
   145 150 155 160
   Phe Phe Arg Gln Leu Pro Trp Glu Leu Glu Glu Ser Ala Arg Val Asp
   165 170 175
   Gly Ala Thr Arg Gly Gln Ala Phe Arg Met Ile Leu Leu Pro Leu Ala
   180 185 190
   Ala Pro Ala Leu Phe Thr Thr Ala Ile Leu Ala Phe Ile Ala Thr Trp
   195 200 205
   Asn Glu Phe Met Leu Ala Arg Gln Leu Ser Asn Thr Ser Thr Glu Pro
   210 215 220
   Val Thr Val Ala Ile Ala Arg Phe Thr Gly Pro Ser Ser Phe Glu Tyr
   225 230 235 240
   Pro Tyr Ala Ser Val Met Ala Ala Gly Ala Leu Val Thr Ile Pro Leu
   245 250 255
   Ile Ile Met Val Leu Ile Phe Gln Arg Arg Ile Val Ser Gly Leu Thr
                   260                 265                 270
   Ala Gly Gly Val Lys Ala
           275
<210> 11
   <211> 525
   <212> DNA
   <213> Corynebacterium glutamicum
<220>
   <221> CDS
   <222> (151)..(375)
   <223> hypothetisches Protein
<400> 11
   cggaccaagc tccttcgaat acccctacgc ctctgtcatg gcagcgggag ctttggtgac      60
   catcccactg atcatcatgg ttctcatctt ccaacgccgc atcgtctccg gactcaccgc     120
   aggtggcgtg aaagcctaga ctagatactc atg agt gct gat aaa tcc cag gac     174
                                    Met Ser Ala Asp Lys Ser Gln Asp
                                    1               5
   caa tcc gaa tcg caa cgc aaa ggg ctt caa ccc gaa gcg ctg ctt gga      222
   Gln Ser Glu Ser Gln Arg Lys Gly Leu Gln Pro Glu Ala Leu Leu Gly
       10                  15                  20
   ttc ctg gga ttt ttc tca ttc ctc gcc gtc atc cag gca gtc atc aac      270
   Phe Leu Gly Phe Phe Ser Phe Leu Ala Val Ile Gln Ala Val Ile Asn
   25                  30                  35                  40
   gtg tta cgc ccc gaa cct gcc gtg tgg cca gct ctt ctc gcg ctc gtt      318
   Val Leu Arg Pro Glu Pro Ala Val Trp Pro Ala Leu Leu Ala Leu Val
                   45                  50                  55
   tta gta atc gcc aca gtg tca gta tgg agg gct tgg cga aag cgc cgc      366
   Leu Val Ile Ala Thr Val Ser Val Trp Arg Ala Trp Arg Lys Arg Arg
               60                  65                  70
   cct aat taa agttcctgcg ccaacgccac gataattcca gatggcccgc               415
   Pro Asn
   gcagataaca caatcggtag gtgtcctcgt aatttgcgat cccatctagt ggttccgcac     475
   cgatatgttc gatcgtttcc tcaatatcat ccaccgcaaa catcaaacgg                525
<210> 12
   <211> 74
   <212> PRT
   <213> Corynebacterium glutamicum
<400> 12
   Met Ser Ala Asp Lys Ser Gln Asp Gln Ser Glu Ser Gln Arg Lys Gly
   1               5                   10                  15
   Leu Gln Pro Glu Ala Leu Leu Gly Phe Leu Gly Phe Phe Ser Phe Leu
               20                  25                  30
   Ala Val Ile Gln Ala Val Ile Asn Val Leu Arg Pro Glu Pro Ala Val
           35                  40                  45
   Trp Pro Ala Leu Leu Ala Leu Val Leu Val Ile Ala Thr Val Ser Val
       50                  55                  60
   Trp Arg Ala Trp Arg Lys Arg Arg Pro Asn
   65                  70
<210> 13
   <211> 1305
   <212> DNA
   <213> Corynebacterium efficiens
<220>
   <221> CDS
   <222> (151)..(1152)
   <223> ATP-bindendes und -hydrolysierendes (ATPase) Protein des ABC-Transporters mit der Aktivität eines Trehalose-Importers
<400> 13
   atggggggtt ccgcggtggt ggttgccggg atggtggata cccagcgtct ggatcagatc     60
   gcgaccgcgg agaaggtcac cgcacgggtc tgagaatgtg gccggcccac aggtacacaa    120
   ctgggtgtga cactgctaac ttcataggtt atg gcc act gtt tcc ttt gac aaa     174
                                     Met Ala Thr Val Ser Phe Asp Lys
                                     1               5
   gtc tcc atc cgg tac ccc ggt gcg gag cgc ccc acc gtc cat gag ctc      222
   Val Ser Ile Arg Tyr Pro Gly Ala Glu Arg Pro Thr Val His Glu Leu
       10                  15                  20
   gac ctc gag ata gcc gac ggt gaa ttc ctc gta ctc gtc ggc ccg tcg      270
   Asp Leu Glu Ile Ala Asp Gly Glu Phe Leu Val Leu Val Gly Pro Ser
   25                  30                  35                  40
   ggg tgt gga aaa tca acc acg ctg cga gcg ctc gcc ggg ctc gag gag      318
   Gly Cys Gly Lys Ser Thr Thr Leu Arg Ala Leu Ala Gly Leu Glu Glu
                   45                  50                  55
   gtc gaa tcc ggt gtg atc cgc atc gac ggg cag gat gtc acc agt cag      366
   Val Glu Ser Gly Val Ile Arg Ile Asp Gly Gln Asp Val Thr Ser Gln
               60                  65                  70
   gaa cct gcg gag cgt gac atc gcg atg gtg ttc cag aac tac gcc ctc      414
   Glu Pro Ala Glu Arg Asp Ile Ala Met Val Phe Gln Asn Tyr Ala Leu
           75                  80                  85
   tac ccc cac atg tcc gtg gcg cgg aat atg ggt ttc gcc ctc aaa ctg      462
   Tyr Pro His Met Ser Val Ala Arg Asn Met Gly Phe Ala Leu Lys Leu
       90                  95                  100
   gcc aaa ctg ccc cag gcg gag atc gac gcc aag gtc cgg gag gcc gcc      510
   Ala Lys Leu Pro Gln Ala Glu Ile Asp Ala Lys Val Arg Glu Ala Ala
   105                 110                 115                 120
   gag atc ctc ggc ctc acc gac tac ctg gac cgc aaa ccg aag gac ctc       558
   Glu Ile Leu Gly Leu Thr Asp Tyr Leu Asp Arg Lys Pro Lys Asp Leu
                   125                 130                 135
   tcc ggt ggt cag cgc cag cgt gtg gcc atg ggc cgg gcc ctg gtg cgc       606
   Ser Gly Gly Gln Arg Gln Arg Val Ala Met Gly Arg Ala Leu Val Arg
               140                 145                 150
   aac ccg aag gtc ttc ctc atg gat gag ccc ctg tcc aac ctc gat gcc       654
   Asn Pro Lys Val Phe Leu Met Asp Glu Pro Leu Ser Asn Leu Asp Ala
           155                 160                 165
   aaa ctg cgt gtg cag acg cgc gcg gaa gtt gcc gca ctg cag cgt cgc       702
   Lys Leu Arg Val Gln Thr Arg Ala Glu Val Ala Ala Leu Gln Arg Arg
       170                 175                 180
   ctg ggt acc acc acc gtc tat gtc acc cat gat cag gtg gag gcc atg       750
   Leu Gly Thr Thr Thr Val Tyr Val Thr His Asp Gln Val Glu Ala Met
   185                 190                 195                 200
   acg atg ggc gac cgc gtc gcg gtg ctc aag gac gga ctg ctc cag cag       798
   Thr Met Gly Asp Arg Val Ala Val Leu Lys Asp Gly Leu Leu Gln Gln
                   205                 210                 215
   gtg gcc cca ccc cgg gag ctc tac gac acc ccg gtc aat gcg ttc gtc       846
   Val Ala Pro Pro Arg Glu Leu Tyr Asp Thr Pro Val Asn Ala Phe Val
               220                 225                 230
   gcc ggt ttc atc ggc tcc cca tcg atg aat ctc ttc ccc tac gac ggt       894
   Ala Gly Phe Ile Gly Ser Pro Ser Met Asn Leu Phe Pro Tyr Asp Gly
           235                 240                 245
   gtg acc ctg ggt gtg cgt ccg gaa tcc atg ctg gtg gtc acc ggc gag       942
   Val Thr Leu Gly Val Arg Pro Glu Ser Met Leu Val Val Thr Gly Glu
       250                 255                 260
   gcc ccg gcc ggt tac acc gtg gtg gac ggg acg gtg gac atc gtc gag       990
   Ala Pro Ala Gly Tyr Thr Val Val Asp Gly Thr Val Asp Ile Val Glu
   265                 270                 275                 280
   gag ctc ggt tcc gag tcc tat gtt tac gcc acc tgc gac ggc aac cgc      1038
   Glu Leu Gly Ser Glu Ser Tyr Val Tyr Ala Thr Cys Asp Gly Asn Arg
                   285                 290                 295
   ctg gtg gcg cgc tgg gag gac gcc gtg gtg ccc gcg ccg ggt gac cgg      1086
   Leu Val Ala Arg Trp Glu Asp Ala Val Val Pro Ala Pro Gly Asp Arg
               300                 305                 310
   gtg cgg ttc gcc ttc gac ccg gcg ggt tca cac cgt ttc gac ccg acc      1134
   Val Arg Phe Ala Phe Asp Pro Ala Gly Ser His Arg Phe Asp Pro Thr
           315                 320                 325
   agc ggt tac cgg ctc agc tgagggtgac cacggtgggg gtcgcggcgt             1182
   Ser Gly Tyr Arg Leu Ser
       330
   cgtcaagcac tgcccccggc acgggggtga tttgaggtaa accggtgcgg gaaagtggcg   1242
   aaagtcatta gattgaagtc acctgttgca gagaaaggtg acccaccatg tccaagtttt   1302
   ccc                                                                  1305
<210> 14
   <211> 334
   <212> PRT
   <213> Corynebacterium efficiens
<400> 14
   Met Ala Thr Val Ser Phe Asp Lys Val Ser Ile Arg Tyr Pro Gly Ala
   1               5                   10                  15
   Glu Arg Pro Thr Val His Glu Leu Asp Leu Glu Ile Ala Asp Gly Glu
               20                  25                  30
   Phe Leu Val Leu Val Gly Pro Ser Gly Cys Gly Lys Ser Thr Thr Leu
           35                  40                  45
   Arg Ala Leu Ala Gly Leu Glu Glu Val Glu Ser Gly Val Ile Arg Ile
       50                  55                  60
   Asp Gly Gln Asp Val Thr Ser Gln Glu Pro Ala Glu Arg Asp Ile Ala
   65                  70                  75                  80
   Met Val Phe Gln Asn Tyr Ala Leu Tyr Pro His Met Ser Val Ala Arg
                   85                  90                  95
   Asn Met Gly Phe Ala Leu Lys Leu Ala Lys Leu Pro Gln Ala Glu Ile
               100                 105                 110
   Asp Ala Lys Val Arg Glu Ala Ala Glu Ile Leu Gly Leu Thr Asp Tyr
           115                 120                 125
   Leu Asp Arg Lys Pro Lys Asp Leu Ser Gly Gly Gln Arg Gln Arg Val
       130                 135                 140
   Ala Met Gly Arg Ala Leu Val Arg Asn Pro Lys Val Phe Leu Met Asp
   145                 150                 155                 160
   Glu Pro Leu Ser Asn Leu Asp Ala Lys Leu Arg Val Gln Thr Arg Ala
                   165                 170                 175
   Glu Val Ala Ala Leu Gln Arg Arg Leu Gly Thr Thr Thr Val Tyr Val
               180                 185                 190
   Thr His Asp Gln Val Glu Ala Met Thr Met Gly Asp Arg Val Ala Val
           195                 200                 205
   Leu Lys Asp Gly Leu Leu Gln Gln Val Ala Pro Pro Arg Glu Leu Tyr
       210                 215                 220
   Asp Thr Pro Val Asn Ala Phe Val Ala Gly Phe Ile Gly Ser Pro Ser
   225                 230                 235                 240
   Met Asn Leu Phe Pro Tyr Asp Gly Val Thr Leu Gly Val Arg Pro Glu
                   245                 250                 255
   Ser Met Leu Val Val Thr Gly Glu Ala Pro Ala Gly Tyr Thr Val Val
               260                 265                 270
   Asp Gly Thr Val Asp Ile Val Glu Glu Leu Gly Ser Glu Ser Tyr Val
           275                 280                 285
   Tyr Ala Thr Cys Asp Gly Asn Arg Leu Val Ala Arg Trp Glu Asp Ala
       290                 295                 300
   Val Val Pro Ala Pro Gly Asp Arg Val Arg Phe Ala Phe Asp Pro Ala
   305                 310                 315                 320
   Gly Ser His Arg Phe Asp Pro Thr Ser Gly Tyr Arg Leu Ser
                   325                 330
<210> 15
   <211> 1605
   <212> DNA
   <213> Corynebacterium efficiens
<220>
   <221> CDS
   <222> (151)..(1455)
   <223> periplasmatisches (oder Lipoprotein) Substrat-bindendes Protein des ABC-Transporters mit der Aktivität eines Trehalose-Importers
<400> 15
   ttaccggctc agctgagggt gaccacggtg ggggtcgcgg cgtcgtcaag cactgccccc      60
   ggcacggggg tgatttgagg taaaccggtg cgggaaagtg gcgaaagtca ttagattgaa     120
   gtcacctgtt gcagagaaag gtgacccacc atg tcc aag ttt tcc cgc aag acc      174
                                    Met Ser Lys Phe Ser Arg Lys Thr
                                    1               5
   ggc gta tcg ctg gcc gca acc agc ctg atc gcc gcc atc gcc ctg gcc      222
   Gly Val Ser Leu Ala Ala Thr Ser Leu Ile Ala Ala Ile Ala Leu Ala
       10                  15                  20
   ggt tgt ggc aat gac acc gcc gac gat gcc ggc acg acc gac acc agc      270
   Gly Cys Gly Asn Asp Thr Ala Asp Asp Ala Gly Thr Thr Asp Thr Ser
   25                  30                  35                  40
   acc aat gac acc gaa gcc acc acc gcc gcc tcg ggt gag gag ggc cgc      318
   Thr Asn Asp Thr Glu Ala Thr Thr Ala Ala Ser Gly Glu Glu Gly Arg
                   45                  50                  55
   ggc ccg att acc ttc gcc atg ggc aag aac gac acc gac aag atc att       366
   Gly Pro Ile Thr Phe Ala Met Gly Lys Asn Asp Thr Asp Lys Ile Ile
               60                  65                  70
   ccc gtg atc gag aag tgg aac gag gag aac ccc gac cag gag gtg acc       414
   Pro Val Ile Glu Lys Trp Asn Glu Glu Asn Pro Asp Gln Glu Val Thr
           75                  80                  85
   ctc aac gaa ctc gcc ggt gag gcc gac gcc cag cgc gag acc ctc gtg       462
   Leu Asn Glu Leu Ala Gly Glu Ala Asp Ala Gln Arg Glu Thr Leu Val
       90                  95                  100
   cag tcc ctc cag gcc ggc aac tcc gat tat gac gtc atg gcc ctc gat       510
   Gln Ser Leu Gln Ala Gly Asn Ser Asp Tyr Asp Val Met Ala Leu Asp
   105                 110                 115                 120
   gtc atc tgg acc gcc gac ttc gcc gcc aac cag tgg ctc gcg ccg ctt       558
   Val Ile Trp Thr Ala Asp Phe Ala Ala Asn Gln Trp Leu Ala Pro Leu
                   125                 130                 135
   gag ggg gaa ctc gag gtc gac acc tcc ggg ctg ctt gag gcc acc gtg       606
   Glu Gly Glu Leu Glu Val Asp Thr Ser Gly Leu Leu Glu Ala Thr Val
               140                 145                 150
   gaa tcc gcc aca tac atg gac acc ctc tac gca ctg ccg cag aac acc       654
   Glu Ser Ala Thr Tyr Met Asp Thr Leu Tyr Ala Leu Pro Gln Asn Thr
           155                 160                 165
   aac ggc cag ctg ctc tac cgc aac acc gag atc atc ccc gag gcc ccg       702
   Asn Gly Gln Leu Leu Tyr Arg Asn Thr Glu Ile Ile Pro Glu Ala Pro
       170                 175                 180
   gag aac tgg gct gac ctc gtc gaa tcc tgc acc ctg gcg gag gag gcc       750
   Glu Asn Trp Ala Asp Leu Val Glu Ser Cys Thr Leu Ala Glu Glu Ala
   185                 190                 195                 200
   gag gtt gac tgc ctg acc acc cag ctc aag cag tac gag ggc ctg acc       798
   Glu Val Asp Cys Leu Thr Thr Gln Leu Lys Gln Tyr Glu Gly Leu Thr
                   205                 210                 215
   gtc aac acc atc ggc ttc atg gag ggc tgg ggc ggt tcc gtc ctg gac       846
   Val Asn Thr Ile Gly Phe Met Glu Gly Trp Gly Gly Ser Val Leu Asp
               220                 225                 230
   gat gac ggc acc acc gtg gtc gtc gac tcc gac gag tcg aag gag ggc       894
   Asp Asp Gly Thr Thr Val Val Val Asp Ser Asp Glu Ser Lys Glu Gly
           235                 240                 245
   ctg cag gcg ctt gtc gac gcc tac gag gac ggc acc atc tcg tcc gcg       942
   Leu Gln Ala Leu Val Asp Ala Tyr Glu Asp Gly Thr Ile Ser Ser Ala
       250                 255                 260
   tcc acc gca gcc acc gag gag gag acc aac ctg gcc ttc acc gcc ggt       990
   Ser Thr Ala Ala Thr Glu Glu Glu Thr Asn Leu Ala Phe Thr Ala Gly
   265                 270                 275                 280
   gag acc gcc tac gcc atc aac tgg ccg tac atg tac acc aac gcc gag      1038
   Glu Thr Ala Tyr Ala Ile Asn Trp Pro Tyr Met Tyr Thr Asn Ala Glu
                   285                 290                 295
   gac tcc gag gcc acc gcc ggc aag ttc gag gtc cag cca ctc gtg ggc      1086
   Asp Ser Glu Ala Thr Ala Gly Lys Phe Glu Val Gln Pro Leu Val Gly
               300                 305                 310
   aag gac ggc gtg ggt gtg tcc acc ctc ggt ggc tac aac aac gcc atc      1134
   Lys Asp Gly Val Gly Val Ser Thr Leu Gly Gly Tyr Asn Asn Ala Ile
           315                 320                 325
   aac atc aac tcg gag aac aag gca acc gcc cgc gac ttc atc gag ttc      1182
   Asn Ile Asn Ser Glu Asn Lys Ala Thr Ala Arg Asp Phe Ile Glu Phe
       330                 335                 340
   atc atc aac gag gag aac cag acc tgg ttc gcc gac aac tcc ttc cca      1230
   Ile Ile Asn Glu Glu Asn Gln Thr Trp Phe Ala Asp Asn Ser Phe Pro
   345                 350                 355                 360
   ccg gtg ctc gcc tcc atc tac gac gat gag gaa ctg atc gag cag tac      1278
   Pro Val Leu Ala Ser Ile Tyr Asp Asp Glu Glu Leu Ile Glu Gln Tyr
                   365                 370                 375
   cca tac ctg ccc gcg ctg aag gaa tcc ctg gag aac gcg gca ccg cgt      1326
   Pro Tyr Leu Pro Ala Leu Lys Glu Ser Leu Glu Asn Ala Ala Pro Arg
               380                 385                 390
   ccg gtc tcc ccg ttc tac acc gcc atc tcc aag gcc atc cag gac aac      1374
   Pro Val Ser Pro Phe Tyr Thr Ala Ile Ser Lys Ala Ile Gln Asp Asn
           395                 400                 405
   gcc tac gca gcc atc aac ggc aac gtc gac gtc gac cag gcc acc gct      1422
   Ala Tyr Ala Ala Ile Asn Gly Asn Val Asp Val Asp Gln Ala Thr Ala
       410                 415                 420
   gac atg aag gca gca atc gag aac gcc tcc tag agcgacaggg acacccccac    1475
   Asp Met Lys Ala Ala Ile Glu Asn Ala Ser
   425                 430
   cccatgacac tccggtcacc caccaggtga ccggggtttt gtcatagtct gggcgggaac    1535
   aggtgttgtc acccaactgc tttcccagtg tcggatcacg tgtctgctca agtgtcggat    1595
   ccaacgtccc                                                           1605
<210> 16
   <211> 434
   <212> PRT
   <213> Corynebacterium efficiens
<400> 16
   Met Ser Lys Phe Ser Arg Lys Thr Gly Val Ser Leu Ala Ala Thr Ser
   1               5                   10                  15
   Leu Ile Ala Ala Ile Ala Leu Ala Gly Cys Gly Asn Asp Thr Ala Asp
               20                  25                  30
   Asp Ala Gly Thr Thr Asp Thr Ser Thr Asn Asp Thr Glu Ala Thr Thr
           35                  40                  45
   Ala Ala Ser Gly Glu Glu Gly Arg Gly Pro Ile Thr Phe Ala Met Gly
       50                  55                  60
   Lys Asn Asp Thr Asp Lys Ile Ile Pro Val Ile Glu Lys Trp Asn Glu
   65 70 75 80
   Glu Asn Pro Asp Gln Glu Val Thr Leu Asn Glu Leu Ala Gly Glu Ala
   85 90 95
   Asp Ala Gln Arg Glu Thr Leu Val Gln Ser Leu Gln Ala Gly Asn Ser
   100 105 110
   Asp Tyr Asp Val Met Ala Leu Asp Val Ile Trp Thr Ala Asp Phe Ala
   115 120 125
   Ala Asn Gln Trp Leu Ala Pro Leu Glu Gly Glu Leu Glu Val Asp Thr
   130 135 140
   Ser Gly Leu Leu Glu Ala Thr Val Glu Ser Ala Thr Tyr Met Asp Thr
   145 150 155 160
   Leu Tyr Ala Leu Pro Gln Asn Thr Asn Gly Gln Leu Leu Tyr Arg Asn
   165 170 175
   Thr Glu Ile Ile Pro Glu Ala Pro Glu Asn Trp Ala Asp Leu Val Glu
   180 185 190
   Ser Cys Thr Leu Ala Glu Glu Ala Glu Val Asp Cys Leu Thr Thr Gln
   195 200 205
   Leu Lys Gln Tyr Glu Gly Leu Thr Val Asn Thr Ile Gly Phe Met Glu
   210 215 220
   Gly Trp Gly Gly Ser Val Leu Asp Asp Asp Gly Thr Thr Val Val Val
   225 230 235 240
   Asp Ser Asp Glu Ser Lys Glu Gly Leu Gln Ala Leu Val Asp Ala Tyr
   245 250 255
   Glu Asp Gly Thr Ile Ser Ser Ala Ser Thr Ala Ala Thr Glu Glu Glu
   260 265 270
   Thr Asn Leu Ala Phe Thr Ala Gly Glu Thr Ala Tyr Ala Ile Asn Trp
   275 280 285
   Pro Tyr Met Tyr Thr Asn Ala Glu Asp Ser Glu Ala Thr Ala Gly Lys
   290 295 300
   Phe Glu Val Gln Pro Leu Val Gly Lys Asp Gly Val Gly Val Ser Thr
   305 310 315 320
   Leu Gly Gly Tyr Asn Asn Ala Ile Asn Ile Asn Ser Glu Asn Lys Ala
                   325                 330                 335
   Thr Ala Arg Asp Phe Ile Glu Phe Ile Ile Asn Glu Glu Asn Gln Thr
               340                 345                 350
   Trp Phe Ala Asp Asn Ser Phe Pro Pro Val Leu Ala Ser Ile Tyr Asp
           355                 360                 365
   Asp Glu Glu Leu Ile Glu Gln Tyr Pro Tyr Leu Pro Ala Leu Lys Glu
       370                 375                 380
   Ser Leu Glu Asn Ala Ala Pro Arg Pro Val Ser Pro Phe Tyr Thr Ala
   385                 390                 395                 400
   Ile Ser Lys Ala Ile Gln Asp Asn Ala Tyr Ala Ala Ile Asn Gly Asn
                   405                 410                 415
   Val Asp Val Asp Gln Ala Thr Ala Asp Met Lys Ala Ala Ile Glu Asn
               420                 425                 430
   Ala Ser
<210> 17
   <211> 786
   <212> DNA
   <213> Corynebacterium efficiens
<220>
   <221> CDS
   <222> (151)..(636)
   <223> Funktion unbekannt
<400> 17
   cccccacccc atgacactcc ggtcacccac caggtgaccg gggttttgtc atagtctggg      60
   cgggaacagg tgttgtcacc caactgcttt cccagtgtcg gatcacgtgt ctgctcaagt     120
   gtcggatcca acgtccctga ggaggacccc atg tca cac cag cgc tcc ccc gag      174
                                    Met Ser His Gln Arg Ser Pro Glu
                                    1               5
   aca ccc gag atg ctg tcc tac acc atc tcc gga ttc atc tcc cgg tgc       222
   Thr Pro Glu Met Leu Ser Tyr Thr Ile Ser Gly Phe Ile Ser Arg Cys
       10                  15                  20
   ccc gtc cag gtc tat gag gcc atc gtc gat cac cgt caa ctc tcc cga       270
   Pro Val Gln Val Tyr Glu Ala Ile Val Asp His Arg Gln Leu Ser Arg
   25                  30                  35                  40
   cat ttc gcc acc ggc ggg gca cag ggc agg atg agc gcc ggc gcg acg       318
   His Phe Ala Thr Gly Gly Ala Gln Gly Arg Met Ser Ala Gly Ala Thr
                   45                  50                  55
   gtg acc tgg gac ttc gac gat ggg tcc ggc ccc tgc acc gtc gag gtc       366
   Val Thr Trp Asp Phe Asp Asp Gly Ser Gly Pro Cys Thr Val Glu Val
               60                  65                  70
   ctc cag gcg gcg cat tcc cgg tgt ctg atc ctg gag tgg tcc agc ccc       414
   Leu Gln Ala Ala His Ser Arg Cys Leu Ile Leu Glu Trp Ser Ser Pro
           75                  80                  85
   gat gcg ggt gaa ccc gcc ggg agc acc acg gtg gag ttc gcc ttc gaa       462
   Asp Ala Gly Glu Pro Ala Gly Ser Thr Thr Val Glu Phe Ala Phe Glu
       90                  95                  100
   ccc gcc aat gac ttc acc cgc acc aaa ctg acc atc acg gaa tca ggg       510
   Pro Ala Asn Asp Phe Thr Arg Thr Lys Leu Thr Ile Thr Glu Ser Gly
   105                 110                 115                 120
   tgg cct ccc acc acc gcc ggc acc agg aaa gcg ctg cgc gaa tgc cac       558
   Trp Pro Pro Thr Thr Ala Gly Thr Arg Lys Ala Leu Arg Glu Cys His
                   125                 130                 135
   cgg tgg acc acc atg ctc acc ggt ctg aag gcc tgg ttg gaa cac ggg       606
   Arg Trp Thr Thr Met Leu Thr Gly Leu Lys Ala Trp Leu Glu His Gly
               140                 145                 150
   gtg gtc ctc ggc agg gat cta cat cgc tag ggagccttgt taaccggagg         656
   Val Val Leu Gly Arg Asp Leu His Arg
           155                 160
   tagagggtgg aacggaggtg gggttactgt tccctcactg acaccagggt tctatgatcc     716
   aagtaacact tttcctgatt tctcttcttt tcccatccat cccctctacc ccaaggagca     776
   ctggtgacat                                                            786
<210> 18
   <211> 161
   <212> PRT
   <213> Corynebacterium efficiens
<400> 18
   Met Ser His Gln Arg Ser Pro Glu Thr Pro Glu Met Leu Ser Tyr Thr
   1               5                   10                  15
   Ile Ser Gly Phe Ile Ser Arg Cys Pro Val Gln Val Tyr Glu Ala Ile
               20                  25                  30
   Val Asp His Arg Gln Leu Ser Arg His Phe Ala Thr Gly Gly Ala Gln
           35                  40                  45
   Gly Arg Met Ser Ala Gly Ala Thr Val Thr Trp Asp Phe Asp Asp Gly
       50                  55                  60
   Ser Gly Pro Cys Thr Val Glu Val Leu Gln Ala Ala His Ser Arg Cys
   65                  70                  75                  80
   Leu Ile Leu Glu Trp Ser Ser Pro Asp Ala Gly Glu Pro Ala Gly Ser
                   85                  90                  95
   Thr Thr Val Glu Phe Ala Phe Glu Pro Ala Asn Asp Phe Thr Arg Thr
               100                 105                 110
   Lys Leu Thr Ile Thr Glu Ser Gly Trp Pro Pro Thr Thr Ala Gly Thr
           115                 120                 125
   Arg Lys Ala Leu Arg Glu Cys His Arg Trp Thr Thr Met Leu Thr Gly
       130                 135                 140
   Leu Lys Ala Trp Leu Glu His Gly Val Val Leu Gly Arg Asp Leu His
   145                 150                 155                 160
   Arg
<210> 19
   <211> 1347
   <212> DNA
   <213> Corynebacterium efficiens
<220>
   <221> CDS
   <222> (151)..(1197)
   <223> integrales Membranprotein (Permease) des ABC-Transporters mit der Aktivität eines Trehalose-Importers
<400> 19
   agggagcctt gttaaccgga ggtagagggt ggaacggagg tggggttact gttccctcac      60
   tgacaccagg gttctatgat ccaagtaaca cttttcctga tttctcttct tttcccatcc     120
   atcccctcta ccccaaggag cactggtgac atg gcc aag atg aaa cag gcg cga      174
                                    Met Ala Lys Met Lys Gln Ala Arg
                                    1               5
   tca gcc gca tgg ttg atc gcg cca gcc atg att gtc ctg acg gtg gtg       222
   Ser Ala Ala Trp Leu Ile Ala Pro Ala Met Ile Val Leu Thr Val Val
       10                  15                  20
   atc ggc tac ccc atc gtc cgt gcc gtc tgg ttg tcc ttc cag gcg gac       270
   Ile Gly Tyr Pro Ile Val Arg Ala Val Trp Leu Ser Phe Gln Ala Asp
   25                  30                  35                  40
   aag ggt ctc gat ccc acc acc ggg ttg ttc acc gac ggt ggt ttc gcc       318
   Lys Gly Leu Asp Pro Thr Thr Gly Leu Phe Thr Asp Gly Gly Phe Ala
                   45                  50                  55
   ggt ttc gac aat tac ctg tac tgg ctc acc caa cgc tgc atg tcc ccc       366
   Gly Phe Asp Asn Tyr Leu Tyr Trp Leu Thr Gln Arg Cys Met Ser Pro
               60                  65                  70
   gac ggc acc gtg ggt acc tgt ccg ccc ggt acc ctg gcc acc gac ttc       414
   Asp Gly Thr Val Gly Thr Cys Pro Pro Gly Thr Leu Ala Thr Asp Phe
   75 80 85
   tgg ccg gcc ctg cgc atc acc ctg ttc ttc acc gtg gtc acc gtc acc 462
   Trp Pro Ala Leu Arg Ile Thr Leu Phe Phe Thr Val Val Thr Val Thr
   90 95 100
   ctg gag acc atc ctg ggt atg gtc atg gcc ctg atc atg agc aag gag 510
   Leu Glu Thr Ile Leu Gly Met Val Met Ala Leu Ile Met Ser Lys Glu
   105 110 115 120
   ttc cgc ggc cgg gcc ctc gtc cgc gcc gcg gtc ctg atc ccg tgg gcg 558
   Phe Arg Gly Arg Ala Leu Val Arg Ala Ala Val Leu Ile Pro Trp Ala
   125 130 135
   atc ccg acg gcg gtc acc gcg aag ctg tgg cag ttc ctg ttc gcc cca 606
   Ile Pro Thr Ala Val Thr Ala Lys Leu Trp Gln Phe Leu Phe Ala Pro
   140 145 150
   cgg ggc atc atc aat gaa ctc ttc gga ctc aat atc agc tgg acc acc 654
   Arg Gly Ile Ile Asn Glu Leu Phe Gly Leu Asn Ile Ser Trp Thr Thr
   155 160 165
   gat ccg tgg gcg gca cgc gcc gcg gtc atc ctc gcc gat gtc tgg aag 702
   Asp Pro Trp Ala Ala Arg Ala Ala Val Ile Leu Ala Asp Val Trp Lys
   170 175 180
   acc acc ccg ttc atg gcg ctg ctc atc ctc gcc ggg ctg cag atg atc 750
   Thr Thr Pro Phe Met Ala Leu Leu Ile Leu Ala Gly Leu Gln Met Ile
   185 190 195 200
   ccc aag ggc acc tat gag gcc gcc cgt gtg gac ggg gcc agc gcc tgg 798
   Pro Lys Gly Thr Tyr Glu Ala Ala Arg Val Asp Gly Ala Ser Ala Trp
   205 210 215
   cag cag ttc acc agg atc acc ctc ccc ctg gtc aaa ccg gcc ctg atg 846
   Gln Gln Phe Thr Arg Ile Thr Leu Pro Leu Val Lys Pro Ala Leu Met
   220 225 230
   gtc gcg gtg ctg ttc cgc acc ctg gat gcc ctg cgc atg tac gac ctg 894
   Val Ala Val Leu Phe Arg Thr Leu Asp Ala Leu Arg Met Tyr Asp Leu
   235 240 245
   ccg gtg atc atg atc tcc gcc tcc tcg aac tcc ccc acc gcc gtg atc 942
   Pro Val Ile Met Ile Ser Ala Ser Ser Asn Ser Pro Thr Ala Val Ile
   250 255 260
   tcc cag ctg gtg gtc gag gac atg cgt cag aac aac ttc aac tcg gcc 990
   Ser Gln Leu Val Val Glu Asp Met Arg Gln Asn Asn Phe Asn Ser Ala
   265 270 275 280
   tcc gcg ctg tcg acg ttg atc ttc ctg ctc atc ttc ttc gtg gcc ttc 1038
   Ser Ala Leu Ser Thr Leu Ile Phe Leu Leu Ile Phe Phe Val Ala Phe
   285 290 295
   gtc atg atc cgg ttc ctc ggg gcg gat gtt tcc ggg cag cgc gga acg 1086
   Val Met Ile Arg Phe Leu Gly Ala Asp Val Ser Gly Gln Arg Gly Thr
   300 305 310
   gag aag aac agg cgg cgg tgg cgc agg ccc ggc cgg aag ggc gcg gct 1134
   Glu Lys Asn Arg Arg Arg Trp Arg Arg Pro Gly Arg Lys Gly Ala Ala
   315 320 325
   gtt gcc ggg gca ggc gtc ggc atc acc ggt gcc gcg gtg gca agt gag      1182
   Val Ala Gly Ala Gly Val Gly Ile Thr Gly Ala Ala Val Ala Ser Glu
       330                 335                 340
   gtg gca tca tca tga aacgcaagac caagaaccta atcctcaact acgcaggcgt      1237
   Val Ala Ser Ser
   345
   ggtgttcatc ctgttctggg ggctggcgcc gttctactgg atggtggtca ctgcactgcg    1297
   ggattcccgc cacaccttcg acaccacccc ctggcccacg cacgtgaccc               1347
<210> 20
   <211> 348
   <212> PRT
   <213> Corynebacterium efficiens
<400> 20
   Met Ala Lys Met Lys Gln Ala Arg Ser Ala Ala Trp Leu Ile Ala Pro
   1               5                   10                  15
   Ala Met Ile Val Leu Thr Val Val Ile Gly Tyr Pro Ile Val Arg Ala
               20                  25                  30
   Val Trp Leu Ser Phe Gln Ala Asp Lys Gly Leu Asp Pro Thr Thr Gly
           35                  40                  45
   Leu Phe Thr Asp Gly Gly Phe Ala Gly Phe Asp Asn Tyr Leu Tyr Trp
       50                  55                  60
   Leu Thr Gln Arg Cys Met Ser Pro Asp Gly Thr Val Gly Thr Cys Pro
   65                  70                  75                  80
   Pro Gly Thr Leu Ala Thr Asp Phe Trp Pro Ala Leu Arg Ile Thr Leu
                   85                  90                  95
   Phe Phe Thr Val Val Thr Val Thr Leu Glu Thr Ile Leu Gly Met Val
               100                 105                 110
   Met Ala Leu Ile Met Ser Lys Glu Phe Arg Gly Arg Ala Leu Val Arg
           115                 120                 125
   Ala Ala Val Leu Ile Pro Trp Ala Ile Pro Thr Ala Val Thr Ala Lys
       130                 135                 140
   Leu Trp Gln Phe Leu Phe Ala Pro Arg Gly Ile Ile Asn Glu Leu Phe
   145                 150                 155                 160
   Gly Leu Asn Ile Ser Trp Thr Thr Asp Pro Trp Ala Ala Arg Ala Ala
                   165                 170                 175
   Val Ile Leu Ala Asp Val Trp Lys Thr Thr Pro Phe Met Ala Leu Leu
   180 185 190
   Ile Leu Ala Gly Leu Gln Met Ile Pro Lys Gly Thr Tyr Glu Ala Ala
   195 200 205
   Arg Val Asp Gly Ala Ser Ala Trp Gln Gln Phe Thr Arg Ile Thr Leu
   210 215 220
   Pro Leu Val Lys Pro Ala Leu Met Val Ala Val Leu Phe Arg Thr Leu
   225 230 235 240
   Asp Ala Leu Arg Met Tyr Asp Leu Pro Val Ile Met Ile Ser Ala Ser
   245 250 255
   Ser Asn Ser Pro Thr Ala Val Ile Ser Gln Leu Val Val Glu Asp Met
   260 265 270
   Arg Gln Asn Asn Phe Asn Ser Ala Ser Ala Leu Ser Thr Leu Ile Phe
   275 280 285
   Leu Leu Ile Phe Phe Val Ala Phe Val Met Ile Arg Phe Leu Gly Ala
   290 295 300
   Asp Val Ser Gly Gln Arg Gly Thr Glu Lys Asn Arg Arg Arg Trp Arg
   305 310 315 320
   Arg Pro Gly Arg Lys Gly Ala Ala Val Ala Gly Ala Gly Val Gly Ile
   325 330 335
   Thr Gly Ala Ala Val Ala Ser Glu Val Ala Ser Ser
   340 345
<210> 21
   <211> 1137
   <212> DNA
   <213> Corynebacterium efficiens
<220>
   <221> CDS
   <222> (151)..(987)
   <223> integrales Membranprotein (Permease) des ABC-Transporters mit der Aktivität eines Trehalose-Importers
<400> 21
   gatccggttc ctcggggcgg atgtttccgg gcagcgcgga acggagaaga acaggcggcg 60
   gtggcgcagg cccggccgga agggcgcggc tgttgccggg gcaggcgtcg gcatcaccgg 120
   tgccgcggtg gcaagtgagg tggcatcatc atg aaa cgc aag acc aag aac cta 174
                                       Met Lys Arg Lys Thr Lys Asn Leu
                                       1               5
   atc ctc aac tac gca ggc gtg gtg ttc atc ctg ttc tgg ggg ctg gcg      222
   Ile Leu Asn Tyr Ala Gly Val Val Phe Ile Leu Phe Trp Gly Leu Ala
       10                  15                  20
   ccg ttc tac tgg atg gtg gtc act gca ctg cgg gat tcc cgc cac acc      270
   Pro Phe Tyr Trp Met Val Val Thr Ala Leu Arg Asp Ser Arg His Thr
   25                  30                  35                  40
   ttc gac acc acc ccc tgg ccc acg cac gtg acc ctg cag aac ttc cgg      318
   Phe Asp Thr Thr Pro Trp Pro Thr His Val Thr Leu Gln Asn Phe Arg
                   45                  50                  55
   gat gcg ctg gcc acc gac aag ggc aac aac ttc ctg gcg gcg atc ggc      366
   Asp Ala Leu Ala Thr Asp Lys Gly Asn Asn Phe Leu Ala Ala Ile Gly
               60                  65                  70
   aac tcg ctg atc gtc agt ctc acc acc acc gcc ctc gcg gtg atc gtg      414
   Asn Ser Leu Ile Val Ser Leu Thr Thr Thr Ala Leu Ala Val Ile Val
           75                  80                  85
   ggc gtg ttc acc gcc tat gcg ctg gca cgc ctg gac ttc ccc ggt aag      462
   Gly Val Phe Thr Ala Tyr Ala Leu Ala Arg Leu Asp Phe Pro Gly Lys
       90                  95                  100
   ggg atc atc acc ggc atc atc ctg gcg gcc tcg atg ttc ccg ggt atc      510
   Gly Ile Ile Thr Gly Ile Ile Leu Ala Ala Ser Met Phe Pro Gly Ile
   105                 110                 115                 120
   gcc ctg gtg acc ccg ctg ttc cag ctg ttc ggc aac atc ggc tgg atc      558
   Ala Leu Val Thr Pro Leu Phe Gln Leu Phe Gly Asn Ile Gly Trp Ile
                   125                 130                 135
   ggc acc tac cag gcg ctg atc atc ccg aac atc tcc ttc gcc ctg ccg      606
   Gly Thr Tyr Gln Ala Leu Ile Ile Pro Asn Ile Ser Phe Ala Leu Pro
               140                 145                 150
   ctg acc atc tac acc ctg gtg tcc ttc ttc cgc cag ctg ccg tgg gag      654
   Leu Thr Ile Tyr Thr Leu Val Ser Phe Phe Arg Gln Leu Pro Trp Glu
           155                 160                 165
   ctc gag gag gcc gcc cgt gtg gac ggc gcg acc cgg ggg cag gcc ttc      702
   Leu Glu Glu Ala Ala Arg Val Asp Gly Ala Thr Arg Gly Gln Ala Phe
       170                 175                 180
   cgc aag atc ctg tta ccc ctg gcc gcc ccg gcg ctg ttc acc acc gcg      750
   Arg Lys Ile Leu Leu Pro Leu Ala Ala Pro Ala Leu Phe Thr Thr Ala
   185                 190                 195                 200
   atc ctg gcg ttc atc gcc tcg tgg aat gag ttc atg ctg gcc cgt cag      798
   Ile Leu Ala Phe Ile Ala Ser Trp Asn Glu Phe Met Leu Ala Arg Gln
                   205                 210                 215
   ctg tcc acc acc gcc acc gaa ccg gtc acc gtg gcc atc gcc cgc ttc      846
   Leu Ser Thr Thr Ala Thr Glu Pro Val Thr Val Ala Ile Ala Arg Phe
               220                 225                 230
   tcc ggg ccg agt tcc ttc gag tac ccg tat gcc tcg gtg atg gca gcc      894
   Ser Gly Pro Ser Ser Phe Glu Tyr Pro Tyr Ala Ser Val Met Ala Ala
           235                 240                 245
   ggt gcc ctg gtc acc gtc cca ctg atc atc atg gtg ctc atc ttc cag      942
   Gly Ala Leu Val Thr Val Pro Leu Ile Ile Met Val Leu Ile Phe Gln
       250                 255                 260
   cga cgc atc gtc tcc ggc ctg acc gcg ggt ggt gtg aag gcc tag          987
   Arg Arg Ile Val Ser Gly Leu Thr Ala Gly Gly Val Lys Ala
   265                 270                 275
   actgtcggtc atgagcacga acgaacccag ggaccagtcc gaacacaaac gccgagccct   1047
   ccagctcgat gcattcatcg ggttcctggg gttcttcgcc ttcctgtcgg tgatccaggc   1107
   cgtgatcaat gtgctccagc ccgaaccgaa                                     1137
<210> 22
   <211> 278
   <212> PRT
   <213> Corynebacterium efficiens
<400> 22
   Met Lys Arg Lys Thr Lys Asn Leu Ile Leu Asn Tyr Ala Gly Val Val
   1               5                   10                  15
   Phe Ile Leu Phe Trp Gly Leu Ala Pro Phe Tyr Trp Met Val Val Thr
               20                  25                  30
   Ala Leu Arg Asp Ser Arg His Thr Phe Asp Thr Thr Pro Trp Pro Thr
           35                  40                  45
   His Val Thr Leu Gln Asn Phe Arg Asp Ala Leu Ala Thr Asp Lys Gly
       50                  55                  60
   Asn Asn Phe Leu Ala Ala Ile Gly Asn Ser Leu Ile Val Ser Leu Thr
   65                  70                  75                  80
   Thr Thr Ala Leu Ala Val Ile Val Gly Val Phe Thr Ala Tyr Ala Leu
                   85                  90                  95
   Ala Arg Leu Asp Phe Pro Gly Lys Gly Ile Ile Thr Gly Ile Ile Leu
               100                 105                 110
   Ala Ala Ser Met Phe Pro Gly Ile Ala Leu Val Thr Pro Leu Phe Gln
           115                 120                 125
   Leu Phe Gly Asn Ile Gly Trp Ile Gly Thr Tyr Gln Ala Leu Ile Ile
       130                 135                 140
   Pro Asn Ile Ser Phe Ala Leu Pro Leu Thr Ile Tyr Thr Leu Val Ser
   145                 150                 155                 160
   Phe Phe Arg Gln Leu Pro Trp Glu Leu Glu Glu Ala Ala Arg Val Asp
                   165                 170                 175
   Gly Ala Thr Arg Gly Gln Ala Phe Arg Lys Ile Leu Leu Pro Leu Ala
               180                 185                 190
   Ala Pro Ala Leu Phe Thr Thr Ala Ile Leu Ala Phe Ile Ala Ser Trp
           195                 200                 205
   Asn Glu Phe Met Leu Ala Arg Gln Leu Ser Thr Thr Ala Thr Glu Pro
       210                 215                 220
   Val Thr Val Ala Ile Ala Arg Phe Ser Gly Pro Ser Ser Phe Glu Tyr
   225                 230                 235                 240
   Pro Tyr Ala Ser Val Met Ala Ala Gly Ala Leu Val Thr Val Pro Leu
                   245                 250                 255
   Ile Ile Met Val Leu Ile Phe Gln Arg Arg Ile Val Ser Gly Leu Thr
               260                 265                 270
   Ala Gly Gly Val Lys Ala
           275
<210> 23
   <211> 534
   <212> DNA
   <213> Corynebacterium efficiens
<220>
   <221> CDS
   <222> (151)..(384)
   <223> hyopthetisches Protein
<400> 23
   ccgggccgag ttccttcgag tacccgtatg cctcggtgat ggcagccggt gccctggtca      60
   ccgtcccact gatcatcatg gtgctcatct tccagcgacg catcgtctcc ggcctgaccg     120
   cgggtggtgt gaaggcctag actgtcggtc atg agc acg aac gaa ccc agg gac     174
                                    Met Ser Thr Asn Glu Pro Arg Asp
                                    1               5
   cag tcc gaa cac aaa cgc cga gcc ctc cag ctc gat gca ttc atc ggg       222
   Gln Ser Glu His Lys Arg Arg Ala Leu Gln Leu Asp Ala Phe Ile Gly
       10                  15                  20
   ttc ctg ggg ttc ttc gcc ttc ctg tcg gtg atc cag gcc gtg atc aat       270
   Phe Leu Gly Phe Phe Ala Phe Leu Ser Val Ile Gln Ala Val Ile Asn
   25                  30                  35                  40
   gtg ctc cag ccc gaa ccg aag gtc tgg ccg gca ctg ctg gcc ctg ctg       318
   Val Leu Gln Pro Glu Pro Lys Val Trp Pro Ala Leu Leu Ala Leu Leu
                   45                  50                  55
   ctg gtg ctg gcg acg gtg agc ctg tgg cgg gcc cgg cgc gac cga tct       366
   Leu Val Leu Ala Thr Val Ser Leu Trp Arg Ala Arg Arg Asp Arg Ser
               60                  65                  70
   ccc cgg acg ggg gct taa gcacccatgg ccatcgtcta caacgccgcc             414
   Pro Arg Thr Gly Ala
           75
   accacggtca acggctttct cgcagatgac cgtgattccc tgcagtggct cttcgacgtc     474
   cccggatccg ccgagacgga agcggatatc accacattcc tcgatagcgt cggcgctgta     534
<210> 24
   <211> 77
   <212> PRT
   <213> Corynebacterium efficiens
<400> 24
   Met Ser Thr Asn Glu Pro Arg Asp Gln Ser Glu His Lys Arg Arg Ala
   1               5                   10                  15
   Leu Gln Leu Asp Ala Phe Ile Gly Phe Leu Gly Phe Phe Ala Phe Leu
               20                  25                  30
   Ser Val Ile Gln Ala Val Ile Asn Val Leu Gln Pro Glu Pro Lys Val
           35                  40                  45
   Trp Pro Ala Leu Leu Ala Leu Leu Leu Val Leu Ala Thr Val Ser Leu
       50                  55                  60
   Trp Arg Ala Arg Arg Asp Arg Ser Pro Arg Thr Gly Ala
   65                  70                  75
<210> 25
   <211> 6199
   <212> DNA
   <213> Corynebacterium glutamicum
<400> 25
   gaaaattgtc ggcgcgatca ttccggcgct ggcgtgaagt gcgatttggt acagctgata     60
   cgcgatgaaa gccagcagca atcatccacg ggtatgccca cagtttgttt tcaggactgc    120
   gaccaggagt accactttcg caaggccgtg cgtcagtaga tatctagcgc tgaacaacgt    180
   agcgtggctg gtgagtgatt cactgctgtg cccaaggaac gtggcgatgc cattgtcggg    240
   atcttcattc agttcgtttt gggtgagcag aacggtccag tggtgaaggc tttcgggatc    300
   gacaagaagg aggagcactc cgccgatgag ctcaaataag ccgttgagtc ctttgagctt    360
   gatgccgccc caaaagagtt gttgccaccg atcgcgaact ttggcagtag ccatgcgttc    420
   tgctcctgac cttgaacagc ggtcccaatt tagacccgct aaacccacaa tgtgtactgg    480
   tgctggtaat ttagtagaac atggcaacgg tcacattcga caaggtcaca atccggtacc    540
   ccggcgcgga gcgcgcaaca gttcatgagc ttgatttaga tatcgctgat ggcgagtttt 600
   tggtgctcgt cggcccttcg ggttgtggta aatccactac gctgcgtgct ttggcggggc 660
   ttgagggcgt ggagtcgggt gtgatcaaaa ttgatggcaa ggatgtcact ggtcaggagc 720
   cggcggatcg cgatatcgcg atggtgttcc agaattatgc tctgtaccct cacatgacgg 780
   tggcgaagaa tatgggtttt gcgctgaagt tggctaagct gccgcaggcg cagatcgatg 840
   cgaaggtcaa tgaggctgcg gaaattcttg ggttgacgga gtttttggat cgcaagccta 900
   aggatttatc gggtggtcag cgtcagcgtg tggcgatggg tcgcgcgttg gtgcgtgatc 960
   cgaaggtgtt cctcatggat gagccgctgt ccaacctgga tgcgaaattg cgcgtgcaaa 1020
   cccgcgcgga ggtcgctgct ttgcagcgtc gcctgggcac caccacggtg tatgtcaccc 1080
   acgatcaggt tgaggcaatg acgatgggcg atcgggttgc ggtgctcaag gacgggttgc 1140
   tgcagcaggt cgcaccgccc agggagcttt acgacgcccc ggtcaacgaa ttcgttgcgg 1200
   gcttcatcgg ctcgccgtcc atgaacctct tccctgccaa cgggcacaag atgggtgtgc 1260
   gcccggagaa gatgctggtc aatgagaccc ctgagggttt cacaagcatt gatgctgtgg 1320
   tggatatcgt cgaggagctt ggctccgaat cgtatgttta tgccacttgg gagggccacc 1380
   gcctggtggc ccgttgggtg gaaggccccg tgccagcccc tggcacgcct gtgacttttt 1440
   cctatgatgc ggcgcaggcg catcatttcg atctggagtc gggcgagcgt atcgcttagt 1500
   ttcggacgtg gggaggcgtc gaaaagcatc tttatttttg accctccggg ggtgatttaa 1560
   cctaaaattc cacacaaacg tgttcgaggt cattagattg ataagcatct gttgttaaga 1620
   aaggtgactt cctatgtcct cgatttcccg caagaccggc gcgtcacttg cagccaccac 1680
   actgttggca gcgatcgcac tggccggttg tagttcagac tcaagctccg actccacaga 1740
   ttccaccgct agcgaaggcg cagacagccg cggccccatc acctttgcga tgggcaaaaa 1800
   cgacaccgac aaagtcattc cgatcatcga ccgctggaac gaagcccacc ccgatgagca 1860
   ggtaacgctc aacgaactcg ccggtgaagc cgacgcgcag cgcgaaaccc tcgtgcaatc 1920
   cctgcaggcc ggcaactctg actacgacgt catggcgctc gacgtcatct ggaccgcaga 1980
   cttcgcggca aaccaatggc tcgcaccact tgaaggcgac ctcgaggtag acacctccgg 2040
   actgctgcaa tccaccgtgg attccgcaac ctacaacggc accctctacg cactgccaca 2100
   gaacaccaac ggccagctac tgttccgcaa caccgaaatc atcccagaag caccagcaaa 2160
   ctgggctgac ctcgtggaat cctgcacgct tgctgaagaa gcaggcgttg attgcctgac 2220
   cactcagctc aagcagtacg aaggcctttc agtgaacacc atcggcttca tcgaaggttg 2280
   gggaggcagc gtcctagacg atgacggcaa cgtcaccgta gacagcgacg acgccaaggc 2340
   aggccttcaa gcgcttgtcg acggcttcga cgacggcacc atctccaagg catcccttgc 2400
   agcgaccgaa gaagaaacca acctcgcatt caccgaaggc caaaccgcct acgccattaa 2460
   ctggccatac atgtacacca actccgaaga agccgaagca accgcaggca aattcgaagt 2520
   acagcccctc gtaggtaaag acggcgtcgg cgtatccacc cttggtggct acaacaacgg 2580
   catcaacgtc aactccgaaa acaaggcaac cgcccgcgac ttcatcgaat tcatcatcaa 2640
   cgaagagaac caaacctggt tcgcggacaa ctccttccca ccagttctgg catccatcta 2700
   cgatgatgag tcccttgttg agcagtaccc atacctgcca gcactgaagg aatccctgga 2760
   aaacgcagca ccacgcccag tgtctccttt ctacccagcc atctccaagg caatccagga 2820
   caacgcctac gcagcgctta acggcaacgt cgacgttgac caggcaacca ccgatatgaa 2880
   ggcagcgatc gaaaacgctt ccagctagtt cggtaattta gttcattctc cggccacctt 2940
   ccctgaaatc cttagcggat ttccacaaag gtggccggag ttttgtccta ttgttgggtg 3000
   taattgaact tgtgtgaaag gagtccggat ggcttccggc aaagatcttc aagtttccac 3060
   atttggctac atctcccgct gccccgtgca ggtctacgaa gcaatcgcag atcccagaca 3120
   actagaacgc tacttcgcca ccggcggagt atctggccgc ctcgaaaccg gatcgactgt 3180
   ctattgggac ttcgttgatt ttcccggtgc gtttccggtc caagttgtct cagctacaca 3240
   ggctgaacac attgaactcc gctggggaca agcaaatgag ctgcgttccg tcaacttcga 3300
   gttcgaacct tttagaaatt tcacccgcac gaaactcacc atcaccgaag gcagttggcc 3360
   gctcactccc gcaggagccc aagaggctct gggcagccag atgggatgga ctggcatgct 3420
   gtccgcacta aaagcgtggc tggaatacgg agtgaacctc cgcgacgggt tttataagca 3480
   ataggcaatg tgtccatcac gatgtgtggc ggattatgat ccatgtaaca agaatgtgca 3540
   gtttcacaga actgacaatc aacttatttt gacctgacaa aaggagcgac gacacatggc 3600
   cacattcaaa caggccagaa gcgctgcctg gctgatcgcc cccgccctcg tggtccttgc 3660
   agtggtgatc ggatatccca tcgtccgagc aatttggcta tccttccagg ccgacaaagg 3720
   cctcgacccc accaccggac tcttcaccga cggtggcttc gcaggactag acaattacct 3780
   ctactggctc acccaacgat gcatgggttc agacggcacc atccgtacct gcccacccgg 3840
   cacactagcc accgacttct ggccagcact acgcatcacg ttgttcttca ccgtggttac 3900
   cgtcggcttg gaaactatcc tcggcaccgc catggcactg atcatgaaca aagaattccg 3960
   tggccgcgca cttgttcgcg cagcgattct tatcccttgg gcaatcccca ccgccgtcac 4020
   cgcaaaactg tggcagttca tcttcgcacc acaaggcatc atcaactcca tgtttggact 4080
   tagtgtcagt tggaccaccg atccgtgggc agctagagcc gccgtcattc ttgccgacgt 4140
   ctggaaaacc acaccattca tggcactgct gatcctcgcc ggtctgcaaa tgatcccgaa 4200
   ggaaacctac gaagcagccc gcgtcgatgg cgcaaccgcg tggcagcaat tcaccaagat 4260
   caccctcccg ctggtgcgcc cagctttgat ggtggcagta ctcttccgca ccctcgatgc 4320
   gctacgcatg tatgacctcc ccgtcatcat gatctccagc tcctccaact cccccaccgc 4380
   tgttatctcc cagctggttg tggaagacat gcgccaaaac aacttcaact ccgcttccgc 4440
   cctttccaca ctgatcttcc tgctgatctt cttcgtggcg ttcatcatga tccgattcct 4500
   cggcgcagat gtttcgggcc aacgcggaat aaagaaaaag aaactgggcg gaaccaagga 4560
   tgagaaaccc accgctaagg atgctgttgt aaaggccgat tctgctgtga aggaagccgc 4620
   taagccatga ctaaacgaac aaaaggactc atcctcaact acgccggagt ggtgttcatc 4680
   ctcttctggg gactagctcc cttctactgg atggttatca ccgcactgcg cgattccaag 4740
   cacacctttg acaccacccc atggccaacg cacgtcacct tggataactt ccgggacgca 4800
   ctggccaccg acaaaggcaa caacttcctc gcagccattg gcaactcact ggtcatcagc 4860
   gtcaccacaa cagcgatcgc tgttctcgtg ggagtgttca ccgcctacgc tctagcccga 4920
   ctggaattcc cgggcaaagg cattgtcacc ggcatcatct tggcagcctc catgttcccc 4980
   ggcatcgccc tggtcactcc gctgttccag ctcttcggtg acctcaactg gatcggcacc 5040
   taccaagcgc tgattatccc gaacatttcc ttcgcgctac ctctgacgat ctacacgctc 5100
   gtatccttct tcaggcaact gccctgggaa ctcgaagaat cagcacgtgt cgacggcgcc 5160
   acacgtggcc aagccttccg catgatcctg cttcctctag cagcgcccgc actatttacc 5220
   accgcgatcc tcgcattcat tgcaacgtgg aacgaattca tgctggcccg ccaactatcc 5280
   aacacctcca cagagccagt gaccgttgcg atcgcaaggt tcaccggacc aagctccttc 5340
   gaatacccct acgcctctgt catggcagcg ggagctttgg tgaccatccc actgatcatc 5400
   atggttctca tcttccaacg ccgcatcgtc tccggactca ccgcaggtgg cgtgaaagcc 5460
   tagactagat actcatgagt gctgataaat cccaggacca atccgaatcg caacgcaaag 5520
   ggcttcaacc cgaagcgctg cttggattcc tgggattttt ctcattcctc gccgtcatcc 5580
   aggcagtcat caacgtgtta cgccccgaac ctgccgtgtg gccagctctt ctcgcgctcg 5640
   ttttagtaat cgccacagtg tcagtatgga gggcttggcg aaagcgccgc cctaattaaa 5700
   gttcctgcgc caacgccacg ataattccag atggcccgcg cagataacac aatcggtagg 5760
   tgtcctcgta atttgcgatc ccatctagtg gttccgcacc gatatgttcg atcgtttcct 5820
   caatatcatc caccgcaaac atcaaacggt gcatcccaat ctggttaggt gcagatggag 5880
   cggttgcaat cggttccggt tgtagatatt gagtaagctc cacccgagaa tgtccatccg 5940
   gagttttcag caccgcgatc tcagatcgaa ttccgctgag accaacggtc cgatcagcaa 6000
   aatccccttg gaccattgtt cggccatcta gggacatccc taatttctca aagaaaccga 6060
   ctgcttcatc caacgattcc accacaatcg ccacgttgtc caaacgttta attcccatga 6120
   tccccatcgt aggtagcatc gtgtgatggc gatcatctac aacacatcga gcacgctcaa 6180
   cggcttcatc gcagacaaa 6199
<210> 26
   <211> 1701
   <212> DNA
   <213> Corynebacterium glutamicum
<220>
   <221> misc_feature
   <222> (1)..(6)
   <223> XbaI Schnittstelle
<220>
   <221> misc_feature
   <222> (7)..(606)
   <223> stromaufwärts gelegene flankierende Region
<220>
   <221> Promoter
   <222> (607)..(1095)
   <223> Promotor Pgap
<220>
   <221> misc_feature
   <222> (607)..(612)
   <223> ScaI Schnittstelle
<220>
   <221> Mutation
   <222> (1079)..(1079)
   <223> Austausch Nukleobase Thymin (T) durch Guanin (G)
<220>
   <221> Mutation
   <222> (1080)..(1080)
   <223> Austausch Nukleobase Thymin (T) durch Cytosin (C)
<220>
   <221> Mutation
   <222> (1081)..(1081)
   <223> Austausch Nukleobase Thymin (T) durch Guanin (G)
<220>
   <221> CDS
   <222> (1096)..(1695)
   <223> cg0832
<220>
   <221> misc_feature
   <222> (1096)..(1695)
   <223> stromabwärts gelegene flankierende Region
<220>
   <221> misc_feature
   <222> (1696)..(1701)
   <223> HindIII Schnittstelle
<400> 26
   tctagagggt gtaattgaac ttgtgtgaaa ggagtccgga tggcttccgg caaagatctt     60
   caagtttcca catttggcta catctcccgc tgccccgtgc aggtctacga agcaatcgca    120
   gatcccagac aactagaacg ctacttcgcc accggcggag tatctggccg cctcgaaacc    180
   ggatcgactg tctattggga cttcgttgat tttcccggtg cgtttccggt ccaagttgtc    240
   tcagctacac aggctgaaca cattgaactc cgctggggac aagcaaatga gctgcgttcc    300
   gtcaacttcg agttcgaacc ttttagaaat ttcacccgca cgaaactcac catcaccgaa    360
   ggcagttggc cgctcactcc cgcaggagcc caagaggctc tgggcagcca gatgggatgg    420
   actggcatgc tgtccgcact aaaagcgtgg ctggaatacg gagtgaacct ccgcgacggg    480
   ttttataagc aataggcaat gtgtccatca cgatgtgtgg cggattatga tccatgtaac    540
   aagaatgtgc agtttcacag aactgacaat caacttattt tgacctgaca aaaggagcga    600
   cgacacagta cttgaagcct aaaaacgacc gagcctattg ggattaccat tgaagccagt    660
   gtgagttgca tcacattggc ttcaaatctg agactttaat ttgtggattc acgggggtgt    720
   aatgtagttc ataattaacc ccattcgggg gagcagatcg tagtgcgaac gatttcaggt    780
   tcgttccctg caaaaactat ttagcgcaag tgttggaaat gcccccgttt ggggtcaatg    840
   tccatttttg aatgtgtctg tatgattttg catctgctgc gaaatctttg tttccccgct    900
   aaagttgagg acaggttgac acggagttga ctcgacgaat tatccaatgt gagtaggttt    960
   ggtgcgtgag ttggaaaaat tcgccatact cgcccttggg ttctgtcagc tcaagaattc   1020
   ttgagtgacc gatgctctga ttgacctaac tgcttgacac attgcatttc ctacaatcgc   1080
   gagaggagac acaac atg gcc aca ttc aaa cag gcc aga agc gct gcc tgg    1131
                    Met Ala Thr Phe Lys Gln Ala Arg Ser Ala Ala Trp
                    1               5                   10
   ctg atc gcc ccc gcc ctc gtg gtc ctt gca gtg gtg atc gga tat ccc     1179
   Leu Ile Ala Pro Ala Leu Val Val Leu Ala Val Val Ile Gly Tyr Pro
           15                  20                  25
   atc gtc cga gca att tgg cta tcc ttc cag gcc gac aaa ggc ctc gac     1227
   Ile Val Arg Ala Ile Trp Leu Ser Phe Gln Ala Asp Lys Gly Leu Asp
       30                  35                  40
   ccc acc acc gga ctc ttc acc gac ggt ggc ttc gca gga cta gac aat     1275
   Pro Thr Thr Gly Leu Phe Thr Asp Gly Gly Phe Ala Gly Leu Asp Asn
   45                  50                  55                  60
   tac ctc tac tgg ctc acc caa cga tgc atg ggt tca gac ggc acc atc     1323
   Tyr Leu Tyr Trp Leu Thr Gln Arg Cys Met Gly Ser Asp Gly Thr Ile
                   65                  70                  75
   cgt acc tgc cca ccc ggc aca cta gcc acc gac ttc tgg cca gca cta     1371
   Arg Thr Cys Pro Pro Gly Thr Leu Ala Thr Asp Phe Trp Pro Ala Leu
               80                  85                  90
   cgc atc acg ttg ttc ttc acc gtg gtt acc gtc ggc ttg gaa act atc     1419
   Arg Ile Thr Leu Phe Phe Thr Val Val Thr Val Gly Leu Glu Thr Ile
           95                  100                 105
   ctc ggc acc gcc atg gca ctg atc atg aac aaa gaa ttc cgt ggc cgc     1467
   Leu Gly Thr Ala Met Ala Leu Ile Met Asn Lys Glu Phe Arg Gly Arg
       110                 115                 120
   gca ctt gtt cgc gca gcg att ctt atc cct tgg gca atc ccc acc gcc      1515
   Ala Leu Val Arg Ala Ala Ile Leu Ile Pro Trp Ala Ile Pro Thr Ala
   125                 130                 135                 140
   gtc acc gca aaa ctg tgg cag ttc atc ttc gca cca caa ggc atc atc      1563
   Val Thr Ala Lys Leu Trp Gln Phe Ile Phe Ala Pro Gln Gly Ile Ile
                   145                 150                 155
   aac tcc atg ttt gga ctt agt gtc agt tgg acc acc gat ccg tgg gca      1611
   Asn Ser Met Phe Gly Leu Ser Val Ser Trp Thr Thr Asp Pro Trp Ala
               160                 165                 170
   gct aga gcc gcc gtc att ctt gcc gac gtc tgg aaa acc aca cca ttc      1659
   Ala Arg Ala Ala Val Ile Leu Ala Asp Val Trp Lys Thr Thr Pro Phe
           175                 180                 185
   atg gca ctg ctg atc ctc gcc ggt ctg caa atg atc aagctt               1701
   Met Ala Leu Leu Ile Leu Ala Gly Leu Gln Met Ile
       190                 195                 200
<210> 27
   <211> 200
   <212> PRT
   <213> Corynebacterium glutamicum
<400> 27
   Met Ala Thr Phe Lys Gln Ala Arg Ser Ala Ala Trp Leu Ile Ala Pro
   1               5                   10                  15
   Ala Leu Val Val Leu Ala Val Val Ile Gly Tyr Pro Ile Val Arg Ala
               20                  25                  30
   Ile Trp Leu Ser Phe Gln Ala Asp Lys Gly Leu Asp Pro Thr Thr Gly
           35                  40                  45
   Leu Phe Thr Asp Gly Gly Phe Ala Gly Leu Asp Asn Tyr Leu Tyr Trp
       50                  55                  60
   Leu Thr Gln Arg Cys Met Gly Ser Asp Gly Thr Ile Arg Thr Cys Pro
   65                  70                  75                  80
   Pro Gly Thr Leu Ala Thr Asp Phe Trp Pro Ala Leu Arg Ile Thr Leu
                   85                  90                  95
   Phe Phe Thr Val Val Thr Val Gly Leu Glu Thr Ile Leu Gly Thr Ala
               100                 105                 110
   Met Ala Leu Ile Met Asn Lys Glu Phe Arg Gly Arg Ala Leu Val Arg
           115                 120                 125
   Ala Ala Ile Leu Ile Pro Trp Ala Ile Pro Thr Ala Val Thr Ala Lys
       130                 135                 140
   Leu Trp Gln Phe Ile Phe Ala Pro Gln Gly Ile Ile Asn Ser Met Phe
   145                 150                 155                 160
   Gly Leu Ser Val Ser Trp Thr Thr Asp Pro Trp Ala Ala Arg Ala Ala
                   165                 170                 175
   Val Ile Leu Ala Asp Val Trp Lys Thr Thr Pro Phe Met Ala Leu Leu
               180                 185                 190
   Ile Leu Ala Gly Leu Gln Met Ile
           195                 200
<210> 28
   <211> 20
   <212> DNA
   <213> Artificial Sequence
<220>
   <223> primer
<220>
   <221> Primer
   <222> (1)..(20)
   <223> cg0832_1.p
<400> 28
   gctggaatac ggagtgaacc 20
<210> 29
   <211> 20
   <212> DNA
   <213> Artificial Sequence
<220>
   <223> primer
<220>
   <221> Primer
   <222> (1)..(20)
   <223> cg0832_2.p
<400> 29
   gggattgccc aagggataag 20
<210> 30
   <211> 28
   <212> DNA
   <213> Artificial
<220>
   <223> synthetische DNA
<400> 30
   gctctagatg cgttctgctc ctgacctt 28
<210> 31
   <211> 28
   <212> DNA
   <213> Artificial
<220>
   <223> synthetische DNA
<400> 31
   cgggatcctt tgcgttgcga ttcggatt 28

## Beschreibung

Die Erfindung betrifft einen Mikroorganismus, der eine organisch-chemische Verbindung produziert und/oder ausscheidet, wobei der Mikroorganismus eine erhöhte Expression eines Trehalose-Importers aufweist; sowie ein Verfahren zur Herstellung einer organisch-chemischen Verbindung unter Verwendung des erfindungsgemäßen Mikroorganismus.

### Stand der Technik

L-Aminosäuren finden in der Humanmedizin, in der pharmazeutischen Industrie, in der Lebensmittelindustrie und ganz besonders in der Tierernährung Anwendung.

Es ist bekannt, dass L-Aminosäuren, wie beispielsweise das L-Lysin, durch Fermentation von Stämmen coryneformer Bakterien, insbesondere Corynebacterium glutamicum, oder von Stämmen der Familie der Enterobacteriaceae, insbesondere Escherichia coli, hergestellt werden. Wegen der großen ökonomischen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien, wie zum Beispiel die Zuckerkonzentration während der Fermentation, oder die Aufarbeitung zur Produktform durch zum Beispiel lonenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite oder auxotroph für regulatorisch bedeutsame Metabolite sind und die L-Aminosäuren produzieren. Ein bekannter Antimetabolit ist das Lysinanalogon S-(2-Aminoethyl)-L-Cystein (AEC).

Seit einigen Jahren werden ebenfalls Methoden der rekombinanten DNA-Technik zur Stammverbesserung von L-Aminosäure produzierenden Stämmen der Gattung Corynebacterium, insbesondere Corynebacterium glutamicum, eingesetzt, oder der Gattung Escherichia, insbesondere Escherichia coli, indem man einzelne Aminosäure-Biosynthesegene modifiziert bzw. verstärkt oder abschwächt und die Auswirkung auf die Aminosäure-Produktion untersucht.

Die Nukleotidsequenzen der Chromosomen zahlreicher Bakterien sind bekannt.

Die Nukleotidsequenz des Genoms von Corynebacterium glutamicum ATCC13032 ist bei Ikeda und Nakagawa (Applied Microbiology and Biotechnology 62, 99-109 (2003)), in der EP 1 108 790 und bei Kalinowski et al. (Journal of Biotechnolgy 104(1-3), (2003)) beschrieben.

Die Nukleotidsequenz des Genoms von Corynebacterium glutamicum R ist bei Yukawa et al. (Microbiology 153(4): 1042-1058 (2007)) beschrieben.

Die Nukleotidsequenz des Genoms von Corynebacterium efficiens ist bei Nishio et al (Genome Research. 13 (7), 1572-1579 (2003)) beschrieben.

Die Nukleotidsequenz des Genoms von Corynebacterium diphteriae NCTC 13129 wurde von Cerdeno-Tarraga et al. (Nucleic Acids Research 31 (22), 6516-6523 (2003)) beschrieben.

Die Nukleotidsequenz des Genoms von Corynebacterium jeikeum wurde von Tauch et al. (Journal of Bacteriology 187 (13), 4671-4682 (2005)) beschrieben.

Eine zusammenfassende Darstellung zu verschiedenen Aspekten der fermentativen Produktion von L-Aminosäuren findet man bei R. Faurie und J. Thommel in Advances in Biochemical Engineering Biotechnology, Band 79 (Springer-Verlag, Berlin, Heidelberg (Deutschland) 2003).

Bei industriellen Fermentationen von *C. glutamicum* findet man im Überstand signifikante Mengen von ausgeschiedener Trehalose. Die extern akkumulierte Trehalose wird dabei von den Zellen nicht wieder in den Stoffwechselkreislauf zurückgeführt. Damit stellt die extern akkumulierte Trehalose in industriellen Fermentationen einen signifikanten Verlust sowohl in Bezug auf maximal erreichbare Produktbildung als auch in Hinsicht auf die im Fermenter erreichte Biomassekonzentration dar.

Als erwünschtes Ziel steht eine Verwertung der extern akkumulierten Trehalose im Vordergrund. Wenn dieses Ziel erreicht wird, hätte dies mehrere mögliche positive Folgen: (1) Nutzbarmachung von Substrat-Kohlenstoff, der sonst am Ende der Fermentation unverwertet zurück bleibt, (2) Erhöhung der in der Fermentation erreichbaren Biomasse, (3) Erhöhung der Produktausbeute bei biotechnologischen Produktionsprozessen, z.B. der Aminosäureproduktion, (4) Vermeidung einer unerwünschten Kontamination im Produktüberstand am Ende der Fermentation.

Die EP 2 107 128 A2 offenbart eine Nukleinsäure, die für ein Trehalose- bzw. Maltosebindendes Protein codiert. Das Dokument offenbart Transformanten, welche diese Nukleinsäure enthalten, im Allgemeinen, sowie die Verwendung dieser Transformanten zur Herstellung von Aminosäuren. Eine erhöhte Expression dieser Nukleinsäure, speziell in *Corynebacterium glutamicum,* ist nicht offenbart.

Die WO 02/26989 A1 offenbart Nukleotidsequenzen, die das *msiK*-Gen kodieren. Dieses *msiK*-Gen, isoliert aus *Corynebacterium glutamicum,* kodiert für ein Zuckerimportprotein. Die Überexpression des *msiK*-Gens führt gemäß WO 02/26989 A1 zu einer verbesserten Produktion von Aminosäuren. Es kann davon ausgegangen werden, dass die verstärkte Produktion von Aminosäuren durch die verbesserte Aufnahme von Zucker aus dem umgebenden Medium geschieht. Mögliche Zucker, die als Kohlenstoffquelle verwendet werden könnten, sind gemäß der WO 02/26989 A1 Glucose, Saccharose, Lactose, Fructose, Maltose, Melasse oder Stärke. Trehalose wird weder als Kohlenstoffquelle, noch als Nebenprodukt genannt. Inwieweit durch das Zuckerimportprotein MsiK nicht nur die üblicherweise als Kohlenstoffquelle verwendeten Zucker, d.h. Glucose oder Saccharose, verstärkt aufgenommen werden, sondern auch andere Kohlenhydrate ist der WO 02/26989 A1 nicht zu entnehmen.

Die WO 02/22669 A1 offenbart den SugA Zuckertransporter aus *Corynebacterium glutamicum*, der identisch ist mit SEQ ID NO. 8 der vorliegenden Anmeldung. Das Dokument offenbart ferner die Inaktivierung des sugA-Gens in Corvnebakterien und die Verwendung der Bakterien für die fermentative Herstellung von L- Aminosäuren, insbesondere L-Lvsin.

Die Aufgabe der vorliegenden Erfindung ist es, verbesserte Mikroorganismen und Verfahren zur fermentativen Herstellung einer organisch-chemischen Verbindung bereitzustellen.

Die vorliegende Erfindung stellt einen Mikroorganismus der Art *Corynebacterium glutamicum* bereit, der eine organisch-chemische Verbindung produziert und/oder ausscheidet, wobei sich der Mikroorganismus durch eine erhöhte Expression von einem Polynukleotid kodierend für ein Polypeptid mit einer Aminosäuresequenz auszeichnet, die eine Identität von 70% oder mehr zu der in SEQ ID NO:8 oder 20 gezeigten Aminosäuresequenz aufweist, wobei dieses Polypeptid, welches eine Untereinheit eines Proteinkomplexes, nämlich eine Proteinuntereinheit des ABC-Transporters mit der Aktivität eines Trehalose-Importers ist, die Aktivität einer Permease aufweist, wobei der Mikroorganismus in der Lage ist, Trehalose aus dem Medium aufzunehmen.

Die Expression des Polynukleotids kodierend für eine Proteinuntereinheit des ABC-Transporters mit der Aktivität eines Trehalose-Importers wird durch eine oder mehrere Maßnahmen ausgewählt aus der folgenden Gruppe erhöht:
a) die Expression des Gens steht unter der Kontrolle eines Promotors, der in dem für das Verfahren verwendeten Mikroorganismus stärker ist als der ursprüngliche Promotor des Gens;
b) Erhöhung der Kopienzahl des Gens kodierend für ein Polypeptid mit der Aktivität eines Trehalose-Importers; vorzugsweise durch Einfügen des Gens in Plasmiden mit erhöhter Kopienzahl und/oder durch Integration des Gens in das Chromosom des Mikroorganismus in mindestens einer Kopie;
c) die Expression des Gens erfolgt unter Verwendung einer Ribosomen-Bindestelle, die in dem für das Verfahren verwendeten Mikroorganismus stärker ist als die ursprüngliche Ribosomen-Bindestelle des Gens;
d) die Expression des Gens erfolgt unter Optimierung der Kodon-Verwendung (codon usage) des für das Verfahren verwendeten Mikroorganismus;
e) die Expression des Gens erfolgt unter Reduzierung von mRNA Sekundärstrukturen in der vom Gen transkribierten mRNA;
f) die Expression des Gens erfolgt unter Eliminierung von RNA-Polymerase-Terminatoren in der vom Gen transkribierten mRNA;
g) die Expression des Gens erfolgt unter Verwendung mRNA-stabilisierender Sequenzen in der vom Gen transkribierten mRNA.

Die genannten Maßnahmen zur Erhöhung der Expression können in geeigneter Weise miteinander kombiniert werden. Bevorzugt wird die Expression des Polynukleotids kodierend für eine Proteinuntereinheit des ABC-Transporters mit der Aktivität eines Trehalose-Importers durch die Kombination mindestens zwei der Maßnahmen ausgewählt aus der Gruppe a), b) und c) erhöht, besonders bevorzugt durch die Kombination der Maßnahmen a) und b).

Weiterhin stellt die vorliegende Erfindung ein Verfahren bereit zur fermentativen Herstellung einer organisch-chemischen Verbindung enthaltend die Schritte:
a) Kultivieren des oben beschriebenen Mikroorganismus gemäß der vorliegenden Erfindung in einem geeignetem Medium, wobei eine Fermentationsbrühe erhalten wird, und
b) Anreichern der organisch-chemischen Verbindung in der Fermentationsbrühe aus a);
wobei eine Anreicherung von Trehalose in der Fermentationsbrühe verringert oder vermieden wird. Bevorzugt ist dabei, dass im Vergleich zu dem jeweiligen Ausgangsstamm des Mikroorganismus die Anreicherung von Trehalose in der Fermentationsbrühe um 50% oder mehr, um 70% oder mehr, um 80% oder mehr, um 90% oder mehr, um 95% oder mehr, um 98% oder mehr, um 99% oder mehr und am meisten bevorzugt um 99,5% oder mehr verringert wird.

Die Vorteile der vorliegenden Erfindung liegen darin, dass (1) Substrat-Kohlenstoff in Form von Trehalose, der sonst am Ende der Fermentation in der Fermentationsbrühe unverwertet zurück bleibt, nutzbar gemacht wird; (2) die in der Fermentation erreichbare Biomasse erhöht wird; (3) die Produktausbeute bei biotechnologischen Produktionsprozessen, z.B. der Aminosäureproduktion, erhöht wird und (4) eine unerwünschte Kontamination im Produktüberstand am Ende der Fermentation vermieden wird.

Überraschenderweise wurde ein Trehalose-Aufnahmesystem in *C. glutamicum* identifiziert. Die Verstärkung der Expression aller Gene des das Trehalosimportsystem kodierenden Operons führte zu einer Erhöhung des Zielproduktes (der organisch-chemischen Verbindung), bei Verwendung eines entsprechenden Produktionsstammes. Auch wurde überraschenderweise festgestellt, dass bei Expression lediglich nur einer der Untereinheiten (z.B. Permeaseunter-einheit) eine entsprechende Trehalose-Aufnahme erfolgt. Die vorliegende Erfindung stellt damit Mikroorganismen (Produktionsstämme) bereit, die die extern akkumulierte Trehalose durch ein aktives Transportsystem in der Plasmamembran in die Zelle aufnehmen. Da *C. glutamicum* metabolisch die Kapazität besitzt, Trehalose im Cytoplasma zu verstoffwechseln, ergeben sich die genannten Vorteile der vorliegenden Erfindung. Bevorzugt ist der Mikroorganismus in der Lage, eine Anreicherung von Trehalose im Medium (Kultivierungsmedium) zu verringern, im Vergleich zum jeweiligen Ausgangsstamm des Mikroorganismus, oder insbesondere zu vermeiden.

Die Proteinuntereinheiten des ABC-Transporters mit der Aktivität eines Trehalose-Importers sind wie folgt: integrales Membranenprotein (Permease), ATP-bindendes und -hydrolysierendes (ATPase) Protein und periplasmisches (oder Lipoprotein) Substrat-bindenes Protein. Dabei ist der Aufbau eines ABC-Transporters wie folgt: zwei Permeasen, zwei ATPasen und ein periplasmisches (oder Lipoprotein) Substrat-bindenes Protein. Dabei können die beiden Permeasen bzw. die ATPasen in ihrer Aminosäuresequenz jeweils voneinander verschieden sein.

In einer bevorzugten Ausführungsform des Mikroorganismus gemäß der vorliegenden Erfindung weist dieser
ferner eine im Vergleich zu dem jeweiligen Ausgangstamm erhöhte Expression mindestens eines Polynukleotids ausgewählt aus der Gruppe a) bis f) auf:
a) ein Polynukleotid kodierend für ein Polypeptid mit einer Aminosäuresequenz, die eine Identität von 70% oder mehr zu der in SEQ ID NO:2 oder 14 gezeigten Aminosäuresequenz aufweist;
b) ein Polynukleotid kodierend für ein Polypeptid mit einer Aminosäuresequenz, die eine Identität von 70% oder mehr zu der in SEQ ID NO:4 oder 16 gezeigten Aminosäuresequenz aufweist;
c) ein Polynukleotid kodierend für ein Polypeptid mit einer Aminosäuresequenz, die eine Identität von 70% oder mehr zu der in SEQ ID NO:6 oder 18 gezeigten Aminosäuresequenz aufweist;
d) ein Polynukleotid kodierend für ein Polypeptid mit einer Aminosäuresequenz, die eine Identität von 70% oder mehr zu der in SEQ ID NO:10 oder 22 gezeigten Aminosäuresequenz aufweist;
e ein Polynukleotid kodierend für ein Polypeptid mit einer Aminosäuresequenz, die eine Identität von 70% oder mehr zu der in SEQ ID NO:12 oder 24 gezeigten Aminosäuresequenz aufweist.

Besonders bevorzugt weist der Mikroorganismus ferner eine im Vergleich zu dem jeweiligen Ausgangstamm erhöhte Expression der folgenden Polynukleotide auf:
ein Polynukleotid kodierend für ein Polypeptid mit einer Aminosäuresequenz, die eine Identität von 70% oder mehr zu der in SEQ ID NO:10 oder 22 gezeigten Aminosäuresequenz aufweist.

In einer weiteren bevorzugten Ausführungsform des Mikroorganismus weist dieser ferner eine im Vergleich zu dem jeweiligen Ausgangstamm erhöhte Expression der Polynukleotide a) und b) auf:
a) ein Polynukleotid kodierend für ein Polypeptid mit einer Aminosäuresequenz, die eine Identität von 70% oder mehr zu der in SEQ ID NO:2 oder 14 gezeigten Aminosäuresequenz aufweist;
b) ein Polynukleotid kodierend für ein Polypeptid mit einer Aminosäuresequenz, die eine Identität von 70% oder mehr zu der in SEQ ID NO:4 oder 16 gezeigten Aminosäuresequenz aufweist;
   sowie des Polynukleotids d)
d) ein Polynukleotid kodierend für ein Polypeptid mit einer Aminosäuresequenz, die eine Identität von 70% oder mehr zu der in SEQ ID NO:10 oder 22 gezeigten Aminosäuresequenz aufweist.

Weiterhin ist bevorzugt, dass der Mikroorganismus eine im Vergleich zu dem jeweiligen Ausgangstamm erhöhte Expression der Polynukleotide a), b) c), d) und e) aufweist.

Unter einer organisch-chemischen Verbindung versteht man für die Maßnahmen der Erfindung ein Vitamin wie z.B. Thiamin (Vitamin B1), Riboflavin (Vitamin B2), Cyanocobalamin (Vitamin B12), Folsäure (Vitamin M), Tocopherol (Vitamin E) oder Nicotinsäure/Nicotinsäureamid, ein Nukleosid oder Nukleotid wie z.B. S-Adenosyl-Methionin, Inosin-5-'-Monophosphorsäure und Guanosin-5'-Monophosphorsäure, L-Aminosäuren oder auch ein Amin wie z.B. Cadaverin. Bevorzugt hergestellt werden L-Aminosäuren und diese enthaltende Produkte.

Die organisch-chemische Verbindung, die von dem erfindungsgemäßen Mikroorganismus produziert und/oder ausgeschieden wird, ist bevorzugt ausgewählt aus der Gruppe Vitamin, Nukleosid oder Nukleotid, L-Aminosäuren und Amin.

Der Begriff L-Aminosäuren umfasst die proteinogenen Aminosäuren, sowie L-Omithin und L-Homoserin. Unter proteinogenen L-Aminosäuren versteht man die L-Aminosäuren die in natürlichen Proteinen, das heißt in Proteinen von Mikroorganismen, Pflanzen, Tieren und Menschen, vorkommen. Zu den proteinogenen Aminosäuren zählen L-Asparaginsäure, L-Asparagin, L-Threonin, L-Serin, L-Glutaminsäure, L-Glutamin, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan, L-Arginin, L-Prolin und gegebenenfalls L-Selenocystein und L-Pyrrolysin.

In besonders bevorzugter Weise ist die organisch-chemische Verbindung ausgewählt aus der Gruppe proteinogene L-Aminosäure, L-Ornithin und L-Homoserin. Besonders bevorzugt ist die proteinogene L-Aminosäure ausgewählt aus der Gruppe L-Lysin, L-Methionin, L-Valin, L-Prolin, L-Glutamat und L-Isoleucin, insbesondere L-Lysin.

Werden im Folgenden Aminosäuren oder L-Aminosäuren erwähnt, umfasst der Begriff auch deren Salze wie beispielsweise das Lysin-Monohydrochlorid oder Lysin-Sulfat im Falle der Aminosäure L-Lysin.

Wie oben erwähnt betrifft die vorliegende Erfindung auch ein Verfahren zur fermentativen Herstellung einer organisch-chemischen Verbindung enthaltend die Schritte:
a) Kultivieren des oben beschriebenen Mikroorganismus gemäß der vorliegenden Erfindung in einem geeignetem Medium, wobei eine Fermentationsbrühe erhalten wird, und
b) Anreichern der organisch-chemischen Verbindung in der Fermentationsbrühe aus a);
wobei eine Anreicherung von Trehalose in der Fermentationsbrühe verringert oder vermieden wird.

Bevorzugt ist dabei, dass im Vergleich zu dem jeweiligen Ausgangsstamm des Mikroorganismus die Anreicherung von Trehalose in der Fermentationsbrühe um 50% oder mehr, um 70% oder mehr, um 80% oder mehr, um 90% oder mehr, um 95% oder mehr, um 98% oder mehr, um 99% oder mehr und am meisten bevorzugt um 99,5% oder mehr verringert wird.

Bevorzugte Mikroorganismen der Art *Corynebacterium glutamicum* sind:
Corynebacterium glutamicum, wie zum Beispiel der
Typstamm ATCC13032 oder der Stamm R.

Einige Vertreter der Art Corynebacterium glutamicum sind im Stand der Technik auch unter anderen Bezeichnungen bekannt. Hierzu gehören beispielsweise:
Stamm ATCC13870 der als Corynebacterium acetoacidophilum bezeichnet wurde,
Stamm DSM20137 der als Corynebacterium lilium bezeichnet wurde,
Stamm ATCC17965 der als Corynebacterium melassecola bezeichnet wurde,
Stamm ATCC14067 der als Brevibacterium flavum bezeichnet wurde,
Stamm ATCC13869 der als Brevibacterium lactofermentum bezeichnet wurde, und
Stamm ATCC14020 der als Brevibacterium divaricatum bezeichnet wurde.

Der Begriff "Micrococcus glutamicus" für Corynebacterium glutamicum war ebenfalls gebräuchlich.

Die für die Maßnahmen der Überexpression des Trehalose-Importers eingesetzten Mikroorganismen bzw. Stämme (Ausgangsstämme) besitzen bevorzugt bereits die Fähigkeit die gewünschte(n) L-Aminosäure(en) in der Zelle anzureichern oder in das sie umgebende Nährmedium auszuscheiden und dort zu akkumulieren. Hierfür wird im Folgenden auch der Ausdruck "produzieren" verwendet. Insbesondere besitzen die für die Maßnahmen der Überexpression eingesetzten Stämme die Fähigkeit ≥ (mindestens) ≥ 0,10 g/l, 0,25 g/l, ≥ 0,5 g/l, ≥ 1,0 g/l, ≥ 1,5 g/l, ≥ 2,0 g/l, ≥ 4 g/l oder ≥ 10 g/l der gewünschten Verbindung in ≤ (maximal) 120 Stunden, ≤ 96 Stunden, ≤ 48 Stunden, ≤ 36 Stunden, ≤ 24 Stunden oder ≤ 12 Stunden in der Zelle oder im Nährmedium anzureichern bzw. zu akkumulieren. Bei den Ausgangsstämmen handelt es sich bevorzugt um Stämme, die durch Mutagenese und Selektion, durch rekombinante DNA-Techniken oder durch eine Kombination beider Methoden hergestellt wurden.

Es ist für den Fachmann verständlich, dass man zu einem für die Maßnahmen der Erfindung geeignetem Mikroorganismus auch dadurch gelangen kann, indem man in einem Wildstamm, wie zum Beispiel in dem Corynebacterium glutamicum Typstamm ATCC 13032 oder in dem Stamm ATCC 14067, zunächst einen Trehalose-Importer überexprimiert und anschließend durch weitere, im Stand der Technik beschriebene, genetische Maßnahmen, den Mikrorganismus veranlasst, die gewünschte(n) L-Aminosäure(en) zu produzieren. Die Transformation des Wildtyps allein mit dem genannten Polynukleotid ist keine erfindungsgemäße Maßnahme.

L-Lysin ausscheidende bzw. produzierende Stämme der Art Corynebacterium glutamicum sind beispielsweise:
Corynebacterium glutamicum MH20-22B (= DSM16835) beschrieben in Menkel et al. (Applied and Environmental Microbiology 55(3), 684-688 (1989)) und hinterlegt als DSM16835,
Corynebacterium glutamicum DM1729 beschrieben bei Georgi et al. (Metabolic Engineering 7, 291-301 (2005)) und in EP 1 717 616 A2 und hinterlegt als DSM17576,
Corynebacterium glutamicum DSM13994 beschrieben in US 6,783,967, und
Corynebacterium glutamicum DM1933 beschrieben bei Blombach et al. (Appl Environ Microbiol. 2009 Jan;75(2):419-27).

L-Lysin produzierende Mikrorganismen besitzen typischerweise eine "feed back" resistente bzw. desensibilisierte Aspartatkinase. Unter "feed back" resistenten Aspartatkinasen versteht man Aspartatkinasen (LysC), die im Vergleich zur Wildform (Wildtyp) eine geringere Empfindlichkeit gegenüber der Hemmung durch Mischungen von Lysin und Threonin oder Mischungen von AEC (Aminoethylcystein) und Threonin oder Lysin allein oder AEC allein aufweisen. Die für diese im Vergleich zum Wildtyp desensibilisierten Aspartatkinasen kodierenden Gene bzw. Allele werden auch als lysC^{FBR}-Allele bezeichnet. Im Falle der Aspartatkinasen der Art Corynebacterium glutamicum ist der Stamm ATCC13032 der geeignete Wildtyp. Im Stand der Technik sind zahlreiche lysC^{FBR}-Allele beschrieben, die für Aspartatkinasevarianten kodieren, welche Aminosäureaustausche im Vergleich zum Wildtypprotein besitzen. Bei Bakterien der Gattung Corynebacterium wird das lysC-Gen auch als ask-Gen bezeichnet. Bei Enterobacteriaceae wird die vom lysC-Gen kodierte Aspartatkinase auch als Aspartokinase III bezeichnet.

Eine ausführliche Liste mit Angaben welche Aminosäureaustausche im Aspatatkinaseprotein von Corynebacterium glutamicum zu einer Desensibilisierung führen ist in unter anderem in der WO2009141330 enthalten. Bevorzugt werden Aspartatkinasevarianten, welche folgende Aminosäureaustausche tragen, ausgewählt aus der Gruppe: an Position 380 der Aminosäuresequenz L-Isoleucin anstelle von L-Threonin und gegebenenfalls an Position 381 L-Phenylalanin anstelle L-Serin, an Position 311 L-Isoleucin anstelle L-Threonin und an Position 279 L-Threonin anstelle L-Alanin.

Eine ausführliche Liste mit Angaben welche Aminosäureaustausche im Aspatatkinase III Protein von Escherichia coli zu einer Desensibilisierung gegenüber der Hemmung durch L-Lysin führen ist in unter anderem in der EP 0 834 559 A1 auf Seite 3 (Zeilen 29 bis 41) enthalten. Bevorzugt wird eine Aspartkinasevariante, welche an Position 323 der Aminosäuresequenz L-Asparaginsäure anstelle Glycin und/oder an Position 318 L-Isoleucin anstelle L-Methionin enthält.

Ein L-Methionin ausscheidender bzw. produzierender Stamm der Art Corynebacterium glutamicum ist beispielsweise
Corynebacterium glutamicum DSM 17322 beschrieben in WO 2007/011939.

Bekannte Vertreter L- Tryptophan produzierender bzw. ausscheidender Stämme coryneformer Bakterien sind beispielsweise:
Corynebacterium glutamicum K76 (=Ferm BP-1847) beschrieben in US 5,563,052,
Corynebacterium glutamicum BPS13 (=Ferm BP-1777) beschrieben in US 5,605,818, und
Corynebacterium glutamicum Ferm BP-3055 beschrieben in US 5,235,940.

Bekannte Vertreter L-Valin produzierender bzw. ausscheidender Stämme coryneformer Bakterien sind beispielsweise:
Corynebacterium glutamicum FERM BP-3006 beschrieben in US 5,521,074, und
Corynebacterium glutamicum FERM BP-1764 beschrieben in US 5,188,948.

Bekannte Vertreter L-Isoleucin produzierender bzw. ausscheidender Stämme coryneformer Bakterien sind beispielsweise:
Corynebacterium glutamicum FERM BP-758 beschrieben in US 4,656,135.

Unter einem ABC-Transporter mit der Aktivität eines Trehalose-Importers versteht man ein Protein bzw. einen Proteinkomplex mit mehreren Untereinheiten, der den Transport von Trehalose aus der Umgebung in die Zelle des betreffenden Mikroorganismus katalysiert.

ABC-Transporter bilden eine der größten Familien von Membranproteinen, die als gemeinsames Strukturelement eine ATP-bindende Kassette (von englisch: *ATP binding cassette,* ABC) besitzen und spezifische Substrate aktiv über eine Zellmembran transportieren. Wenn die Substrate von ABC-Transportern gegen einen Konzentrationsgradienten transportiert werden, muss für den Vorgang Energie aufgewandt werden; das geschieht durch die Bindung und Hydrolyse von ATP an der ATPase-Einheit.

Die Struktur eines prokaryotischen ABC-Transporters besteht normalerweise aus drei Bestandteilen: zwei integrale Membranenproteine (Permease), wobei jedes fünf bis sieben Transmembransegmente aufweist, zwei Zusatzproteine, die ATP binden und hydrolysieren (ATPase) und ein periplasmatisches Substrat-bindenes Protein (oder in der Membran verankertes Lipoprotein). Viele der Gene für die drei Bestandteile bilden Operons. ABC-Transporter gehören damit zu den *primär aktiven Transportern* einerseits und zu den membranständigen ATPasen andererseits.

In den öffentlichen Datenbanken, wie beispielsweise der Datenbank UniProtKB (Universal Protein Resource Knowledgebase), sind ABC-Transporter verschiedenster Lebewesen beschrieben. Die Datenbank UniProtKB wird vom UniProt Konsortium unterhalten, dem das European Bioinformatics Institute (EBI, Wellcome Trust, Hinxton Cambridge, United Kingdom), das Swiss Institute of Bioinformatics (SIB, Centre Medical Universitaire, Genf, Schweiz) und die Protein Information Resource (PIR, Georgetown University, Washington, DC, US) angehören.

Die Gene für einen Trehalose-Importer können aus den Organismen mithilfe der Polymerase-Kettenreaktion (PCR) unter Verwendung geeigneter Primer isoliert werden. Anleitungen findet man unter anderem im Laborhandbuch "PCR" von Newton und Graham (Spektrum Akademischer Verlag, Heidelberg, Deutschland, 1994)und in der WO 2006/100211 auf Seite 14 bis 17.

Für die Maßnahmen der Erfindung können die Gene des Trehalose-Importers aus Corynebacterien eingesetzt werden. Bevorzugt werden Gene eingesetzt, die für Polypeptide mit Aktivität eines Trehalose-Importers kodieren, deren Aminosäuresequenz ≥ (mindestens) ≥ 50%, ≥ 60%, ≥ 70%, ≥ 80%, ≥ 90%, ≥ 92%, ≥ 94%, ≥ 96%, ≥ 97%, ≥ 98%, ≥ 99%, identisch sind mit der Aminosäuresequenz ausgewählt aus SEQ ID NO: 2, 4, 6, 8, 10 und ggf. 12, bzw. 14, 16, 18, 20, 22, 24. Während der Untersuchungen, die zur vorliegenden Erfindung führten, wurde das für den Trehalose-Importer kodierende Operon von *Corynebakterium glutamicum* identifiziert. Das Operon, das in *Corynebakterium glutamicum* den Trehalose-Importer kodiert, weist mehrere Leseraster bzw. Gene auf.

Die Tabelle 1 fasst die Informationen über die Leseraster des Operons, das für den Trehalose-Importer von *Corynebacterium glutamicum* kodiert, zusammen.

**Tabelle 1: Die Gene/Leseraster des Operons, das für den Trehalose-Importer von Corynebacterium glutamicum kodiert**

| **Bezeichnung des Leserasters im Operon** | **kodiert für** | **Länge (Anzahl der Aminosäurereste)** | **SEQ ID NO** : |
|---|---|---|---|
| cg0835 (msik2) | ATPase | 332 | 2 |
| cg0834 | periplasmatisches Substratbindeprotein | 424 | 4 |
| cg0833 | unbekannt | 151 | 6 |
| cg0832 | Permease | 344 | 8 |
| cg0831 | Permease | 278 | 10 |
| cg0830 | hypothetischer Leserahmen | 74 | 12 |

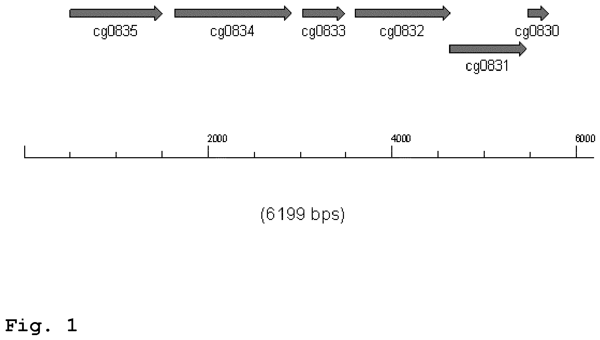

Die genomische Anordnung der Leseraster ist in Figur 1 dargestellt und die Sequenz der Region unter SEQ ID NO:25 hinterlegt.

Ein Gen ist chemisch gesehen ein Polynukleotid. Ein Polynukleotid, das ein Protein/Polypeptide kodiert wird hier synonym zum Begriff "Gen" gebraucht.

In einer bevorzugten Ausführungsform des Mikroorganismus überexprimiert dieser ein oder mehrere Gen(e) kodierend für ein oder mehrere Polypeptid(e) ausgewählt aus den folgenden a) bis f):
a)
   i) ein Polypeptid bestehend aus oder enthaltend die in SEQ ID NO: 2 gezeigte Aminosäuresequenz;
   ii) ein Polypeptid mit einer Aminosäuresequenz, die zu 70% oder mehr identisch ist mit der Aminosäuresequenz von i), wobei das Polypeptid eine Untereinheit eines Proteinkomplexes mit der Aktivität eines Trehalose-Importers ist;
   iii) ein Polypeptid, das eine Aminosäuresequenz enthaltend eine Deletion, Substitution, Insertion und/oder Addition von 1 bis 66, 1 bis 33, 1 bis 17, 1 bis 7 Aminosäureresten bezüglich der in SEQ ID NO: 2 gezeigten Aminosäuresequenz aufweist, wobei das Polypeptid eine Untereinheit eines Proteinkomplexes mit der Aktivität eines Trehalose-Importers ist;
b)
   i) ein Polypeptid bestehend aus oder enthaltend die in SEQ ID NO: 4 gezeigte Aminosäuresequenz;
   ii) ein Polypeptid mit einer Aminosäuresequenz, die zu 70% oder mehr identisch ist mit der Aminosäuresequenz von i), wobei das Polypeptid eine Untereinheit eines Proteinkomplexes mit der Aktivität eines Trehalose-Importers ist;
   iii) ein Polypeptid, das eine Aminosäuresequenz enthaltend eine Deletion, Substitution, Insertion und/oder Addition von 1 bis 85, 1 bis 42, 1 bis 21, 1 bis 9 Aminosäureresten bezüglich der in SEQ ID NO: 4 gezeigten Aminosäuresequenz aufweist, wobei das Polypeptid eine Untereinheit eines Proteinkomplexes mit der Aktivität eines Trehalose-Importers ist;
c)
   i) ein Polypeptid bestehend aus oder enthaltend die in SEQ ID NO: 6 gezeigte Aminosäuresequenz;
   ii) ein Polypeptid mit einer Aminosäuresequenz, die zu 70% oder mehr identisch ist mit der Aminosäuresequenz von i), wobei das Polypeptid eine Untereinheit eines Proteinkomplexes mit der Aktivität eines Trehalose-Importers ist;
   iii) ein Polypeptid, das eine Aminosäuresequenz enthaltend eine Deletion, Substitution, Insertion und/oder Addition von 1 bis 30, 1 bis 15, 1 bis 6, 1 bis 3 Aminosäureresten bezüglich der in SEQ ID NO: 6 gezeigten Aminosäuresequenz aufweist, wobei das Polypeptid eine Untereinheit eines Proteinkomplexes mit der Aktivität eines Trehalose-Importers ist;
d)
   i) ein Polypeptid bestehend aus oder enthaltend die in SEQ ID NO: 8 gezeigte Aminosäuresequenz;
   ii) ein Polypeptid mit einer Aminosäuresequenz, die zu 70% oder mehr identisch ist mit der Aminosäuresequenz von i), wobei das Polypeptid eine Untereinheit eines Proteinkomplexes mit der Aktivität eines Trehalose-Importers ist;
   iii) ein Polypeptid, das eine Aminosäuresequenz enthaltend eine Deletion, Substitution, Insertion und/oder Addition von 1 bis 69, 1 bis 34, 1 bis 17, 1 bis 7 Aminosäureresten bezüglich der in SEQ ID NO: 8 gezeigten Aminosäuresequenz aufweist, wobei das Polypeptid eine Untereinheit eines Proteinkomplexes mit der Aktivität eines Trehalose-Importers ist;
e)
   i) ein Polypeptid bestehend aus oder enthaltend die in SEQ ID NO: 10 gezeigte Aminosäuresequenz;
   ii) ein Polypeptid mit einer Aminosäuresequenz, die zu 70% oder mehr identisch ist mit der Aminosäuresequenz von i), wobei das Polypeptid eine Untereinheit eines Proteinkomplexes mit der Aktivität eines Trehalose-Importers ist;
   iii) ein Polypeptid, das eine Aminosäuresequenz enthaltend eine Deletion, Substitution, Insertion und/oder Addition von 1 bis 56, 1 bis 28, 1 bis 14, 1 bis 6 Aminosäureresten bezüglich der in SEQ ID NO: 10 gezeigten Aminosäuresequenz aufweist, wobei das Polypeptid eine Untereinheit eines Proteinkomplexes mit der Aktivität eines Trehalose-Importers ist;
f)
   i) ein Polypeptid bestehend aus oder enthaltend die in SEQ ID NO: 12 gezeigte Aminosäuresequenz;
   ii) ein Polypeptid mit einer Aminosäuresequenz, die zu 70% oder mehr identisch ist mit der Aminosäuresequenz von i), wobei das Polypeptid eine Untereinheit eines Proteinkomplexes mit der Aktivität eines Trehalose-Importers ist;
   iii) ein Polypeptid, das eine Aminosäuresequenz enthaltend eine Deletion, Substitution, Insertion und/oder Addition von 1 bis 15, 1 bis 8, 1 bis 4, 1 bis 2 Aminosäureresten bezüglich der in SEQ ID NO: 12 gezeigten Aminosäuresequenz aufweist, wobei das Polypeptid eine Untereinheit eines Proteinkomplexes mit der Aktivität eines Trehalose-Importers ist.

In bevorzugten Ausführungsformen sind Varianten umfasst, die wenigstens 75%, wenigstens 80%, wenigstens 85%, wenigstens 90%, wenigstens 95%, wenigstens 98% oder wenigstens 99% identisch zu den oben beschriebenen Aminosäuresequenzen sind, d.h. wobei wenigstens 75%, wenigstens 80%, wenigstens 85%, wenigstens 90%, wenigstens 95%, wenigstens 98% oder wenigstens 99% der Aminosäurepositionen identisch zu jenen der oben beschriebenen Aminosäuresequenzen sind. Die prozentuale Identität wird vorzugsweise über die gesamte Länge der Aminosäure oder Nukleinsäureregion berechnet. Eine Reihe von Programmen, die auf einer Vielzahl von Algorithmen beruhen, steht dem Fachmann für den Sequenzvergleich zur Verfügung. In diesem Zusammenhang ergeben die Algorithmen von Needleman und Wunsch oder Smith und Waterman besonders verlässliche Ergebnisse. Für das Alignment der Sequenzen stehen das Programm PileUp (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970)) und Smith and Waterman (Adv. Appl. Math. 2; 482-489 (1981))], die zum Softwarepaket GCG gehören [Genetics Computer Group, 575 Science Drive, Madison, Wisconsin, USA 53711 (1991)] zur Verfügung. Die oben aufgeführten Prozentwerte für die Sequenzidentität werden vorzugsweise mit dem Programm GAP über die gesamte Sequenzregion berechnet.

Gegebenenfalls werden konservative Aminosäureaustausche bevorzugt. Bei den aromatischen Aminosäuren spricht man von konservativen Austauschen, wenn Phenylalanin, Tryptophan und Tyrosin gegeneinander ausgetauscht werden. Bei den hydrophoben Aminosäuren spricht man von konservativen Austauschen, wenn Leucin, Isoleucin und Valin gegeneinander ausgetauscht werden. Bei den polaren Aminosäuren spricht man von konservativen Austauschen, wenn Glutamin und Asparagin gegeneinander ausgetauscht werden. Bei den basischen Aminosäuren spricht man von konservativen Austauschen, wenn Arginin, Lysin und Histidin gegeneinander ausgetauscht werden. Bei den sauren Aminosäuren spricht man von konservativen Austauschen, wenn Asparaginsäure und Glutaminsäure gegeneinander ausgetauscht werden. Bei den Hydroxyl-Gruppen enthaltenden Aminosäuren spricht man von konservativen Austauschen, wenn Serin und Threonin gegeneinander ausgetauscht werden.

Weiterhin können Polynukleotide verwendet werden, die mit der zu SEQ ID NO:1, 3, 5, 7, 9, 11, bevorzugt der Kodierregion von SEQ ID NO: 1, 3, 5, 7, 9, 11, komplementären Nukleotidsequenz unter stringenten Bedingungen hybridisieren und für ein Polypeptid, welches ein Bestandteil eines Trehalose-Importers ist, kodieren.

Anleitungen zur Hybridisierung von Nukleinsäuren beziehungsweise Polynukleotiden findet der Fachmann unter anderem im Handbuch "The DIG System Users Guide for Filter Hybridization" der Firma Boehringer Mannheim GmbH (Mannheim, Deutschland, 1993) und bei Liebl et al. (International Journal of Systematic Bacteriology 41: 255-260 (1991)). Die Hybridisierung findet unter stringenten Bedingungen statt, das heißt, es werden nur Hybride gebildet, bei denen die Sonde d.h. ein Polynukleotid umfassend die zu SEQ ID NO: 1, 3, 5, 7, 9, 11, bevorzugt die Kodierregion von SEQ ID NO: 1, 3, 5, 7, 9, 11, komplementäre Nukleotidsequenz und die Zielsequenz, d. h. die mit der Sonde behandelten beziehungsweise identifizierten Polynukleotide, mindestens 70% identisch sind. Es ist bekannt, dass die Stringenz der Hybridisierung einschließlich der Waschschritte durch Variieren der Pufferzusammensetzung, der Temperatur und der Salzkonzentration beeinflusst bzw. bestimmt wird. Die Hybridisierungsreaktion wird im Allgemeinen bei relativ niedriger Stringenz im Vergleich zu den Waschschritten durchgeführt (Hybaid Hybridisation Guide, Hybaid Limited, Teddington, UK, 1996).

Für die Hybridisierungsreaktion kann beispielsweise ein Puffer entsprechend 5x SSC-Puffer bei einer Temperatur von ca. 50°C - 68°C eingesetzt werden. Dabei können Sonden auch mit Polynukleotiden hybridisieren, die weniger als 70% Identität zur Nukleotidsequenz der eingesetzten Sonde aufweisen. Solche Hybride sind weniger stabil und werden durch Waschen unter stringenten Bedingungen entfernt. Dies kann beispielsweise durch Senken der Salzkonzentration auf 2x SSC oder 1x SSC und gegebenenfalls nachfolgend 0,5x SSC (The DIG System User's Guide for Filter Hybridisation, Boehringer Mannheim, Mannheim, Deutschland, 1995) erreicht werden, wobei eine Temperatur von ca. 50°C - 68°C, ca. 52°C - 68°C, ca. 54°C - 68°C, ca. 56°C - 68°C, ca. 58°C - 68°C, ca. 60°C - 68°C, ca. 62°C - 68°C, ca. 64°C - 68°C, ca. 66°C - 68°C eingestellt wird. Temperaturbereiche von ca. 64°C - 68°C oder ca. 66°C - 68°C werden bevorzugt. Es ist gegebenenfalls möglich, die Salzkonzentration bis auf eine Konzentration entsprechend 0,2x SSC oder 0,1x SSC zu senken. Gegebenenfalls enthält der SSC-Puffer Natriumdodecylsulfat (SDS) in einer Konzentration von 0,1%. Durch schrittweise Erhöhung der Hybridisierungstemperatur in Schritten von ca. 1 - 2°C von 50°C auf 68°C können Polynukleotidfragmente isoliert werden, die mindestens 70%, mindestens 80%, mindestens 90%, mindestens 92%, mindestens 94%, mindestens 96%, mindestens 97%, mindestens 98% oder mindestens 99%, gegebenenfalls 100% Identität zur Sequenz beziehungsweise komplementären Sequenz der eingesetzten Sonde besitzen und für Polypeptid, welches ein Bestandteil eines Trehalose-Importers ist, kodieren. Weitere Anleitungen zur Hybridisierung sind in Form sogenannter Kits am Markt erhältlich (z.B. DIG Easy Hyb von der Firma Roche Diagnostics GmbH, Mannheim, Deutschland, Catalog No. 1603558).

Für die Maßnahmen der Erfindung wird ein für einen Bestandteil eines Trehalose-Importers kodierendes Gen in einem die gewünschte(n) Aminosäure(n) produzierenden Mikroorganismus bzw. Ausgangs- oder Elternstamm überexprimiert.

Unter Überexpression versteht man allgemein eine Erhöhung der intrazellulären Konzentration oder Aktivität einer Ribonukleinsäure, eines Proteins (Polypeptids) oder eines Enzyms im Vergleich zum Ausgangsstamm (Elternstamm) oder Wildtypstamm, wenn dies der Ausgangsstamm ist. Unter einem Ausgangsstamm (Elternstamm) versteht man den Stamm, an dem die zur Überexpression führende Maßnahme durchgeführt wurde.

Bei der Überexpression werden die Methoden der rekombinanten Überexpression bevorzugt. Hierunter werden alle Methoden zusammengefasst bei denen ein Mikroorganismus unter Verwendung eines in-vitro bereitgestellten DNA-Moleküls hergestellt wird. Derartige DNA-Moleküle umfassen beispielsweise Promotoren, Expressionskassetten, Gene, Allele, Kodierregionen etc.. Diese werden durch Methoden der Transformation, Konjugation, Transduktion oder gleichartige Methoden in den gewünschten Mikroorganismus überführt.

Durch die Maßnahmen der Überexpression, wird die Aktivität oder Konzentration des entsprechenden Polypeptids im Allgemeinen um mindestens 10%, 25%, 50%, 75%, 100%, 150%, 200%, 300%, 400% oder 500%, bevorzugt maximal bis 1000%, 2000%, 4000%, 10000% oder 20000% bezogen auf die Aktivität oder Konzentration des Polypeptids im Stamm vor der zur Überexpression führenden Maßnahme, erhöht.

Zur Erzielung einer Überexpression stehen im Stand der Technik eine Vielzahl von Methoden zur Verfügung. Hierzu gehören die Erhöhung der Kopienzahl und die Modifikation der Nukleotidsequenzen, die die Expression des Gens steuern bzw. kontrollieren. Die Transkription eines Gens wird unter anderem kontrolliert durch den Promotor und gegebenenfalls durch Proteine, welche die Transkription unterdrücken (Repressorproteine) oder fördern (Aktivatorproteine). Die Translation der gebildeten RNA wird unter anderem kontrolliert durch die Ribosomenbindungsstelle und das Startkodon. Polynukleotide bzw.

DNA-Moleküle, welche einen Promotor und eine Ribosomenbindungsstelle und gegebenenfalls ein Startkodon umfassen, werden auch als Expressionskassette bezeichnet.

Die Erhöhung der Kopienzahl kann durch Plasmide erfolgen, die im Cytoplasma des Mikroorganismus replizieren. Hierzu sind im Stand der Technik eine Fülle von Plasmiden für die verschiedensten Gruppen von Mikroorganismen beschrieben, mit denen man die gewünschte Erhöhung der Kopienzahl des Gens einstellen kann. Geeignete Plasmide für die Gattung Escherichia sind beispielsweise im Handbuch Molecular Biology, Labfax (Ed.: T. A. Brown, Bios Scientific, Oxford, UK, 1991) beschrieben. Geeignete Plasmide für die Gattung Corynebacterium sind beispielsweise bei Tauch et al. (Journal of Biotechnology 104 (1-3), 27-40, (2003)), oder bei Stansen et al. (Applied and Environmental Microbiology 71, 5920-5928 (2005)) beschrieben.

Die Erhöhung der Kopienzahl um mindestens eine (1) Kopie kann weiterhin durch Einfügen weiterer Kopien in das Chromosom des Mikroorganismus erfolgen. Geeignete Methoden für die Gattung Corynebacterium sind beispielsweise in der Patentschrift WO 03/014330, WO 03/040373 und WO 04/069996 beschrieben. Geeignete Methoden für die Gattung Escherichia sind beispielsweise der Einbau einer Genkopie in die att-site des Phagen (Yu und Court, Gene 223, 77-81 (1998)), die chromosomale Amplifikation mit Hilfe des Phagen Mu, wie in der EP 0 332 448 beschrieben, oder die von Hamilton et al. (Journal of Bacteriology 174, 4617 - 4622 (1989)) oder Link et al. (Journal of Bacteriology 179, 6228-6237 (1997)) beschriebenen Methoden des Genaustausches mit Hilfe konditional replizierender Plasmide.

Die Erhöhung der Genexpression kann weiterhin durch Verwendung eines starken Promotors erzielt werden, der funktionell mit dem zu exprimierenden Gen verknüpft wird. Vorzugsweise wird ein Promotor verwendet, der stärker ist als der natürliche, d. h. der im Wildtyp oder Elternstamm vorhandene Promotor. Hierfür stehen im Stand der Technik eine Fülle von Methoden zur Verfügung.

Unter einer "funktionellen Verknüpfung" versteht man in diesem Zusammenhang die sequentielle Anordnung eines Promotors mit einem Gen, die zur Expression des Gens und deren Steuerung führt.

Geeignete Promotoren für die Gattung Corynebacterium finden sich unter anderem bei Morinaga et al. (Journal of Biotechnology 5, 305-312, (1987)), in den Patentschriften EP 0 629 699 A2, US 2007/0259408 A1, WO 2006/069711, EP 1 881 076 A1 und EP 1 918 378 A1 und in zusammenfassenden Darstellungen wie dem "Handbook of Corynebacterium glutamicum" (Eds.: Lothar Eggeling und Michael Bott, CRC Press, Boca Raton, US (2005)) oder dem Buch "Corynebacteria, Genomics and Molecular Biology" (Ed.:Andreas Burkovski, Caister Academic Press, Norfolk, UK (2008)). Beispiele für Promotoren, die eine kontrollierte, das heißt induzierbare oder reprimierbare Expression erlauben, sind beispielsweise bei Tsuchiya und Morinaga (Bio/Technology 6, 428-430 (1988)) beschrieben.

Derartige Promotoren oder Expressionskassetten werden typischerweise im Abstand von 1 bis 1000, bevorzugt 1 bis 500, Nukleotiden stromaufwärts des ersten Nukleotids des Startkodons der Kodierregion des Gens eingesetzt.

Es ist ebenfalls möglich, mehrere Promotoren vor das gewünschte Gen zu positionieren bzw. funktionell mit dem zum exprimierenden Gen zu verknüpfen und auf diese Weise zu einer erhöhten Expression zu gelangen. Beispiele hierfür werden in der Patentschrift WO 2006/069711 beschrieben.

Die Struktur von Promotoren von Escherichia coli ist gut bekannt. Es ist daher möglich, die Stärke eines Promotors durch Modifikation seiner Sequenz mittels einem oder mehreren Austausch(en) und/oder einer oder mehrerer Insertion(en) und/oder einer oder mehrerer Deletion(en) von Nukleotiden zu erhöhen. Beispiele hierfür finden sich unter anderem in "Herder Lexikon der Biologie" (Spektrum Akademischer Verlag, Heidelberg, Deutschland (1994)).

Beispiele für die Veränderung von Promotoren zur Erhöhung der Expression in coryneformen Bakterien finden sich in der US 6,962,805 B2 und in einer Publikation von Vasicovä et al. (Bacteriol. 1999 Oct;181(19):6188-91.). Die Verstärkung eins Zielgens durch Ersatz eines homologen Promotors findet sich beispielsweise in der EP 1 697 526 B1.

Die Struktur der Ribosomenbindungsstelle Corynebacterium glutamicum ist ebenfalls gut bekannt und beispielsweise bei Amador (Microbiology 145, 915-924 (1999)) und in Hand- und Lehrbüchern der Genetik, wie beispielsweise "Gene und Klone" (Winnacker, Verlag Chemie, Weinheim, Deutschland (1990)) oder "Molecular Genetics of Bacteria" (Dale und Park, Wiley and Sons Ltd., Chichester, UK (2004)) beschrieben.

Zur Erzielung einer Überexpression ist es ebenfalls möglich, die Expression von Aktivatorproteinen zu steigern oder die Expression von Repressorproteinen zu verringern oder auszuschalten.

Die genannten Maßnahmen zur Überexpression können in geeigneter Weise miteinander kombiniert werden. So kann beispielsweise die Verwendung einer geeigneten Expressionskassette mit der Erhöhung der Kopienzahl und bevorzugt die Verwendung eines geeigneten Promotors mit der Erhöhung der Kopienzahl kombiniert werden.

Anleitungen zum Umgang mit DNA, Verdauung und Ligation von DNA, Transformation und Selektion von Transformanten findet man unter anderem in dem bekannten Handbuch von Sambrook et al. "Molecular Cloning: A Laboratory Manual, Second Edition (Cold Spring Harbor Laboratory Press, 1989).

Das Ausmaß der Expression beziehungsweise Überexpression kann durch Messung der Menge der vom Gen transkribierten mRNA, durch Bestimmung der Menge des Polypeptids und durch Bestimmung der Enzymaktivität festgestellt werden.

Für die Bestimmung der Menge an mRNA können unter anderem die Methode des "Northern Blotting's" und der quantitativen RT-PCR verwendet werden. Bei der quantitativen RT-PCR wird der Polymerase-Kettenreaktion eine reverse Transkription vorgeschaltet. Hierzu kann das LightCycler™ System der Firma Roche Diagnostics (Boehringer Mannheim GmbH, Roche Molecular Biochemicals, Mannheim, Deutschland) verwendet werden, wie beispielsweise bei Jungwirth et al. (FEMS Microbiology Letters 281, 190-197 (2008)) beschrieben. Die Konzentration des Proteins kann über 1- und 2-dimensionale Proteingelauftrennung und anschließende optische Identifizierung der Proteinkonzentration mit enstprechender Auswertesoftware im Gel bestimmt werden. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise. Die Proteinkonzentration kann ebenfalls durch Western-Blot-Hybridisierung mit einem für das nachzuweisende Protein spezifischen Antikörper (Sambrook et al., Molecular cloning: a laboratory manual. 2nd Ed. Cold Spring Harbor Laboratory Press, Cold Spring Harbor, N.Y., 1989) und anschließender optischer Auswertung mit ensprechender Software zur Konzentrationsbestimmung (Lohaus und Meyer (1998) Biospektrum 5:32-39; Lottspeich, Angewandte Chemie 321: 2630-2647 (1999)) bestimmt werden.

Die hergestellten Mikroorganismen können kontinuierlich - wie beispielsweise in der WO 05/021772 beschrieben- oder diskontinuierlich im batch - Verfahren (Satzkultivierung bzw. Satzverfahren) oder im fed batch (Zulaufverfahren) oder repeated fed batch Verfahren (repetitives Zulaufverfahren) zum Zwecke der Produktion der gewünschten organisch chemischen Verbindung kultiviert werden. Eine Zusammenfassung allgemeiner Art über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) verfügbar.

Das zu verwendende Kulturmedium beziehungsweise Fermentationsmedium muß in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten. Die Begriffe Kulturmedium und Fermentationsmedium beziehungsweise Medium sind gegenseitig austauschbar.

Als Kohlenstoffquelle können Zucker und Kohlehydrate wie z.B. Glucose, Saccharose, Lactose, Fructose, Maltose, Melasse, Saccharose-haltige Lösungen aus der Zuckerrüben- oder Zuckerrohrverarbeitung, Stärke, Stärkehydrolysat und Cellulose, Öle und Fette, wie zum Beispiel Sojaöl, Sonnenblumenöl, Erdnußöl und Kokosfett, Fettsäuren, wie zum Beispiel Palmitinsäure, Stearinsäure und Linolsäure, Alkohole wie zum Beispiel Glycerin, Methanol und Ethanol und organische Säuren, wie zum Beispiel Essigsäure oder Milchsäure verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden.

Das Kulturmedium muß weiterhin Salze beispielsweise in Form von Chloriden oder Sulfaten von Metallen wie beispielsweise Natrium, Kalium, Magnesium, Calcium und Eisen enthalten, wie zum Beispiel Magnesiumsulfat oder Eisensulfat, die für das Wachstum notwendig sind. Schließlich können essentielle Wuchsstoffe wie Aminosäuren beispielsweise Homoserin und Vitamine beispielsweise Thiamin, Biotin oder Pantothensäure zusätzlich zu den oben genannten Stoffen eingesetzt werden.

Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak beziehungsweise Ammoniakwasser oder saure Verbindungen wie Phosphorsäure oder Schwefelsäure in geeigneter Weise eingesetzt. Der pH wird im Allgemeinen auf einen Wert von 6,0 bis 8,5 vorzugsweise 6,5 bis 8 eingestellt. Zur Kontrolle der Schaumentwicklung können Antischaummittel, wie zum Beispiel Fettsäurepolyglykolester eingesetzt werden. Zur Aufrechterhaltung der Stabilität von Plasmiden können dem Medium geeignete, selektiv wirkende Stoffe, wie zum Beispiel Antibiotika hinzugefügt werden. Die Fermentation wird bevorzugt unter aeroben Bedingungen durchgeführt. Um diese aufrechtzuerhalten, werden Sauerstoff oder Sauerstoff-haltige Gasmischungen, wie zum Beispiel Luft in die Kultur eingetragen. Die Verwendung von Flüssigkeiten, die mit Wasserstoffperoxid angereichert sind, ist ebenfalls möglich. Gegebenenfalls wird die Fermentation bei Überdruck, beispielsweise bei einem Überdruck von 0,03 bis 0,2 MPa, gefahren. Die Temperatur der Kultur liegt normalerweise bei 20°C bis 45°C und vorzugsweise bei 25°C bis 40°C, besonders bevorzugt bei 30° bis 37°C. Bei batch-Verfahren wird die Kultivierung bevorzugt solange fortgesetzt, bis sich eine für die Maßnahme der Gewinnung der gewünschten organisch chemischen Verbindung ausreichende Menge, gebildet hat. Dieses Ziel wird normalerweise innerhalb von 10 Stunden bis 160 Stunden erreicht. Bei kontinuierlichen Verfahren sind längere Kultivierungszeiten möglich. Durch die Tätigkeit der Mikroorganismen kommt es zu einer Anreicherung (Akkumulation) der organisch chemischen Verbindung im Fermentationsmedium und/oder in den Zellen der Mikroorganismen.

Beispiele für geeignete Fermentationsmedien finden sich unter anderem in den Patentschriften 5,770,409, US 5,990,350, US 5,275,940, WO 2007/012078, US 5,827,698, WO 2009/043803, US 5,756,345 oder US 7,138,266.

Die Analyse von L-Aminosäuren zur Bestimmung der Konzentration zu einem oder mehreren Zeitpunkt(en) im Verlauf der Fermentation kann durch Trennung der L-Aminosäuren mittels lonenaustauschchromatographie vorzugsweise Kationenaustauschchromatographie mit anschließender Nachsäulenderivatisierung unter Verwendung von Ninhydrin erfolgen, so wie bei Spackman et al. (Analytical Chemistry 30: 1190-1206 (1958)) beschrieben. Anstelle von Ninhydrin kann auch ortho-Phtadialdehyd zur Nachsäulenderivatisierung eingesetzt werden. Einen Übersichtsartikel zur lonenaustauschchromatographie findet man bei Pickering (LC·GC (Magazine of Chromatographic Science) 7(6), 484-487 (1989)).

Es ist ebenfalls möglich eine Vorsäulenderivatisierung beispielsweise unter Verwendung von ortho-Phtadialdehyd oder Phenylisothiocyanat vorzunehmen und die entstandenen Aminosäurederivate durch Reversed-Phase-Chromatographie (RP) vorzugsweise in Form der Hochleistungsflüssigkeits-chromatographie (HPLC) aufzutrennen. Eine derartige Methode ist beispielsweise bei Lindroth et al. (Analytical Chemistry 51: 1167-1174 (1979)) beschrieben.

Die Detektion erfolgt photometrisch (Absorption, Fluoreszenz).

Eine zusammenfassende Darstellung zur Aminosäureanalyse findet man unter anderem im Lehrbuch "Bioanalytik" von Lottspeich und Zorbas (Spektrum Akademischer Verlag, Heidelberg, Deutschland 1998).

Die Leistung der erfindungsgemäßen Verfahren bzw. Fermentationsprozesse bezüglich eines oder mehrerer der Parameter ausgewählt aus der Gruppe der Konzentration (gebildete Verbindung pro Volumen), der Ausbeute (gebildete Verbindung pro verbrauchter KohlenstoffQuelle), der Bildung (gebildete Verbindung pro Volumen und Zeit) und der spezifischen Bildung (gebildete Verbindung pro Zelltrockenmasse bzw. Biotrockenmasse und Zeit oder gebildete Verbindung pro Zellprotein und Zeit) oder auch anderer Prozess-Parameter und Kombinationen davon, wird um mindestens 0,5%, mindestens 1%, mindestens 1,5% oder mindestens 2% erhöht, bezogen auf Verfahren bzw. Fermentationsprozesse mit Mikroorganismen, in denen die Aktivität eines Trehalose-Importers erhöht vorliegt.

Durch die Maßnahmen der Fermentation erhält man eine Fermentationsbrühe, welche die gewünschte organisch chemische Verbindung, bevorzugt L-Aminosäure, enthält.

Anschließend erfolgt die Bereitstellung bzw. Herstellung oder Gewinnung eines die organisch chemische Verbindung enthaltenden Produktes in flüssiger oder fester Form.

Unter einer Fermentationsbrühe versteht man ein Fermentationsmedium bzw. Nährmedium, in dem ein Mikroorganismus für eine gewisse Zeit und bei einer gewissen Temperatur kultiviert wurde. Das Fermentationsmedium beziehungsweise die während der Fermentation eingesetzten Medien enthält/enthalten sämtliche Substanzen beziehungsweise Komponenten, die eine Produktion der gewünschten Verbindung und typischerweise die Vermehrung bzw. Lebensfähigkeit sicherstellen.

Bei Abschluss der Fermentation enthält die entstandene Fermentationsbrühe dementsprechend
a) die infolge der Vermehrung der Zellen des Mikroorganismus entstandene Biomasse (Zellmasse) des Mikroorganismus,
b) die im Laufe der Fermentation gebildete gewünschte organisch chemische Verbindung,
c) die im Laufe der Fermentation gebildeten organischen Nebenprodukte, und
d) die durch die Fermentation nicht verbrauchten Bestandteile des eingesetzten Fermentationsmediums beziehungsweise der Einsatzstoffe wie beispielsweise Vitamine wie Biotin oder Salze wie Magnesiumsulfat.

Zu den organischen Nebenprodukten gehören Stoffe, die von den bei der Fermentation eingesetzten Mikroorganismen neben der jeweiligen gewünschten Verbindung erzeugt und gegebenenfalls ausgeschieden werden. Hierzu gehören auch Zucker wie zum Beispiel Trehalose.

Die Fermentationsbrühe wird dem Kulturgefäß beziehungsweise dem Fermentationsbehälter entnommen, gegebenenfalls gesammelt, und dazu verwendet, ein die organisch chemische Verbindung enthaltendes Produkt, bevorzugt ein L-Aminosäure-haltiges Produkt in flüssiger oder fester Form bereitzustellen. Hierfür wird auch der Ausdruck "Gewinnen des L-Aminosäure haltigen Produktes" verwendet. Im einfachsten Fall stellt die dem Fermentationsbehälter entnommene L-Aminosäure-haltige Fermentationsbrühe selbst das gewonnene Produkt dar.

Durch eine oder mehrere der Maßnahmen ausgewählt aus der Gruppe
a) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung des Wassers,
b) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%) Entfernung der Biomasse, wobei diese gegebenenfalls vor der Entfernung inaktiviert wird,
c) teilweise (> 0% bis < 80%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der im Laufe der Fermentation gebildeten organischen Nebenprodukte, und
d) teilweise (> 0%) bis vollständige (100%) oder nahezu vollständige (≥ 80%, ≥ 90%, ≥ 95%, ≥ 96%, ≥ 97%, ≥ 98%, ≥ 99%, ≥ 99,3%, ≥ 99,7%) Entfernung der durch die Fermentation nicht verbrauchten Bestandteile des eingesetzten Fermentationsmediums beziehungsweise der Einsatzstoffe,
aus der Fermentationsbrühe erzielt man eine Konzentrierung bzw. Reinigung der gewünschten organisch chemischen Verbindung. Auf diese Weise werden Produkte isoliert, die einen gewünschten Gehalt an der Verbindung aufweisen.

Die teilweise (> 0% bis < 80%) bis vollständige (100%) oder nahezu vollständige (≥ 80% bis < 100%) Entfernung des Wassers (Maßnahme a)) wird auch als Trocknung bezeichnet.

In einer Variante des Verfahrens gelant man durch vollständige oder nahezu vollständige Entfernung des Wassers, der Biomasse, der organischen Nebenprodukte und der nicht verbrauchten Bestandteile des eingesetzten Fermentationsmediums gelangt man zu reinen ((≥ 80 Gew.-%, ≥ 90 Gew.-%) oder hochreinen (≥ 95 Gew.-%, ≥ 97 Gew.-%, ≥ 99% Gew.-%) Produktformen der gewünschten organisch chemischen Verbindung, bevorzugt L-Aminosäuren. Für die Maßnahmen gemäß a), b), c) oder d) sind im Stand der Technik eine Fülle von technischen Anleitungen verfügbar.

Im Falle der Aminosäure L-Lysin sind im Stand der Technik im Wesentlichen vier verschiedene Produktformen bekannt.

Eine Gruppe L-Lysin-haltiger Produkte umfasst konzentrierte, wässrige, alkalische Lösungen von aufgereinigtem L-Lysin (EP-B-0534865). Eine weitere Gruppe, wie beispielsweise in der US 6,340,486 und US 6,465,025 beschrieben, umfasst wässrige, saure, Biomasse-haltige Konzentrate von L-Lysin-haltigen Fermentationsbrühen. Die bekannteste Gruppe fester Produkte umfasst pulverförmige oder kristalline Formen von aufgereinigtem beziehungsweise reinem L-Lysin, das typischerweise in Form eines Salzes wie zum Beispiel L-Lysin-Monohydrochlorid vorliegt. Eine weitere Gruppe fester Produktformen ist beispielsweise in der EP-B-0533039 beschrieben. Die dort beschriebene Produktform enthält neben L-Lysin den größten Teil der während der fermentativen Herstellung verwendeten und nicht verbrauchten Einsatzstoffe und gegebenfalls die Biomasse des eingesetzten Mikroorganismus mit einem Anteil von >0% - 100%.

Entsprechend der verschiedenen Produktformen kennt man verschiedenste Verfahren, bei denen aus der Fermentationsbrühe das L-Lysin-haltige Produkt oder das gereinigte L-Lysin hergestellt wird.

Zur Herstellung von festem, reinem L-Lysin werden im Wesentlichen Methoden der lonenaustauschchromatographie gegebenfalls unter Verwendung von Aktivkohle und Methoden der Kristallisierung angewendet. Auf diese Weise erhält man die entsprechende Base oder ein entsprechendes Salz wie beispielsweise das Monohydrochlorid (Lys-HCl) oder das Lysinsulfat (Lys₂-H₂SO₄).

In der EP-B-0534865 ist ein Verfahren zur Herstellung wässriger, basischer L-Lysin-haltiger Lösungen aus Fermentationsbrühen beschrieben. Bei dem dort beschriebenen Verfahren wird die Biomasse aus der Fermentationsbrühe abgetrennt und verworfen. Mittels einer Base wie beispielsweise Natrium-, Kalium- oder Ammoniumhydroxid wird ein pH-Wert zwischen 9 bis 11 eingestellt. Die mineralischen Bestandteile (anorganischen Salze) werden nach Konzentrierung und Abkühlung durch Kristallisation aus der Brühe abgetrennt und entweder als Dünger verwendet oder verworfen.

Bei Verfahren zur Herstellung von Lysin unter Verwendung von Bakterien der Gattung Corynebacterium werden solche Verfahren bevorzugt, bei denen Produkte erhalten werden, die Bestandteile der Fermentationsbrühe enthalten. Diese werden insbesondere als Tierfuttermitteladditive verwendet.

Je nach Anforderung kann die Biomasse ganz oder teilweise durch Separationsmethoden wie z.B. der Zentrifugation, der Filtration, dem Dekantieren oder einer Kombination hieraus aus der Fermentationsbrühe entfernt oder vollständig in ihr belassen werden. Gegebenenfalls wird die Biomasse beziehungsweise die Biomasse enthaltene Fermentationsbrühe während eines geeigneten Verfahrensschrittes inaktiviert beispielsweise durch thermische Behandlung (Erhitzen) oder durch Säurezugabe.

In einer Verfahrensweise wird die Biomasse vollständig oder nahezu vollständig entfernt, sodass keine (0 %) oder höchstens 30%, höchstens 20%, höchstens 10%, höchstens 5%, höchstens 1% oder höchstens 0,1% Biomasse im hergestellten Produkt verbleibt. In einer weiteren Verfahrensweise wird die Biomasse nicht oder nur in geringfügigen Anteilen entfernt, sodass sämtliche (100%) oder mehr als 70%, 80%, 90%, 95%, 99% oder 99,9% Biomasse im hergestellten Produkt verbleibt. In einem erfindungsgemäßen Verfahren wird dementsprechend die Biomasse in Anteilen ≥ 0% bis ≤ 100% entfernt.

Schließlich kann die nach der Fermentation erhaltene Fermentationsbrühe vor oder nach der vollständigen oder teilweisen Entfernung der Biomasse mit einer anorganischen Säure wie beispielsweise Salzsäure, Schwefelsäure oder Phosphorsäure oder organischen Säure wie beispielsweise Propionsäure auf einen sauren pH Wert eingestellt werden (GB 1,439,728 oder EP 1 331 220). Es ist gleichfalls möglich, die Fermentationsbrühe mit der vollständig enthaltenen Biomasse anzusäuern. Schließlich kann die Brühe auch durch Zusatz von Natriumbisulfit (NaHSO₃, GB 1,439,728) oder einem anderen Salz beispielsweise Ammonium-, Alkali- oder Erdalkalisalz der schwefligen Säure stabilisiert werden.

Bei der Abtrennung der Biomasse werden gegebenenfalls in der Fermentationsbrühe enthaltene organische oder anorganische Feststoffe teilweise oder ganz entfernt. Die in der Fermentationsbrühe gelösten organischen Nebenprodukte und die gelösten nicht verbrauchten Bestandteile des Fermentationsmediums (Einsatzstoffe) bleiben mindestens teilweise (> 0%), bevorzugt zu mindestens 25%, besonders bevorzugt zu mindestens 50% und ganz besonders bevorzugt zu mindestens 75% im Produkt. Gegebenenfalls bleiben diese auch vollständig (100%) oder nahezu vollständig, das heißt > 95% oder > 98% oder größer 99%, im Produkt. Enthält ein Produkt in diesem Sinn mindestens einen Teil der Bestandteile der Fermentationsbrühe, so wird dies auch mit dem Begriff "Produkt auf Fermentationsbrühebasis" umschrieben.

Anschließend wird der Brühe mit bekannten Methoden wie z.B. mit Hilfe eines Rotationsverdampfers, Dünnschichtverdampfers, Fallfilmverdampfers, durch Umkehrosmose oder durch Nanofiltration Wasser entzogen beziehungsweise eingedickt oder konzentriert. Diese aufkonzentrierte Fermentationsbrühe kann anschließend durch Methoden der Gefriertrocknung, der Sprühtrocknung, der Sprühgranulation oder durch anderweitige Verfahren wie zum Beispiel in der zirkulierenden Wirbelschicht gemäß PCT/EP2004/006655 beschrieben, zu rieselfähigen Produkten insbesondere zu einem feinteiligen Pulver oder vorzugsweise grobkörnigem Granulat aufgearbeitet werden. Gegebenenfalls wird aus dem erhaltenen Granulat durch Sieben oder Staubabtrennung ein gewünschtes Produkt isoliert.

Es ist ebenfalls möglich, die Fermentationsbrühe direkt, d. h. ohne vorherige Aufkonzentrierung durch Sprühtrocknung oder Sprühgranulation, zu trocknen.

Unter "rieselfähig" versteht man Pulver, die aus einer Serie von Glasauslaufgefäßen mit verschieden großen Auslauföffnungen mindestens aus dem Gefäß mit der Öffnung 5 mm (Millimeter) ungehindert auslaufen (Klein: Seifen, Öle, Fette, Wachse 94, 12 (1968)).

Mit "feinteilig" ist ein Pulver mit überwiegendem Anteil (> 50 %) einer Korngröße von 20 bis 200 µm Durchmesser gemeint.

Mit "grobkörnig" ist ein Produkt mit einem überwiegendem Anteil (> 50 %) einer Korngröße von 200 bis 2000 µm Durchmesser gemeint.

Die Korngrößenbestimmung kann mit Methoden der Laserbeugungsspektrometrie durchgeführt werden. Die entsprechenden Methoden sind im Lehrbuch zur "Teilchengrößenmessung in der Laborpraxis" von R. H. Müller und R. Schuhmann, Wissenschaftliche Verlagsgesellschaft Stuttgart (1996) oder im Lehrbuch "Introduction to Particle Technology" von M. Rhodes, Verlag Wiley & Sons (1998) beschrieben.

Das rieselfähige, feinteilige Pulver kann wiederum durch geeignete Kompaktier- oder Granulier-Verfahren in ein grobkörniges, gut rieselfähiges, lagerfähiges und weitgehend staubfreies Produkt überführt werden.

Der Begriff "staubfrei" bedeutet, daß das Produkt lediglich geringe Anteile (< 5 %) an Korngrößen unter 100 µm Durchmesser enthält.

"Lagerfähig", im Sinne dieser Erfindung, bedeutet ein Produkt, das mindestens ein (1) Jahr oder länger, bevorzugt mindestens 1,5 Jahre oder länger, besonders bevorzugt zwei (2) Jahre oder länger in trockener und kühler Umgebung gelagert werden kann, ohne dass ein wesentlicher Verlust (maximal 5%) der jeweiligen Aminosäure auftritt.

Ein weiterer Gegenstand der Erfindung ist ein Verfahren, das in seinen Grundzügen in der WO 2007/042363 A1 beschrieben ist. Hierfür wird unter Verwendung der erfindungsgemäß erhaltenen Fermentationsbrühe, aus der die Biomasse gegebenenfalls ganz oder teilweise abgetrennt wurde, ein Verfahren durchgeführt, das folgende Schritte umfasst:
a) den pH-Wert durch Zugabe von Schwefelsäure auf 4,0 bis 5,2, insbesondere 4,9 bis 5,1, absenkt, und ein molares Sulfat/L-Lysin-Verhältnis von 0,85 bis 1,2, bevorzugt 0,9 bis 1,0, besonders bevorzugt >0,9 bis <0,95, in der Brühe einstellt, gegebenenfalls durch Zugabe von einer weiteren oder mehreren Sulfat-haltigen Verbindung(en) und
b) das so erhaltene Gemisch durch Wasserentzug aufkonzentriert und gegebenenfalls granuliert,
   wobei gegebenenfalls vor Schritt a) eine oder beide der folgenden Maßnahmen durchgeführt wird/werden:
c) Messung des molaren Verhältnisses von Sulfat/L-Lysin zur Ermittlung der benötigten Menge an Sulfat-haltigen Verbindung(n)
d) Zusatz einer Sulfat-haltigen Verbindung ausgewählt aus der Gruppe Ammoniumsulfat, Ammoniumhydrogensulfat und Schwefelsäure in entsprechenden Verhältnissen.

Gegebenenfalls wird weiterhin vor Schritt b) ein Salz der schwefligen Säure, bevorzugt Alkalihydrogensulfit, besonders bevorzugt Natriumhydrogensulfit in einer Konzentration von 0,01 bis 0,5 Gew.-%, bevorzugt 0,1 bis 0,3 Gew.-%, besonders bevorzugt 0,1 bis 0,2 Gew.-% bezogen auf die Fermentationsbrühe hinzugesetzt.

Als bevorzugte Sulfat-haltige Verbindungen im Sinne der oben genannten Verfahrensschritte sind insbesondere Ammoniumsulfat und/oder Ammoniumhydrogensulfat oder entsprechende Mischungen von Ammoniak und Schwefelsäure und Schwefelsäure selbst zu nennen.

Das molare Sulfat/L-Lysin-Verhältnis V wird nach der Formel: V = 2 x [SO₄²⁻] / [L-Lysin] berechnet. Diese Formel berücksichtigt die Tatsache, dass das SO₄²⁻ Anion, bzw. die Schwefelsäure zweiwertig ist. Ein Verhältnis V = 1 bedeutet, dass ein stöchiometrisch zusammengesetztes Lys₂₋H₂SO₄) vorliegt, während bei einem Verhältnis von V = 0,9 ein 10% iger Sulfatmangel und bei einem Verhältnis von V = 1,1 ein 10% Sulfatüberschuss gefunden wird.

Vorteilhaft bei der Granulation oder Kompaktierung ist der Einsatz von üblichen organischen oder anorganischen Hilfsstoffen, beziehungsweise Trägern wie Stärke, Gelatine, Cellulosederivaten oder ähnlichen Stoffen, wie sie üblicherweise in der Lebensmittel- oder Futterverarbeitung als Binde-, Gelier-, oder Verdickungsmittel Verwendung finden, oder von weiteren Stoffen wie zum Beispiel Kieselsäuren, Silikaten (EP0743016A) und Stearaten.

Weiterhin ist es vorteilhaft, die Oberfläche der erhaltenen Granulate mit Ölen oder Fetten zu behandeln so wie es in der WO 04/054381 beschrieben ist. Als Öle können Mineralöle, pflanzliche Öle oder Mischungen pflanzlicher Öle verwendet werden. Beispiele für derartige Öle sind Sojaöl, Olivenöl, Sojaöl/Lecithingemische. In gleicher Weise sind auch Silikonöle, Polyethylenglykole oder Hydroxyethylcellulose geeignet. Durch die Behandlung der Oberflächen mit den genannten Ölen erzielt man eine erhöhte Abriebfestigkeit des Produktes und einen Verringerung des Staubanteils. Der Gehalt an Öl im Produkt beträgt 0,02 bis 2,0 Gew.-%, bevorzugt 0,02 bis 1,0 Gew.-%, und ganz besonders bevorzugt 0,2 bis 1,0 Gew.-% bezogen auf die Gesamtmenge des Futtermitteladditivs.

Bevorzugt sind Produkte mit einem Anteil von ≥ 97 Gew.-% einer Korngröße von 100 bis 1800 µm oder einem Anteil von ≥ 95 Gew.-% einer Korngröße von 300 bis 1800 µm Durchmesser. Der Anteil an Staub, d. h. Partikeln mit einer Korngrösse < 100 µm, liegt bevorzugt bei > 0 bis 1 Gew.- besonders bevorzugt bei maximal 0,5 Gew.-%.

Alternativ kann das Produkt aber auch auf einen in der Futtermittelverarbeitung bekannten und üblichen organischen oder anorganischen Trägerstoff wie zum Beispiel Kieselsäuren, Silikate, Schrote, Kleien, Mehle, Stärken, Zucker oder andere aufgezogen und/oder mit üblichen Verdickungs- oder Bindemitteln vermischt und stabilisiert werden. Anwendungsbeispiele und Verfahren hierzu sind in der Literatur (Die Mühle + Mischfuttertechnik 132 (1995) 49, Seite 817) beschrieben.

Schließlich kann das Produkt auch durch Beschichtungsverfahren ("Coating") mit Filmbildnern wie beispielsweise Metallcarbonaten, Kieselsäuren, Silikaten, Alginaten, Stearaten, Stärken, Gummis und Celluloseether, wie in der DE-C-4100920 beschrieben, in einen Zustand gebracht werden, in dem es stabil gegenüber der Verdauung durch Tiermägen, insbesondere dem Magen von Wiederkäuern, ist.

Zur Einstellung einer gewünschten L-Lysin Konzentration im Produkt kann je nach Anforderung das L-Lysin während des Verfahrens in Form eines Konzentrates oder gegebenenfalls einer weitgehend reinen Substanz beziehungsweise dessen Salz in flüssiger oder fester Form hinzugefügt werden. Diese können einzeln oder als Mischungen zur erhaltenen oder aufkonzentrierten Fermentationsbrühe, oder auch während des Trocknungs- oder Granulationsprozesses, hinzugefügt werden.

Ein weiterer Gegenstand der Erfindung ist ein Verfahren zur Herstellung eines festen Lysin-haltigen Produktes, das in seinen Grundzügen in der US 20050220933 beschrieben ist. Hierbei wird unter Verwendung der erfindungsgemäß erhaltenen Fermentationsbrühe ein Verfahren durchgeführt, das folgende Schritte umfasst:
a) Filtration der Fermentationsbrühe, bevorzugt mit einem Membranfilter, so dass ein Biomasse-haltiger Schlamm und ein Filtrat erhalten wird,
b) Aufkonzentration des Filtrates, bevorzugt so, dass ein Feststoffgehalt von 48 bis 52 Gew.-% erhalten wird,
c) Granulation des in Schritt b) erhaltenen Konzentrates, bevorzugt bei einer Temperatur von 50°C bis 62°C, und
d) Beschichtung des in c) erhaltenen Granulates mit einem oder mehreren der Beschichtungsmittel (coating agent(s))

Die Aufkonzentrierung des Filtrates in Schritt b) kann auch derart erfolgen, dass ein Feststoffgehalt von > 52 bis ≤ 55 Gew.-%, von > 55 bis ≤ 58 Gew.-% oder von > 58 bis ≤ 61 Gew.-% erhalten wird.

Zur Beschichtung in Schritt d) werden bevorzugt Beschichtungsmittel verwendet, welche ausgewählt werden aus der Gruppe, bestehend aus
d1) der in Schritt a) erhaltenen Biomasse,
d2) einer L-Lysin-haltigen Verbindung, bevorzugt ausgewählt aus der Gruppe L-Lysinhydrochlorid oder L-Lysinsulfat,
d3) einem im wesentlichen L-Lysin-freien Stoff mit L-Lysingehalt < 1 Gew.-%, bevorzugt < 0,5 Gew.-%, bevorzugt ausgewählt aus der Gruppe Stärke, Karageenan, Agar, Kieselsäuren, Silikate, Schrote, Kleien und Mehle, und
d4) einem wasserabstoßenden Stoff, bevorzugt ausgewählt aus der Gruppe Öle, Polyethylenglykole und flüssige Paraffine.

Durch die Maßnahmen entsprechend den Schritten d1) bis d4), insbesondere d1) bis d3), wird der Gehalt an L-Lysin auf einen gewünschten Wert eingestellt.

Bei der Herstellung L-Lysin-haltiger Produkte wird das Verhältnis der Ionen bevorzugt so eingestellt, dass das molare Ionenverhältnis entsprechend nachstehender Formel

2x[SO₄²⁻]+[Cl⁻]-[NH₄⁺]-[Na⁺]-[K⁺]-2x[Mg²⁺]-2x[Ca²⁺]/[L-Lys]

0,68 bis 0,95, bevorzugt 0,68 bis 0,90, besonders bevorzugt 0,68 bis 0,86 ergibt, so wie von Kushiki et al. in der US 20030152633 beschrieben.

Im Falle des L-Lysins hat das auf diese Weise hergestellte feste Produkt auf Fermentationsbrühebasis einen Lysingehalt (als Lysinbase) von 10 Gew.-% bis 70 Gew.-% oder 20 Gew.-% bis 70 Gew.-%, bevorzugt 30 Gew.-% bis 70 Gew.-% und ganz besonders bevorzugt von 40 Gew.-% bis 70 Gew.-% bezogen auf die Trockenmasse des Produkts. Maximale Gehalte an Lysinbase von 71 Gew.-%, 72 Gew.-%, 73 Gew.-% sind ebenfalls möglich.

Der Wassergehalt des L-Lysin-haltigen, festen Produktes beträgt bis zu 5 Gew.-%, bevorzugt bis zu 4 Gew.-%, und besonders bevorzugt weniger als 3 Gew.-%.

Der Stamm DM1729 wurde am 16. September 2005 bei der Deutschen Sammlung für Mikroorganismen und Zellkulturen unter der Zugangsnummer DSM17576 hinterlegt.

### Beschreibung der Figuren

**Figur 1** zeigt die Anordnung der offenen Leserahmen cg0835 bis cg0830. Die Leserahmen kodieren für folgende mutmaßliche Proteine: cg0835: ATPase; cg0834 periplasmatisches Substratbinderotein; cg0832: Permeaseuntereinheit; cg0831 Permeaseuntereinheit.
**Figur 2** zeigt eine schematische Darstellung des Expressionskonstruktes pXMJ19-cg0831. Die folgende Tabelle 2 fasst die verwendeten Abkürzungen und Bezeichnungen zusammen sowie deren Bedeutung. Bei der Angabe der Basenpaarzahlen handelt es sich um Näherungswerte, die im Rahmen der Reproduzierbarkeit von Messungen erhalten werden.

**Tabelle 2:**

| | |
|---|---|
| cat | Chloramphenicol Resistenz-Gen |
| lacl | Codiert für Lac-Repressor |
| Ptac | tac-Promoter |
| oriCg | origin of Corynebacterium glutamicum plasmid pBL1 |
| ori pUC | origin of Escherichia coli plasmid pUC |
| TrrnB | rrnB Terminator |
| cg0831 | codiert für Permeaseuntereinheit |
| cg0832 | codiert für Permeaseuntereinheit |
| cg0833 | codiert für unbekanntes Protein |
| cg0834 | codiert für periplasmatisches Substratbindeprotein |
| cg0835 | codiert für ATPase |

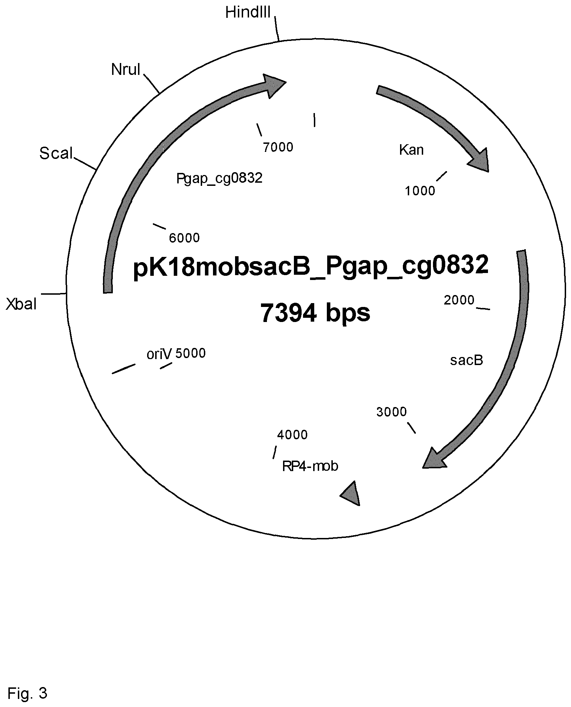

**Figur 3** zeigt eine schematische Darstellung des Plasmids pK18mobsacB_Pgap_cg0832, das zur funktionellen Verknüpfung des ORFs cg0832 mit dem Promotor Pgap verwendet wurde.

Die folgende Tabelle 3 fasst die verwendeten Abkürzungen und Bezeichnungen zusammen sowie deren Bedeutung. Die verwendeten Abkürzungen und Bezeichnungen haben folgende Bedeutung. Bei der Angabe der Basenpaarzahlen handelt es sich um Näherungswerte, die im Rahmen der Reproduzierbarkeit von Messungen erhalten werden.

**Tabelle 3:**

| | |
|---|---|
| Kan: | Kanamycin Resistenz-Gen |
| NruI | Schnittstelle des Restriktionsenzyms Nrul |
| HindIII | Schnittstelle des Restriktionsenzyms HindIII |
| ScaI | Schnittstelle des Restriktionsenzyms ScaI |
| XbaI | Schnittstelle des Restriktionsenzyms Xbal |
| Pgap_cg0832 | DNA-Kassette zur Etablierung einer funktionellen Verküpfung des ORF cg0832 und dem Promotor Pgap. |
| sacB: | sacB-Gen |
| RP4-mob: | mob-Region mit dem Replikationsursprung für den Transfer (oriT) |
| oriV: | Replikationsursprung V |

### Beispiele

### Beispiel 1: Identifizierung eines Trehalose Aufnahmesystems

Für Bakterien der Ordnung Actinomycetales, zu der auch C. glutamicum gehört, wurde bislang die Verstoffwechslung von Trehalose nur für Bakterien der Familie der Streptomycetaceae beschrieben: Streptomyces coelicolor und Streptomyces reticuli nutzen Trehalose als Kohlenstoffquelle. Analysen der Genexpression deuteten in S. coelicolor auf eine Beteiligung der durch agl3E, agl3F und agl3G codierten Komponenten eines ABC-Transportsystem und in S. reticuli der ATPase-Untereinheit MsiK an der Trehaloseaufnahme hin. Durch Analyse der Genomsequenz von C. glutamicum mittels Blast wurden zwei offene Leserahmen (cg2708 und cg0835) mit hoher Ähnlichkeit zu msiK (GenBank Accession Nr. CAA70125) aus S. reticuli identifiziert: das durch cg2708 codierte Protein aus C. glutamicum besitzt eine Identität von 59% zu MsiK aus S. reticuli (e-value 7e-125), es handelt sich dabei allerdings um das ATP-bindende Protein MusE des MusEFGK₂ Maltosetransporters, dessen Deletion sich nicht auf die Trehaloseverwertung auswirkt. Das zweite Protein, welches von cg0835 codiert wird, weist ebenfalls eine hohe Identität von 58% zu MsiK aus S. reticuli auf (e-value 8e-112). Sequenzvergleiche von agl3E, agl3F und agl3E aus S. coelicolor (Accession Nr NP_631226, NP_631225, NP_631224) mit der Genomsequenz von C. glutamicum ergaben keine weiteren sinnvollen Treffer (z.B. 25 % bis 32 % Identität mit Genen des ABC-Aufnahmesystems UgpAEBC, welches die Aufnahme von Glycerol-3-Phosphat katalysiert und Genen des Maltoseaufnahmesystems MusEFGK₂).

Aus der vergleichenden Sequenzanalyse ergibt sich daher der offene Leserahmen cg0835 und die in unmittelbarer Nähe in der Genomsequenz lokalisierten offenen Leserahmen cg0834, cg0832 und cg0831, welche für ein Substratbindeprotein und zwei Permeasekomponenten eines noch nicht charakterisierten ABC-Transporters codieren, als ein mögliches Aufnahmesystem für Trehalose in C. glutamicum (Anordnung siehe Figur 1).

### Beispiel 2: Konstruktion des Vektors pXMJ19_cg0831

Zur Herstellung des Expressionskonstruktes, das die Leseraster cg0832, cg0834, cg0833, cg0832 und cg0831 enthält, wurde der entsprechende Genbereich mittels einer proofreading Polymerase (PRECISOR High-Fidelity DNA Polymerase, Biocat, Heidelberg) amplifiziert und in den Klonierungsvektor pJet (Fermentas, St. Leon-Roth) ligiert.

Hierfür wurden folgende synthetische Oligonukleotide (Primer) verwendet:
Primer cg0831for (SEQ ID No:30):
   5' GCTCTAGATGCGTTCTGCTCCTGACCTT 3'
Primer rev (SEQ ID No:31):
   5' CGGGATCCTTTGCGTTGCGATTCGGATT 3'

Die dargestellten Primer wurden von der Firma MWG Biotech (Ebersberg, Deutschland) synthetisiert. Unterstrichen ist jeweils die Erkennungssequenz für die Restriktionsenzyme Xbal bzw. BamHI.

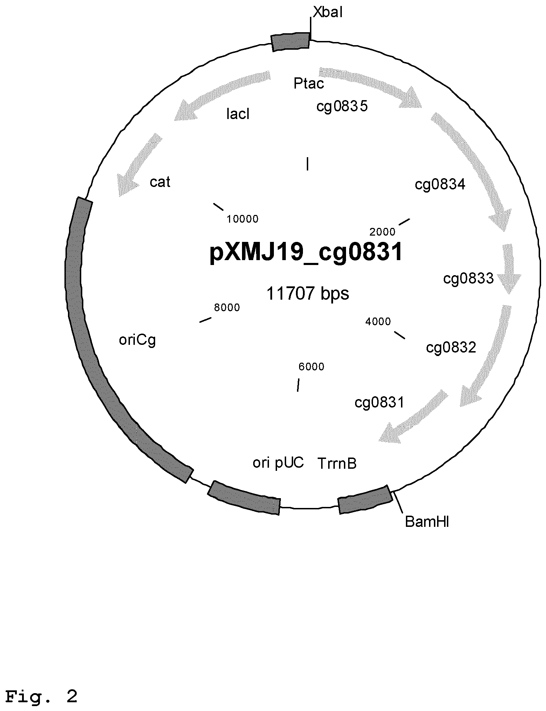

Das erhaltene Fragment wurde anschließend durch die Restriktionsenzyme Xbal und BamHI (New England Biolabs, Schwalbach, Deutschland) aus dem pJet Vektor ausgeschnitten und in den Expressionsvektor pXMJ19 (Jakoby et al., 1999), der zuvor mit Xbal und BamHI linearisiert und anschließend mit Antarctic Phosphatase (New England Biolabs, Schwalbach, Deutschland)dephosphoryliert wurde, ligiert. Anschließend wurden kompetente E. coli DH5amcr Zellen mit 5µl des Ligationsansatzes transformiert. Die erhaltenen Klone wurden durch Restriktion der präparierten Plasmide auf solche untersucht, die das gewünschte Insert enthalten. Das Plasmid hat die Bezeichnung pXMJ19_cg0831 (siehe Figur 2) erhalten.

### Beispiel 3: Herstellung des C.glutamicum Stammes DM1933 / pXMJ19 und DM1933 / pXMJ19_cg0831

Die in Beispiel 2 beschriebenen Plasmide pXMJ19 und pXMJ19_cg0831 wurden mit der Elektroporationsmethode von Liebl et al. (FEMS Microbiological Letters, 53:299-303 (1989)) in Corynebacterium glutamicum DM1933 elektroporiert.

Der Stamm DM1933 ist eine Aminoethylcystein-resistente Mutante von Corynebacterium glutamicum ATCC13032 und in einer Publikation beschrieben (Blombach et al., 2009, Appl. and Env. Microbiol., p. 419-427).

Die Selektion von Plasmid-tragenden Zellen erfolgte durch Ausplattieren des Elektroporationsansatzes auf LB Agar (Sambrook et al., Molecular cloning: a laboratory manual. 2nd Ed. Cold Spring Harbor Laboratory Press, Cold Spring Harbor, N.Y., 1989), der mit 7,5 mg/l Chloramphenicol supplementiert worden war. Plasmid DNA wurde nach den üblichen Methoden aus jeweils einer Transformante isoliert (Peters-Wendisch et al., 1998, Microbiology, 144, 915-927) und durch Restriktionsspaltung mit anschließender Agarosegel-Elektrophorese überprüft.

Die erhaltenen Stämme wurden DM1933 / pXMJ19 und DM1933 / pXMJ19_cg0831 genannt. Das Konstrukt pXMJ19_cg0831 enthält die Leseraster cg0832, cg0834, cg0833, cg0832 und cg0831.

### Beispiel 4: Herstellung von L-Lysin

Die in Beispiel 3 erhaltenen *C. glutamicum* Stämme DM1933 / pXMJ19 und DM1933 / pXMJ19_cg0831 wurden in einem zur Produktion von Lysin geeigneten Nährmedium kultiviert und der Lysingehalt im Kulturüberstand bestimmt.

Dazu wurden die Stämme zunächst auf Agarplatte mit dem entsprechenden Antibiotikum (Hirn-Herz Agar mit Chloramphenicol (7,5 mg/l)) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur und die Hauptkultur wurde das Medium MM verwendet, welches mit Chloramphenicol (7,5 mg/l) versetzt wurde.

Tabelle 4 gibt eine Übersicht über die Zusammensetzung des verwendeten Kultivierungsmediums.

**Tabelle 4: Medium MM**

| | |
|---|---|
| CSL (Corn Steep Liquor) | 5 g/l |
| MOPS (Morpholinopropansufonsäure) | 20 g/l |
| Glucose (getrennt autoklaviert) | 50 g/l |
| Salze: | |
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0 mg/l |
| Biotin (sterilfiltriert) | 0,3 mg/l |
| Thiamin * HCl (sterilfiltriert) | 0,2 mg/l |
| CaCO₃ | 25 g/l |

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃ zugesetzt.

Die Vorkultur wurde 24 Stunden bei 33°C bei 250 rpm auf dem Schüttler inkubiert. Von dieser Vorkultur wurde eine Hauptkultur angeimpft, so daß die Anfangs-OD (660 nm) der Hauptkultur 0,1 OD betrug.

Die Kultivierung erfolgte in 10 ml Volumen in einem 100 ml Erlenmeyerkolben mit Schikanen bei einer Temperatur von 33°C und 80% Luftfeuchtigkeit.

Nach 20 und 40 Stunden (h) wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch lonenaustauschchromatographie und Nachsäulenderivatisierung mit Ninhydrindetektion bestimmt. Die Konzentration an Trehalose wurde mit einem Analysator der Firma Dionex GmbH (65510 Idstein, Deutschland) mittels HPLC auf bestimmt. In Tabelle 5 ist das Ergebnis des Versuchs dargestellt.

**Tabelle 5: Herstellung von L-Lysin und Messung der Trehalose-Konzentration. Alle Werte sind Mittelwerte von 3 unabhängigen Experimenten mit den genannten Stämmen; n.b.= nicht bestimmt.**

| | L-Lysin HCl (g/l) | | OD (660nm) | | Trehalose (g/l) | |
|---|---|---|---|---|---|---|
| Stamm | 20 h | 40 h | 20 h | 40 h | 20 h | 40 h |
| DM1933 / pXMJ19 | 11,84 | 13,65 | 14,04 | 13,12 | n.b. | 3,13 |
| DM1933 / pXMJ19_cg0831 | 11,82 | 14,89 | 14,62 | 13,7 | n.b. | 0 |

Das Ergebnis zeigt, dass bei der Produktion von Lysin aus Trehalose unter Verwendung eines Trehalose-Importer exprimierenden Stammes, keine Trehalose mehr als Nebenprodukt entsteht. Weiterhin ist ersichtlich, dass die Ausbeute des gewünschten Produktes (L-Lysin) erhöht ist.

### Beispiel 5: Konstruktion des Vektors pK18mobsacB_Pgap_cg0832

Ein 1701bp großes DNA-Fragment entsprechend der Nukleotidsequenz (SEQ ID No: 26) zur Überexpression der Gene cg0831 und cg0832 wurde bei der Firma GENEART AG (Regensburg, Deutschland) mittels de novo Gensynthese hergestellt.

Die Positionen der Nukleotide 613 bis 1095 beschreiben ein Promotorfragment aus der Anmeldung US20080050786 (SEQ ID NO:20), wobei durch Mutationen der Nukleobase Thymin an Postion 1079 zur Nukleobase Guanin, der Nukleobase Thymin an Postion 1080 zur Nukleobase Cytosin und der Nukleobase Thymin an Postion 1081 zur Nukleobase Guanin, eine Schnittstelle für das Restriktionsenzym Nrul erstellt wurde. Zusätzlich wurde durch Anfügen einer Linker-Sequenz (SeqID No.28) an das 5' Ende der Promotorsequenz eine Schnittstelle für das Restriktionsenzym ScaI erstellt und befindet sich an Position 607 bis 612. Das hieraus erhaltene Promotorfragment mit einer Länge von 489 bp wurde mit dem Startcodon des Gens cg0832 funktional verknüpft.

Das Konstrukt besitzt zur Integration des Promotors mittels homologer Rekombination eine 600 bp Flanke im downstream Bereich (Positionen 1096 bis 1695), sowie eine 600 bp Flanke im upstream (Positionen 7 bis 606) Bereich des Promotors.

An die flankierenden Bereiche wurden Sequenzen mit Schnittstellen für die Restriktionsenzyme XbaI (Position 1 bis 6) und HindIII (Position 1696 bis 1701) angefügt, womit die Klonierung des Konstruktes in den Austauschvektor pK18mobsacB ermöglicht wird.

Das 1701 bp lange Fragment wurde mit den Restriktionsenzymen XbaI und HindIII verdaut und anschließend in den von Schäfer et al. (Gene, 145, 69-73 (1994)) beschriebenen, mobilisierbaren Vektor pK18mobsacB umkloniert, um eine Integration des Promotors vor das Gen cg0832 zu ermöglichen. Hierzu wurde pK18mobsacB mit den Restriktionsenzymen XbaI und HindIII verdaut. Der so vorbereitete Vektor wurde mit dem Fragment gemischt und der Ansatz mit dem Ready-To-Go T4 DNA Ligase Kit (Amersham-Pharmacia, Freiburg, Deutschland) behandelt.

Anschließend wurde der E.coli Stamm S17-1 (Simon et al., Bio/Technologie 1: 784-791, 1993) mit dem Ligationsansatz transformiert (Hanahan, In. DNA cloning. A practical approach. Vol.1. ILR-Press, Cold Spring Habor, New York, 1989). Die Selektion auf Plasmid-tragende Zellen erfolgte durch Ausplattieren des Tansformationsansatzes auf LB-Agar (Sambrock et al., Molecular Cloning: a laboratory manual. 2nd Ed. Cold Spring Habor, New York, 1989), der mit 50 mg/l Kanamycin supplementiert worden war.

Plasmid-DNA wurde aus einer Transformante mit Hilfe des QIAprep Spin Miniprep Kit der Firma Qiagen isoliert und durch Restriktionsspaltung mit den Enzymen Xbal und HindIII, sowie anschließender Agarosegel-Elektrophorese überprüft. Das Plasmid trägt die Bezeichnung pK18mobsacB_Pgap_cg0832 und ist in Figur 3 dargestellt.

### Beispiel 6: Herstellung des C.glutamicum Stammes DM1933_ Pgap_cg0832

Ziel war es die Mutation Pgap_cg0832 in den Stamm Corynebacterium glutamicum DM1933 einzubringen. Der Stamm DM1933 ist eine Aminoethylcystein-resistente Mutante von Corynebacterium glutamicum ATCC13032 und in einer Publikation beschrieben (Blombach et al., 2009, Appl. and Env. Microbiol., p. 419-427).

Der in Beispiel 5 beschriebene Vektor pK18mobsacB_Pgap_cg0832 wurde nach dem Protokoll von Schäfer et al. (Journal of Microbiology 172: 1663-1666 (1990)) in den C. glutamicum Stamm DM1933 durch Konjugation transferiert. Der Vektor kann in DM1933 nicht selbständig replizieren und bleibt nur dann in der Zelle erhalten, wenn er als Folge eines Rekombinationsereignisses im Chromosom integriert vorliegt. Die Selektion von Transkonjuganten, d.h. von Klonen mit integriertem pK18mobsacB_Pgap_cg0832 erfolgte durch Ausplattieren des Konjugationsansatzes auf LB-Agar, der mit 25 mg/l Kanamycin und 50 mg/l Nalidixinsäure supplementiert worden war. Kanamycin-resistente Transkonjuganten wurden anschließend auf mit Kanamycin (25 mg/l) supplementierten LB-Agarplatten ausgestrichen und für 24 Stunden bei 33°C inkubiert. Zur Selektion von Mutanten, bei denen als Folge eines zweiten Rekombinationsereignisses die Exzision des Plasmides stattgefunden hatte, wurden die Klone 30 Stunden unselektiv in LB-Flüssigmedium kultiviert, anschließend auf LB-Agar, der mit 10% Sucrose supplementiert worden war ausgestrichen und 24 Stunden bei 33°C bebrütet.

Das Plasmid pK18mobsacB_Pgap_cg0832 enthält ebenso wie das Ausgangsplasmid pK18mobsacB neben dem Kanamycin-Resistenzgen eine Kopie des für die Levan-Sucrase aus Bacillus subtilis kodierenden sacB-Gens. Die durch Sucrose induzierbare Expression des sacB-Gens führt zur Bildung der Levan-Sucrase, welche die Synthese des für C. glutamicum toxischen Produktes Levan katalysiert. Auf mit Sucrose supplementiertem LB-Agar wachsen daher nur solche Klone, bei denen das integrierte pK18mobsacB_Pgap_cg0832 als Folge eines zweiten Rekombinationsereignisses exzisiert hat. In Abhängigkeit von der Lage des zweiten Rekombinationsereignisses in bezug auf den Mutationsort findet bei der Exzision der Einbau der Mutation statt oder es verbleibt der ursprüngliche Zustand im Chromosom des Wirtes.

Anschließend wurde ein Klon ermittelt, bei dem der gewünschte Austausch, d. h. der Einbau der Kassette Pgap_cg0832 in das Chromosom, erfolgt war.

Hierzu wurden 50 Klone mit dem Phänotyp "Wachstum in Gegenwart von Sucrose" und "Nicht-Wachstum in Gegenwart von Kanamycin" auf Integration der Kassette Pgap_cg0832 unter Anwendung der Polymerase-Kettenreaktion (PCR) überprüft.

Hierfür wurden folgende synthetische Oligonukleotide (Primer) verwendet:
Primer cg0832_1.p (SEQ ID NO:28):
   5' GCTGGAATACGGAGTGAACC 3'
Primer cg0832_2.p (SEQ ID NO:29):
   5' GGGATTGCCCAAGGGATAAG 3'

Die dargestellten Primer wurden von der Firma MWG Biotech (Ebersberg, Deutschland) synthetisiert. Die Primer cg0832_1.p und cg0832_2.p ermöglichen die Amplifikation eines 570 bp großen DNA-Fragmentes bei Vorliegen der wildtypischen Anordnung. Die Amplifikatgröße beträgt 1059 bp bei Integration des Konstruktes Pgap_cg0832 in das Chromosom.

Die PCR-Reaktionen wurde mit dem Taq PCR Core Kit von Quiagen (Hilden, Deutschland), enthaltend die Taq DNA Polymerase aus *Thermus aquaticus,* in einem Mastercycler der Firma Eppendorf (Hamburg, Deutschland)durchgeführt. Die Bedingungen im Reaktionsansatz wurden nach Angaben des Herstellers eingestellt. Der PCR-Ansatz wurde zuerst einer einleitenden Denaturierung bei 94°C für 2 Minuten unterzogen. Darauf folgten sich 35x wiederholend ein Denaturierungsschritt bei 94°C für 30 Sekunden, ein Schritt zum Binden der Primer an die DNA bei 57°C für 30 Sekunden und der Extensions-Schritt zur Verlängerung der Primer bei 72°C für 60 sec. Nach dem abschliessenden Extensions-Schritt für 5 min bei 72°C wurden die so amplifizierten Produkte in einem Agarosegel elektrophoretisch geprüft.

Auf diese Weise wurden Mutanten identifiziert, welche die Kassette Pgap_cg0832 in Form einer Integration vorliegen haben, wobei einer der erhaltenen Stämme C. glutamicum DM1933_Pgap_cg0832 genannt wurde.

### Beispiel 7: Herstellung von L-Lysin

Der in Beispiel 6 erhaltene *C. glutamicum* Stamm DM1933_Pgap_cg0832 und der Ausgangsstamm DM1933 wurden in einem zur Produktion von Lysin geeigneten Nährmedium kultiviert und der Lysingehalt im Kulturüberstand bestimmt.

Dazu wurden die Stämme zunächst auf Agarplatte (Hirn-Herz Agar) für 24 Stunden bei 33°C inkubiert. Ausgehend von dieser Agarplattenkultur wurde eine Vorkultur angeimpft (10 ml Medium im 100 ml Erlenmeyerkolben). Als Medium für die Vorkultur und die Hauptkultur wurde das Medium MM verwendet (siehe Tabelle 4).

CSL, MOPS und die Salzlösung wurden mit Ammoniakwasser auf pH 7 eingestellt und autoklaviert. Anschließend wurden die sterilen Substrat- und Vitaminlösungen zugesetzt, sowie das trocken autoklavierte CaCO₃ zugesetzt.

Die Vorkultur wurde 24 Stunden bei 33°C bei 250 rpm auf dem Schüttler inkubiert. Von dieser Vorkultur wurde eine Hauptkultur angeimpft, so daß die Anfangs-OD (660 nm) der Hauptkultur 0,1 OD betrug.

Die Kultivierung erfolgte in 10 ml Volumen in einem 100 ml Erlenmeyerkolben mit Schikanen bei einer Temperatur von 33°C und 80% Luftfeuchtigkeit.

Nach 20 und 40 Stunden (h) wurde die OD bei einer Meßwellenlänge von 660 nm mit dem Biomek 1000 (Beckmann Instruments GmbH, München) ermittelt. Die gebildete Lysinmenge wurde mit einem Aminosäureanalysator der Firma Eppendorf-BioTronik (Hamburg, Deutschland) durch lonenaustauschchromatographie und Nachsäulenderivatisierung mit Ninhydrindetektion bestimmt. Die Konzentration an Trehalose wurde mit einem Analysator der Firma Dionex GmbH (65510 Idstein, Deutschland) mittels HPLC auf bestimmt. In Tabelle 6 ist das Ergebnis des Versuchs dargestellt.

**Tabelle 6: Herstellung von L-Lysin und Messung der Trehalose-Konzentration. Alle Werte sind Mittelwerte von 3 unabhängigen Experimenten mit den genannten Stämmen; n.b.= nicht bestimmt.**

| | L-Lysin HCl (g/l) | | OD (660nm) | | Trehalose (g/l) | |
|---|---|---|---|---|---|---|
| Stamm | 20 h | 40 h | 20 h | 40 h | 20 h | 40 h |
| DM1933 | 12,83 | 13,65 | 14,75 | 12,19 | n.b. | 3,03 |
| DM1933_Pgap_cg0832 | 12,91 | 14,15 | 15,11 | 12,34 | n.b. | 0 |

Das Ergebnis zeigt, dass bei der Produktion von Lysin aus Trehalose unter Verwendung eines Stammes, der lediglich die von cg0832 und cg0831 kodierten Untereinheiten (jeweils eine Permeaseuntereinheit) des Trehalose-Importers verstärkt exprimiert, keine Trehalose mehr als Nebenprodukt entsteht. Weiterhin ist ersichtlich, dass die Ausbeute des gewünschten Produktes (L-Lysin) erhöht ist.

## Patentansprüche

1. Ein Mikroorganismus der Art Corynebacterium glutamicum, der eine organisch-chemische Verbindung produziert und/oder ausscheidet, **dadurch gekennzeichnet, dass** der Mikroorganismus eine im Vergleich zu dem jeweiligen Ausgangstamm erhöhte Expression eines Polynukleotids kodierend für ein Polypeptid mit einer Aminosäuresequenz, die eine Identität von 70% oder mehr zu der in SEQ ID NO:8 oder 20 gezeigten Aminosäuresequenz aufweist,
wobei die erhöhte Expression des Polynukleotids durch eine oder mehrere Maßnahmen ausgewählt aus der folgenden Gruppe erhöht wird:
a) die Expression des Gens steht unter der Kontrolle eines Promotors, der in dem für das Verfahren verwendeten Mikroorganismus stärker ist als der ursprüngliche Promotor des Gens;
b) Erhöhung der Kopienzahl des Gens kodierend für ein Polypeptid mit der Aktivität eines Trehalose-Importers; vorzugsweise durch Einfügen des Gens in Plasmiden mit erhöhter Kopienzahl und/oder durch Integration des Gens in das Chromosom des Mikroorganismus in mindestens einer Kopie;
c) die Expression des Gens erfolgt unter Verwendung einer Ribosomen-Bindestelle, die in dem für das Verfahren verwendeten Mikroorganismus stärker ist als die ursprüngliche Ribosomen-Bindestelle des Gens;
d) die Expression des Gens erfolgt unter Optimierung der Kodon-Verwendung (codon usage) des für das Verfahren verwendeten Mikroorganismus;
e) die Expression des Gens erfolgt unter Reduzierung von mRNA Sekundärstrukturen in der vom Gen transkribierten mRNA;
f) die Expression des Gens erfolgt unter Eliminierung von RNA-Polymerase-Terminatoren in der vom Gen transkribierten mRNA;
g) die Expression des Gens erfolgt unter Verwendung mRNA-stabilisierender Sequenzen in der vom Gen transkribierten mRNA.
und wobei dieses Polypeptid, welches eine Untereinheit eines Proteinkomplexes mit der Aktivität eines Trehalose-Importers ist, die Aktivität einer Permease aufweist und wobei der Mikroorganismus in der Lage ist, Trehalose aus dem Medium aufzunehmen.

2. Der Mikroorganismus gemäß Anspruch 1, **dadurch gekennzeichnet, dass** der Mikroorganismus im Vergleich zu dem jeweiligen Ausgangstamm zusätzlich zu der erhöhten Expression eines Polynukleotids kodierend für ein Polypeptid mit einer Aminosäuresequenz, die eine Identität von 70% oder mehr zu der in SEQ ID NO:8 oder 20 gezeigten Aminosäuresequenz aufweist, eine erhöhte Expression mindestens eines Polynukleotids ausgewählt aus der Gruppe a) bis e) aufweist:
a) ein Polynukleotid kodierend für ein Polypeptid mit einer Aminosäuresequenz, die eine Identität von 70% oder mehr zu der in SEQ ID NO:2 oder 14 gezeigten Aminosäuresequenz aufweist;
b) ein Polynukleotid kodierend für ein Polypeptid mit einer Aminosäuresequenz, die eine Identität von 70% oder mehr zu der in SEQ ID NO:4 oder 16 gezeigten Aminosäuresequenz aufweist;
c) ein Polynukleotid kodierend für ein Polypeptid mit einer Aminosäuresequenz, die eine Identität von 70% oder mehr zu der in SEQ ID NO:6 oder 18 gezeigten Aminosäuresequenz aufweist;
d) ein Polynukleotid kodierend für ein Polypeptid mit einer Aminosäuresequenz, die eine Identität von 70% oder mehr zu der in SEQ ID NO:10 oder 22 gezeigten Aminosäuresequenz aufweist;
e) ein Polynukleotid kodierend für ein Polypeptid mit einer Aminosäuresequenz, die eine Identität von 70% oder mehr zu der in SEQ ID NO:12 oder 24 gezeigten Aminosäuresequenz aufweist.

3. Der Mikroorganismus gemäß Anspruch 2, **dadurch gekennzeichnet, dass** der Mikroorganismus im Vergleich zu dem jeweiligen Ausgangstamm zusätzlich zu der erhöhten Expression eines Polynukleotids kodierend für ein Polypeptid mit einer Aminosäuresequenz, die eine Identität von 70% oder mehr zu der in SEQ ID NO:8 oder 20 gezeigten Aminosäuresequenz aufweist, eine erhöhte Expression eines Polynukleotids aufweist, kodierend für ein Polypeptid mit einer Aminosäuresequenz, die eine Identität von 70% oder mehr zu der in SEQ ID NO:10 oder 22 gezeigten Aminosäuresequenz aufweist.

4. Der Mikroorganismus gemäß Anspruch 2, **dadurch gekennzeichnet, dass** der Mikroorganismus im Vergleich zu dem jeweiligen Ausgangstamm zusätzlich zu der erhöhten Expression eines Polynukleotids kodierend für ein Polypeptid mit einer Aminosäuresequenz, die eine Identität von 70% oder mehr zu der in SEQ ID NO:8 oder 20 gezeigten Aminosäuresequenz aufweist, eine erhöhte Expression der Polynukleotide a) und b) auf weist:
a) ein Polynukleotid kodierend für ein Polypeptid mit einer Aminosäuresequenz, die eine Identität von 70% oder mehr zu der in SEQ ID NO:2 oder 14 gezeigten Aminosäuresequenz aufweist;
b) ein Polynukleotid kodierend für ein Polypeptid mit einer Aminosäuresequenz, die eine Identität von 70% oder mehr zu der in SEQ ID NO:4 oder 16 gezeigten Aminosäuresequenz aufweist;
sowie des Polynukleotids d) ein Polynukleotid kodierend für ein Polypeptid mit einer Aminosäuresequenz, die eine Identität von 70% oder mehr zu der in SEQ ID NO:10 oder 22 gezeigten Aminosäuresequenz aufweist.

5. Der Mikroorganismus gemäß einem der Ansprüche 1 oder 2, **dadurch gekennzeichnet, dass** der Mikroorganismus im Vergleich zu dem jeweiligen Ausgangstamm zusätzlich zu der erhöhten Expression eines Polynukleotids kodierend für ein Polypeptid mit einer Aminosäuresequenz, die eine Identität von 70% oder mehr zu der in SEQ ID NO:8 oder 20 gezeigten Aminosäuresequenz aufweist, eine erhöhte Expression der Polynukleotide a), b) c), d) und e) wie in Anspruch 2 definiert aufweist.

6. Der Mikroorganismus gemäß irgendeinem der Ansprüche 1 bis 5, **dadurch gekennzeichnet, dass** die organisch-chemische Verbindung ausgewählt ist aus der Gruppe proteinogene L-Aminosäure, L-Omithin und L-Homoserin.

7. Der Mikroorganismus gemäß Anspruch 6, **dadurch gekennzeichnet, dass** die proteinogene L-Aminosäure ausgewählt aus der Gruppe L-Lysin, L-Methionin, L-Valin, L-Prolin, L-Glutamat und L-Isoleucin, bevorzugt L-Lysin.

8. Ein Verfahren zur fermentativen Herstellung einer organisch-chemischen Verbindung enthaltend die Schritte:
a. Kultivieren des Mikroorganismus gemäß irgendeinem der Ansprüche 1 bis 7 in einem geeignetem Medium, wobei eine Fermentationsbrühe erhalten wird, und
b. Anreichern der organisch-chemischen Verbindung in der Fermentationsbrühe aus a).

9. Das Verfahren nach Anspruch 8 **dadurch gekennzeichnet, dass** die Anreicherung von Trehalose in der Fermentationsbrühe verringert oder vermieden wird.

## Claims

1. Microorganism of the species Corynebacterium glutamicum that produces and/or secretes an organochemical compound, **characterized in that** the microorganism an increased expression, compared to the particular starting strain, of a polynucleotide coding for a polypeptide having an amino acid sequence which has an identity of 70% or more in relation to the amino acid sequence shown in SEQ ID NO: 8 or 20,
wherein the increased expression of the polynucleotide is increased by one or more measures selected from the following group:
a) the expression of the gene is under the control of a promoter which is stronger in the microorganism used for the method than the original promoter of the gene;
b) increasing the copy number of the gene coding for a polypeptide having the activity of a trehalose importer; preferably by insertion of the gene in plasmids with increased copy number and/or by integration of the gene into the chromosome of the microorganism in at least one copy;
c) the expression of the gene is achieved with use of a ribosome binding site which is stronger in the microorganism used for the method than the original ribosome binding site of the gene;
d) the expression of the gene is achieved with optimization of the codon usage of the microorganism used for the method;
e) the expression of the gene is achieved with reduction of mRNA secondary structures in the mRNA transcribed from the gene;
f) the expression of the gene is achieved with elimination of RNA polymerase terminators in the mRNA transcribed from the gene;
g) the expression of the gene is achieved with use of mRNA-stabilizing sequences in the mRNA transcribed from the gene;
and wherein said polypeptide, which is a subunit of a protein complex having the activity of a trehalose importer, has the activity of a permease and wherein the microorganism is capable of taking up trehalose from the medium.

2. Microorganism according to Claim 1, **characterized in that** the microorganism has, compared to the particular starting strain, not only the increased expression of a polynucleotide coding for a polypeptide having an amino acid sequence which has an identity of 70% or more in relation to the amino acid sequence shown in SEQ ID NO: 8 or 20, but also an increased expression of at least one polynucleotide selected from group a) to e) :
a) a polynucleotide coding for a polypeptide having an amino acid sequence which has an identity of 70% or more in relation to the amino acid sequence shown in SEQ ID NO: 2 or 14;
b) a polynucleotide coding for a polypeptide having an amino acid sequence which has an identity of 70% or more in relation to the amino acid sequence shown in SEQ ID NO: 4 or 16;
c) a polynucleotide coding for a polypeptide having an amino acid sequence which has an identity of 70% or more in relation to the amino acid sequence shown in SEQ ID NO: 6 or 18;
d) a polynucleotide coding for a polypeptide having an amino acid sequence which has an identity of 70% or more in relation to the amino acid sequence shown in SEQ ID NO: 10 or 22;
e) a polynucleotide coding for a polypeptide having an amino acid sequence which has an identity of 70% or more in relation to the amino acid sequence shown in SEQ ID NO: 12 or 24.

3. Microorganism according to Claim 2, **characterized in that** the microorganism has, compared to the particular starting strain, not only the increased expression of a polynucleotide coding for a polypeptide having an amino acid sequence which has an identity of 70% or more in relation to the amino acid sequence shown in SEQ ID NO: 8 or 20, but also an increased expression of a polynucleotide coding for a polypeptide having an amino acid sequence which has an identity of 70% or more in relation to the amino acid sequence shown in SEQ ID NO: 10 or 22.

4. Microorganism according to Claim 2, **characterized in that** the microorganism has, compared to the particular starting strain, not only the increased expression of a polynucleotide coding for a polypeptide having an amino acid sequence which has an identity of 70% or more in relation to the amino acid sequence shown in SEQ ID NO: 8 or 20, but also an increased expression of polynucleotides a) and b):
a) a polynucleotide coding for a polypeptide having an amino acid sequence which has an identity of 70% or more in relation to the amino acid sequence shown in SEQ ID NO: 2 or 14;
b) a polynucleotide coding for a polypeptide having an amino acid sequence which has an identity of 70% or more in relation to the amino acid sequence shown in SEQ ID NO: 4 or 16;
and also of polynucleotide d) a polynucleotide coding for a polypeptide having an amino acid sequence which has an identity of 70% or more in relation to the amino acid sequence shown in SEQ ID NO: 10 or 22.

5. Microorganism according to either of Claims 1 and 2, **characterized in that** the microorganism has, compared to the particular starting strain, not only the increased expression of a polynucleotide coding for a polypeptide having an amino acid sequence which has an identity of 70% or more in relation to the amino acid sequence shown in SEQ ID NO: 8 or 20, but also an increased expression of polynucleotides a), b), c), d) and e) as defined in Claim 2.

6. Microorganism according to any of Claims 1 to 5, **characterized in that** the organochemical compound is selected from the group consisting of proteinogenic L-amino acid, L-ornithine and L-homoserine.

7. Microorganism according to Claim 6, **characterized in that** the proteinogenic L-amino acid selected from the group consisting of L-lysine, L-methionine, L-valine, L-proline, L-glutamate and L-isoleucine, preferably L-lysine.

8. Method for the fermentative production of an organochemical compound, containing the steps:
a. cultivating the microorganism according to any of Claims 1 to 7 in a suitable medium to obtain a fermentation broth and
b. enriching the organochemical compound in the fermentation broth from a).

9. Method according to Claim 8, **characterized in that** the enrichment of trehalose in the fermentation broth is reduced or avoided.

## Revendications

1. Microorganisme du type Corynebacterium glutamicum, qui produit et/ou sécrète un composé chimique organique, **caractérisé en ce que** le microorganisme une expression augmentée en comparaison de la souche initiale respective d'un polynucléotide codant pour un polypeptide ayant une séquence d'acides aminés qui présente une identité de 70 % ou plus avec la séquence d'acides aminés indiquée dans SED ID NO : 8 ou 20,
l'expression augmentée du polynucléotide étant augmentée par une ou plusieurs mesures choisies dans le groupe suivant :
a) l'expression du gène est sous le contrôle d'un promoteur qui est plus puissant dans le microorganisme utilisé pour le procédé que le promoteur originel du gène ;
b) l'augmentation du nombre de copies du gène codant pour un polypeptide ayant l'activité d'un importateur de tréhalose, de préférence par insertion du gène dans des plasmides avec un nombre de copies augmenté et/ou par intégration du gène dans le chromosome du microorganisme en au moins une copie ;
c) l'expression du gène a lieu en utilisant un emplacement de liaison de ribosomes, qui est plus puissant dans le microorganisme utilisé pour le procédé que l'emplacement de liaison de ribosomes originel du gène ;
d) l'expression du gène a lieu avec optimisation de l'utilisation des codons (codon usage) du microorganisme utilisé pour le procédé ;
e) l'expression du gène a lieu avec réduction de structures secondaires d'ARNm dans l'ARNm transcrit par le gène ;
f) l'expression du gène a lieu avec élimination de terminateurs d'ARN-polymérase dans l'ARNm transcrit par le gène ;
g) l'expression du gène a lieu en utilisant des séquences stabilisant l'ARNm dans l'ARNm transcrit par le gène,
et ce polypeptide, qui est une sous-unité d'un complexe de protéine ayant l'activité d'un importateur de tréhalose, présentant l'activité d'une perméase et le microorganisme étant en mesure d'absorber de la tréhalose depuis le milieu.

2. Microorganisme selon la revendication 1, **caractérisé en ce que** le microorganisme présente en comparaison de la souche initiale respective, en plus de l'expression augmentée d'un polynucléotide codant pour un polypeptide ayant une séquence d'acides aminés qui présente une identité de 70 % ou plus avec la séquence d'acides aminés indiquée dans SEQ ID NO : 8 ou 20, une expression augmentée d'au moins un polynucléotide choisi dans le groupe a) à e) :
a) un polynucléotide codant pour un polypeptide ayant une séquence d'acides aminés qui présente une identité de 70 % ou plus avec la séquence d'acides aminés indiquée dans SEQ ID NO : 2 ou 14 ;
b) un polynucléotide codant pour un polypeptide ayant une séquence d'acides aminés qui présente une identité de 70 % ou plus avec la séquence d'acides aminés indiquée dans SEQ ID NO : 4 ou 16 ;
c) un polynucléotide codant pour un polypeptide ayant une séquence d'acides aminés qui présente une identité de 70 % ou plus avec la séquence d'acides aminés indiquée dans SEQ ID NO : 6 ou 18 ;
d) un polynucléotide codant pour un polypeptide ayant une séquence d'acides aminés qui présente une identité de 70 % ou plus avec la séquence d'acides aminés indiquée dans SEQ ID NO : 10 ou 22 ;
e) polynucléotide codant pour un polypeptide ayant une séquence d'acides aminés qui présente une identité de 70 % ou plus avec la séquence d'acides aminés indiquée dans SEQ ID NO : 12 ou 24.

3. Microorganisme selon la revendication 2, **caractérisé en ce que** le microorganisme présente en comparaison de la souche initiale respective, en plus de l'expression augmentée d'un polynucléotide codant pour un polypeptide ayant une séquence d'acides aminés qui présente une identité de 70 % ou plus avec la séquence d'acides aminés indiquée dans SEQ ID NO : 8 ou 20, une expression augmentée d'un polynucléotide codant pour un polypeptide ayant une séquence d'acides aminés qui présente une identité de 70 % ou plus avec la séquence d'acides aminés indiquée dans SEQ ID NO : 10 ou 22.

4. Microorganisme selon la revendication 2, **caractérisé en ce que** le microorganisme présente en comparaison de la souche initiale respective, en plus de l'expression augmentée d'un polynucléotide codant pour un polypeptide ayant une séquence d'acides aminés qui présente une identité de 70 % ou plus avec la séquence d'acides aminés indiquée dans SEQ ID NO : 8 ou 20, une expression augmentée des polynucléotides a) et b) :
a) un polynucléotide codant pour un polypeptide ayant une séquence d'acides aminés qui présente une identité de 70 % ou plus avec la séquence d'acides aminés indiquée dans SEQ ID NO : 2 ou 14 ;
b) un polynucléotide codant pour un polypeptide ayant une séquence d'acides aminés qui présente une identité de 70 % ou plus avec la séquence d'acides aminés indiquée dans SEQ ID NO : 4 ou 16 ;
ainsi que du polynucléotide d), un polynucléotide codant pour un polypeptide ayant une séquence d'acides aminés qui présente une identité de 70 % ou plus avec la séquence d'acides aminés indiquée dans SEQ ID NO : 10 ou 22.

5. Microorganisme selon l'une quelconque des revendications 1 ou 2, **caractérisé en ce que** le microorganisme présente en comparaison de la souche initiale respective, en plus de l'expression augmentée d'un polynucléotide codant pour un polypeptide ayant une séquence d'acides aminés qui présente une identité de 70 % ou plus avec la séquence d'acides aminés indiquée dans SEQ ID NO : 8 ou 20, une expression augmentée des polynucléotides a), b), c), d) et e) tels que définis dans la revendication 2.

6. Microorganisme selon l'une quelconque des revendications 1 à 5, **caractérisé en ce que** le composé chimique organique est choisi dans le groupe constitué par un acide L-aminé protéinogène, la L-ornithine et la L-homosérine.

7. Microorganisme selon la revendication 6, **caractérisé en ce que** l'acide L-aminé protéinogène choisi dans le groupe constitué par la L-lysine, la L-méthionine, la L-valine, la L-proline, le L-glutamate et la L-isoleucine, de préférence la L-lysine.

8. Procédé de fabrication par fermentation d'un composé chimique organique contenant les étapes suivantes :
a. la culture du microorganisme selon l'une quelconque des revendications 1 à 7 dans un milieu approprié, un bouillon de fermentation étant obtenu, et
b. l'enrichissement du composé organique chimique dans le bouillon de fermentation de a).

9. Procédé selon la revendication 8, **caractérisé en ce que** l'enrichissement de tréhalose dans le bouillon de fermentation est réduit ou évité.
